# EUROPEAN PATENT APPLICATION

(11) **EP 3 412 309 A1**
(43) Date of publication of application: **12.12.2018**
(21) Application number: 17208413.9
(22) Date of filing: 30.03.2012
(51) Int. Cl.: A61K 39/395, A61P 1/04, C07K 16/00, A61K 31/196, C07K 16/28, A61K 31/519, A61K 31/52, A61K 39/00

(54) **METHODS OF ADMINISTERING BETA7 INTEGRIN ANTAGONISTS**

(30) Priority: 31.03.2011 US 201161470360 P; 21.10.2011 US 201161550216 P
(62) Divisional of application: 12765884.7
(71) Applicant: F. Hoffmann-La Roche AG, 4070 Basel (CH)
(72) Inventor: Stefanich, Eric Gary, South San Francisco, CA 94080 (US); Williams, Marna Bromberg, South San Francisco, CA 94080 (US); Anand, Banmeet, South San Francisco, CA 94080 (US); Tang, Meina, South San Francisco, CA 94080 (US); Visich, Jennifer, South San Francisco, CA 94080 (US); O'Byrne, Sharon, South San Francisco, CA 94080 (US)
(74) Representative: Heiroth, Ulrike Hildegard

(57) **Abstract**

Methods of treating gastrointestinal inflammatory disorders such as inflammatory bowel diseases including ulcerative colitis and Crohn's disease are provided. Also provided are methods of administering integrin beta7 antagonists, such as anti-beta7 antibodies. In addition, particular dosing regimens, including dosing regimens comprising subcutaneous administration and administration using self-inject devices are provided.

## Description

### CROSS REFERENCE TO RELATED APPLICATIONS

This application claims the benefit of priority of provisional U.S. Application No. 61/470,360 filed March 31, 2011 and provisional U.S. Application No. 61/550,216 filed October 21, 2011, both of which are hereby incorporated by reference in their entirety.

### SEQUENCE LISTING

The instant application contains a Sequence Listing which has been submitted in ASCII format via EFS-Web and is hereby incorporated by reference in its entirety. Said ASCII copy, created on March 15, 2012, is named P4622R1WO_SequenceListing.txt and is 17,964 bytes in size.

### FIELD

Methods of treating gastrointestinal inflammatory disorders such as inflammatory bowel diseases including ulcerative colitis and Crohn's disease are provided. Also provided are methods of administering integrin beta7 antagonists, such as anti-beta7 antibodies. In addition, particular dosing regimens, including dosing regimens comprising subcutaneous administration and administration using self-inject devices are provided.

### BACKGROUND

Inflammatory bowel disease (IBD) is a chronic inflammatory autoimmune condition of the gastrointestinal (GI) tract, which presents clinically as either ulcerative colitis (UC) or Crohn's disease (CD). CD is a chronic transmural inflammatory disease with the potential to affect any part of the entire GI tract, and UC is a mucosal inflammation of the colon. Both conditions are characterized clinically by frequent bowel motions, malnutrition, and dehydration, with disruption in the activities of daily living. CD is frequently complicated by the development of malabsorption, strictures, and fistulae and may require repeated surgery. UC, less frequently, may be complicated by severe bloody diarrhea and toxic megacolon, also requiring surgery. Both IBD conditions are associated with an increased risk for malignancy of the GI tract. The etiology of IBD is complex, and many aspects of the pathogenesis remain unclear.

The treatment of moderate to severe IBD poses significant challenges to treating physicians, because conventional therapy with corticosteroids and immunomodulator therapy (e.g., azathioprine, 6 mercaptopurine, and methotrexate) is associated with side effects and intolerance and has not shown proven benefit in maintenance therapy (steroids). Monoclonal antibodies targeting tumor necrosis factor alpha (TNF-α), such as infliximab (a chimeric antibody) and adalimumab (a fully human antibody), are currently used in the management of CD. Infliximab has also shown efficacy and has been approved for use in UC. However, approximately 10%-20% of patients with CD are primary nonresponders to anti TNF therapy, and another ∼20%-30% of CD patients lose response over time (Schnitzler et al., Gut 58:492-500 (2009)). Other adverse events (AEs) associated with anti TNFs include elevated rates of bacterial infection, including tuberculosis, and, more rarely, lymphoma and demyelination (Chang et al., Nat Clin Pract Gastroenterol Hepatology 3:220 (2006); Hoentjen et al., World J. Gastroenterol. 15(17):2067 (2009)). No currently available therapy achieves sustained remission in more than 20%-30% of IBD patients with chronic disease (Hanauer et al., Lancet 359:1541-49 (2002); Sandborn et al., N Engl J Med 353:1912-25 (2005)). In addition, most patients do not achieve sustained steroid-free remission and mucosal healing, clinical outcomes that correlate with true disease modification. Therefore, there is a need to develop more targeted therapy in IBD that is optimized for chronic use: an improved safety profile with sustained remission, particularly steroid-free remission and prevention of long-term complications in a greater proportion of patients, including those patients who either never respond to an anti TNF therapeutic agent or lose response over time.

The integrins are alpha/beta heterodimeric cell surface glycoprotein receptors that play a role in numerous cellular processes including leukocyte adhesion, signaling, proliferation, and migration, as well as in gene regulation (Hynes, R. O., Cell, 1992, 69:11-25; and Hemler, M. E., Annu. Rev. Immunol., 1990, 8:365-368). They are composed of two heterodimeric, non-covalently interacting α and β transmembrane subunits that bind specifically to distinct cell adhesion molecules (CAMs) on endothelia, epithelia, and extracellular matrix proteins. In this manner, integrins can function as tissue-specific cell adhesion receptors aiding in the recruitment of leukocytes from blood into nearly all tissue sites in a highly regulated manner, playing a role in the homing of leukocytes to normal tissue and to sites of inflammation (von Andrian et al., N Engl J Med 343:1020-34 (2000)). In the immune system, integrins are involved in leukocyte trafficking, adhesion and infiltration during inflammatory processes (Nakajima, H. et al., J. Exp. Med., 1994, 179:1145-1154). Differential expression of integrins regulates the adhesive properties of cells and different integrins are involved in different inflammatory responses. (Butcher, E. C. et al., Science, 1996, 272:60-66). The beta7 containing integrins (*i.e.,* alpha4beta7 and alphaEbeta7) are expressed primarily on monocytes, lymphocytes, eosinophils, basophils, and macrophages but not on neutrophils (Elices, M. J. et al., Cell, 1990, 60:577-584).

The α4β7 integrin is a leukocyte-homing receptor that is important in the migration of cells to the intestinal mucosa and associated lymphoid tissues, such as Peyer's patches in the small intestine, lymphoid follicles in the large intestine, and mesenteric lymph nodes. In the gut, leukocyte rolling and firm adhesion to the mucosal endothelium is initiated by signals from chemokines and is mediated via mucosal addressin cell adhesion molecule (MAdCAM)-1-associated sialyl Lewis X. Chemokine signaling induces the α4β7 integrin to undergo a change from low to high MAdCAM-1 binding affinity. The leukocyte then arrests and begins the process of extravasation through the vascular endothelium to underlying tissue. This extravasation process is believed to occur in both the normal immune cell recirculation state and in inflammatory conditions (von Andrian et al., *supra*). The numbers of α4β7⁺ cells in infiltrates and the expression of the ligand MAdCAM-1 are higher at sites of chronic inflammation such as in the intestinal tract of patients with UC or CD (Briskin et al., Am J Pathol 151:97-110 (1997); Souza et al., Gut 45:856-63 (1999)). α4β7 binds preferentially to high endothelial venules expressing MAdCAM-1 and vascular cell adhesion molecule (VCAM)-1, as well as to the extracellular matrix molecule fibronectin fragment CS-1 (Chan et al., J Biol Chem 267:8366-70 (1992); Ruegg et al., J Cell Biol 17:179-89 (1992); Berlin et al., Cell 74:185-95 (1993)). Together with constitutively expressed MAdCAM-1 in gut mucosal vessels, the α4β7 integrin plays a selective role in leukocyte gut tropism but does not seem to contribute to homing of leukocytes to the peripheral tissue or the CNS. Instead, peripheral lymphoid trafficking has been associated with α4β1 interaction with VCAM-1 (Yednock et al., Nature 356:63-6 (1992); Rice et al., Neurology 64:1336-42 (2005)).

Another member of the β7 integrin family, expressed exclusively on T lymphocytes and associated with mucosal tissues, is the αEβ7 integrin, otherwise known as CD103. The αEβ7 integrin binds selectively to E-cadherin on epithelial cells and has been proposed to play a role in the retention of T cells in the mucosal tissue in the intraepithelial lymphocyte compartment (Cepek et al., J Immunol 150:3459-70 (1993); Karecla et al. Eur J Immunol 25:852-6 (1995)). The αEβ7⁺ cells in the lamina propria have been reported to exhibit cytotoxicity against stressed or infected epithelial cells (Hadley et al., J Immunol 159:3748-56 (1997); Buri et al., J Pathol 206:178-85 (2005)). The expression of αEβ7 is increased in CD (Elewaut et al., Acta Gastroenterol Belg 61:288-94 (1998); Oshitani et al., Int J Mol Med 12:715-9 (2003)), and anti-αEβ7 antibody treatment has been reported to attenuate experimental colitis in mice, implicating a role for αEβ7⁺ lymphocytes in experimental models of IBD (Ludviksson et al., J Immunol 162:4975-82 (1999)).

Administration of monoclonal antibodies against alphaE beta7 reportedly prevents and ameliorates immunization induced colitis in IL-2^{-/-} mice, suggesting that the onset and maintenance of inflammatory bowel disease depends on colonic localization of lamina propria CD4⁺ lymphocytes expressing alphaEbeta7 (Ludviksson et al., J Immunol. 1999, 162(8):4975-82). An anti-α4 antibody (natalizumab) reportedly has efficacy in treatment of patients with CD (Sandborn et al., N Engl J Med 2005;353:1912-25) and an anti-α4β7 antibody (MLN-02, MLN0002, vedolizumab) reportedly is effective in patients with UC (Feagan et al., N Engl J Med 2005;352:2499-507). These findings validate α4β7 as a therapeutic target and support the idea that the interaction between α4β7 and MAdCAM-1 mediates the pathogenesis of IBD. Thus, antagonists of beta7 integrin are of great potential as a therapeutic agent in treating IBD.

Humanized monoclonal antibodies targeted against the β7 integrin subunit have been described previously. See, e.g., Intn'1 Patent Pub. No. WO2006/026759. One such antibody, rhuMAb Beta7 (etrolizumab) is derived from the rat anti-mouse/human monoclonal antibody FIB504 (Andrew et al. 1994). It was engineered to include human IgG1-heavy chain and κ1-light chain frameworks. Intn'1 Patent Pub. No. WO2006/026759.

RhuMAb Beta7 binds α4β7 (Holzmann et al., Cell 56:37-46 (1989); Hu et al., Proc Natl Acad Sci USA 89:8254-8 (1992)) and αEβ7 (Cepek et al., J Immunol 150:3459-70 (1993)), which regulate trafficking and retention of lymphocyte subsets, respectively, in the intestinal mucosa. Clinical studies have demonstrated the efficacy of an anti α4 antibody (natalizumab) for the treatment of CD (Sandborn et al., N Engl J Med 353:1912-25 (2005)), and encouraging results have been reported for anti α4β7 antibody (LDP02/MLN02/MLN0002/vedolizumab) in the treatment of UC (Feagan et al., N Engl J Med 352:2499-507 (2005)). These findings help to validate α4β7 as a potential therapeutic target and support the hypothesis that the interaction between α4β7 and mucosal addressin cell adhesion molecule 1 (MAdCAM 1) contributes to the pathogenesis of inflammatory bowel disease (IBD).

Unlike natalizumab, which binds α4 and thus binds both α4β1 and α4β7, rhuMAb Beta7 binds specifically to the β7 subunit of α4β7 and αEβ7 and does not bind to α4 or β1 integrin individual subunits. This was demonstrated by the inability of the antibody to inhibit adhesion of α4β1+α4β7- Ramos cells to vascular cell adhesion molecule 1 (VCAM 1) at concentrations as high as 100 nM. Importantly, this characteristic of rhuMAb Beta7 indicates selectivity: T cell subsets expressing α4β1 but not β7 should not be directly affected by rhuMAb Beta7.

Support for the gut-specific effects of rhuMAb Beta7 on leukocyte homing comes from several in vivo nonclinical studies. In severe combined immunodeficient (SCID) mice reconstituted with CD45RB^{high}CD4+ T cells (an animal model of colitis), rhuMAb Beta7 blocked radiolabeled lymphocyte homing to the inflamed colon but did not block homing to the spleen, a peripheral lymphoid organ. See, e.g., Intn'1 Patent Pub. No. WO2006/026759. In addition, the rat-mouse chimeric anti-murine β7 (anti β7, muFIB504) was unable to reduce the histologic degree of central nervous system (CNS) inflammation or improve disease survival in myelin basic protein T cell receptor (MBP-TCR) transgenic mice with experimental autoimmune encephalitis (EAE), an animal model of multiple sclerosis. *Id.* Furthermore, in two safety studies in cynomolgus monkeys, rhuMAb Beta7 induced a moderate increase in peripheral blood lymphocyte numbers that was largely due to a marked (approximately three- to sixfold) increase in CD45RA⁻β7^{high} peripheral blood T cells, a subset that is phenotypically similar to gut-homing memory/effector T cells in humans. See, e.g., Intn'1 Patent Pub. No. WO2009/140684; Stefanich et al., Br. J. Pharmacol., Accepted Article, doi: 10.1111/j.1476-5381.2011.01205.x. In contrast, rhuMAb Beta7 had minimal to no effect on the number of CD45RA+β7 intermediate peripheral blood T cells, a subset that is phenotypically similar to naive T cells in humans, and no effect on the number of CD45RA⁻β7^{low} peripheral blood T cells, a subset that is phenotypically similar to peripheral homing memory/effector T cells in humans, confirming the specificity of rhuMAb Beta7 for the gut homing lymphocyte subpopulation. Intn'1 Patent Pub. No. WO2009/140684; Stefanich et al., Br. J. Pharmacol., Accepted Article, doi: 10.1111/j.1476-5381.2011.01205.x.

While clinical studies have demonstrated the efficacy of an anti-α4 antibody (natalizumab) for the treatment of CD (Sandborn et al., N Engl J Med 353:1912-25 (2005)), and encouraging results have been reported for anti-α4β7 antibody (LDP02/MLN02/MLN0002/vedolizumab) in the treatment of UC, there remains a need for further improvements in the treatment of these disorders. For example, natalizumab treatment has been associated with confirmed cases of progressive multifocal leukoencephalopathy (PML) in patients with Crohn's disease (and separately, multiple sclerosis) who received concomitant treatment with natalizumab and immunosupressives. PML is a potentially fatal neurological condition linked to reactivation of a polyomavirus (JC virus) and active viral replication in the brain. No known interventions can reliably prevent PML or adequately treat PML, if it occurs. One limitation of vedolizumab treatment is that it is administered intravenously which can be inconvenient for the patient and can also be associated with undesirable or adverse events, e.g., infusion site reactions. Accordingly, there is a need for improved therapeutic approaches to the treatment of gastrointestinal inflammatory disorders such as IBD, e.g., ulcerative colitis and Crohn's disease, as well as more desirable dosing regimens.

The invention described herein meets certain of the above-described needs and provides other benefits.

All references cited herein, including patent applications and publications, are incorporated by reference in their entirety for any purpose.

### SUMMARY

The methods of the invention are based, at least in part, on the discovery that therapeutically effective amounts of integrin beta7 antagonists, such as anti-beta7 antibodies, including rhuMAb Beta7 (etrolizumab) can be administered subcutaneously as well as intravenously.

Accordingly, in one aspect, methods of treating a gastrointestinal inflammatory disorder in a mammalian subject comprising administering to the subject a therapeutically effective amount of an integrin beta7 antagonist are provided. In certain embodiments, the mammalian subject is a patient. In certain embodiments, the patient is human. In certain embodiments, the integrin beta7 antagonist is administered intravenously. In certain embodiments, the integrin beta7 antagonist is administered subcutaneously. In one aspect, the gastrointestinal inflammatory disorder is an inflammatory bowel disease. In certain embodiments, the inflammatory bowel disease us ulcerative colitis (UC) or Crohn's disease (CD). In certain embodiments, the integrin beta7 antagonist is a monoclonal anti-beta7 antibody. In certain such embodiments, the anti-beta7 antibody is selected from a chimeric antibody, a human antibody, and a humanized antibody. In certain embodiments, the anti-beta7 antibody is an antibody fragment. In certain embodiments, the anti-beta7 antibody comprises six hypervariable regions (HVRs), wherein:
(i) HVR-L1 comprises amino acid sequence A1-A11, wherein A1-A11 is RASESVDTYLH (SEQ ID NO:1); RASESVDSLLH (SEQ ID NO:7), RASESVDTLLH (SEQ ID NO:8), or RASESVDDLLH (SEQ ID NO:9) or a variant of SEQ ID NOs:1, 7, 8 or 9 (SEQ ID NO:26) wherein amino acid A2 is selected from the group consisting of A, G, S, T, and V and/or amino acid A3 is selected from the group consisting of S, G, I, K, N, P, Q, R, and T, and/or A4 is selected from the group consisting of E, V, Q, A, D, G, H, I, K, L, N, and R, and/or amino acid A5 is selected from the group consisting of S, Y, A, D, G, H, I, K, N, P, R, T, and V, and/or amino acid A6 is selected from the group consisting of V, R, I, A, G, K, L, M, and Q, and/or amino acid A7 is selected from the group consisting of D, V, S, A, E, G, H, I, K, L, N, P, S, and T, and/or amino acid A8 is selected from the group consisting of D, G, N, E, T, P and S, and/or amino acid A9 is selected from the group consisting of L, Y, I and M, and/or amino acid A10 is selected from the group consisting of L, A, I, M, and V and/or amino acid A11 is selected from the group consisting of H, Y, F, and S;
(ii) HVR-L2 comprises amino acid sequence B1-B8, wherein B1-B8 is KYASQSIS (SEQ ID NO:2), RYASQSIS (SEQ ID NO:20), or XaaYASQSIS (SEQ ID NO:21, where Xaa represents any amino acid) or a variant of SEQ ID NOs:2, 20 or 21 (SEQ ID NO:27) wherein amino acid B1 is selected from the group consisting of K, R, N, V, A, F, Q, H, P, I, L, Y and Xaa (where Xaa represents any amino acid), and/or amino acid B4 is selected from the group consisting of S and D, and/or amino acid B5 is selected from the group consisting of Q and S, and/or amino acid B6 is selected from the group consisting of S, D, L, and R, and/or amino acid B7 is selected from the group consisting of I, V, E, and K;
(iii) HVR-L3 comprises amino acid sequence C1-C9, wherein C1-C9 is QQGNSLPNT (SEQ ID NO:3) or a variant of SEQ ID NO:3 (SEQ ID NO:28) wherein amino acid C8 is selected from the group consisting of N, V, W, Y, R, S, T, A, F, H, I L, and M;
(iv) HVR-H1 comprises amino acid sequence D1-D10 wherein D1-D10 is GFFITNNYWG (SEQ ID NO:4);
(v) HVR-H2 comprises amino acid sequence E1-E17 wherein E1-E17 is GYISYSGSTSYNPSLKS (SEQ ID NO:5), or a variant of SEQ ID NO:5 (SEQ ID NO:29) wherein amino acid E2 is selected from the group consisting of Y, F, V, and D, and/or amino acid E6 is selected from the group consisting of S and G, and/or amino acid E10 is selected from the group consisting of S and Y, and/or amino acid E12 is selected from the group consisting of N, T, A, and D, and/or amino acid 13 is selected from the group consisting of P, H, D, and A, and/or amino acid E15 is selected from the group consisting of L and V, and/or amino acid E17 is selected from the group consisting of S and G; and
(vi) HVR-H3 comprises amino acid sequence F2-F11 wherein F2-F11 is MTGSSGYFDF (SEQ ID NO:6) or RTGSSGYFDF (SEQ ID NO:19); or comprises amino acid sequence F1-F11, wherein F1-F11 is AMTGSSGYFDF (SEQ ID NO:16), ARTGSSGYFDF (SEQ ID NO:17), or AQTGSSGYFDF (SEQ ID NO:18), or a variant of SEQ ID NOs:6, 16, 17, 18, or 19 (SEQ ID NO:30) wherein amino acid F2 is R, M, A, E, G, Q, S, and/or amino acid F11 is selected from the group consisting of F and Y. In certain such embodiments, the anti-beta7 antibody comprises three heavy chain hypervariable region (HVR-H1-H3) sequences and three light chain hypervariable region (HVR-L1-L3) sequences, wherein:
   (i) HVR-L1 comprises SEQ ID NO:7, SEQ ID NO:8 or SEQ ID NO:9;
   (ii) HVR-L2 comprises SEQ ID NO:2;
   (iii) HVR-L3 comprises SEQ ID NO:3;
   (iv) HVR-H1 comprises SEQ ID NO:4;
   (v) HVR-H2 comprises SEQ ID NO:5; and
   (vi) HVR-H3 comprises SEQ ID NO:6 or SEQ ID NO:16 or SEQ ID NO:17 or SEQ ID NO:19.

In another aspect, methods of subcutaneously administering therapeutically effective amounts of anti-beta7 antibodies at a weight-based dose are provided. In certain embodiments, the anti-beta7 antibody is administered at a dose of 0.5 mg/kg. In certain embodiments, the anti-beta7 antibody is administered at a dose of 1.5 mg/kg. In certain embodiments, the anti-beta7 antibody is administered at a dose of 3.0 mg/kg. In certain embodiments, the anti-beta7 antibody is administered at a dose of 4.0 mg/kg. In one aspect, the anti-beta7 antibody is administered at regular intervals over time. In certain embodiments, the anti-beta7 antibody is administered once every week. In certain embodiments, the anti-beta7 antibody is administered once every two weeks. In certain embodiments, the anti-beta7 antibody is administered once every four weeks. In certain embodiments, the anti-beta7 antibody is administered once every six weeks. In certain embodiments, the anti-beta7 antibody is administered once every eight weeks. In certain embodiments, the anti-beta7 antibody is administered for a period of two months. In certain embodiments, the anti-beta7 antibody is administered for a period of three months. In certain embodiments, the anti-beta7 antibody is administered for a period of six months. In certain embodiments, the anti-beta7 antibody is administered for a period of 12 months. In certain embodiments, the anti-beta7 antibody is administered for a period of 18 months. In certain embodiments, the anti-beta7 antibody is administered for a period of 24 months. In certain embodiments, the anti-beta7 antibody is administered for the lifetime of the subject. In certain embodiments the anti-beta7 antibody is rhuMAb Beta7 (etrolizumab).

In yet another aspect, methods of subcutaneously administering therapeutically-effective amounts of anti-beta7 antibodies at a flat dose are provided. In certain embodiments, the anti-beta7 antibody is administered at a flat dose between 50 mg and 450 mg. In certain embodiments, the flat dose is 50 mg. In certain such embodiments, the flat dose is 100 mg. In certain such embodiments, the flat dose is 150 mg. In certain such embodiments, the flat dose is 200 mg. In certain such embodiments, the flat dose is 300 mg. In certain embodiments, the flat dose is 350 mg. In certain such embodiments, the flat dose is 400 mg. In certain such embodiments, the flat dose is 420 mg. In certain such embodiments, the flat dose is 450 mg. In one aspect, the anti-beta7 antibody is administered at regular intervals over time. In certain such embodiments, the anti-beta7 antibody is administered once every week. In certain embodiments, the anti-beta7 antibody is administered once every two weeks. In certain embodiments, the anti-beta7 antibody is administered once every four weeks. In certain embodiments, the anti-beta7 antibody is administered once every six weeks. In certain embodiments, the anti-beta7 antibody is administered once every eight weeks. In certain embodiments, the anti-beta7 antibody is administered for a period of two months. In certain embodiments, the anti-beta7 antibody is administered for a period of three months. In certain embodiments, the anti-beta7 antibody is administered for a period of six months. In certain embodiments, the anti-beta7 antibody is administered for a period of 12 months. In certain embodiments, the anti-beta7 antibody is administered for a period of 18 months. In certain embodiments, the anti-beta7 antibody is administered for a period of 24 months. In certain embodiments, the anti-beta7 antibody is administered for the lifetime of the subject. In certain embodiments the anti-beta7 antibody is rhuMAb Beta7 (etrolizumab).

In still another aspect, methods of administering therapeutically-effective amounts of anti-beta7 antibodies at a flat dose are provided, wherein the administration comprises administration of a first loading dose and administration of at least one subsequent maintenance dose, wherein each of the loading dose and the at least one maintenance dose is administered at a flat dose. In certain embodiments, the loading dose of the anti-beta7 antibody is administered intravenously. In certain embodiments, the loading dose of the anti-beta7 antibody is administered subcutaneously. In certain embodiments, the at least one subsequent maintenance dose of the anti-beta7 antibody is administered intravenously. In certain embodiments, the at least one subsequent maintenance dose of the anti-beta7 antibody is administered subcutaneously. In certain embodiments, each of the loading dose and at least one subsequent maintenance dose is administered intravenously. In certain embodiments, each of the loading dose and at least one subsequent maintenance dose is administered subcutaneously. In certain embodiments, the loading dose is administered intravenously, and each of the at least one subsequent maintenance dose is administered subcutaneously. In certain embodiments, the loading dose is between 400 mg and 450 mg and the maintenance dose is between 50 mg and 350 mg. In certain embodiments, the loading dose is 400 mg. In certain embodiments, the loading dose is 420 mg. In certain embodiments, the loading dose is 430 mg. In certain embodiments, the loading dose is 450 mg. In certain embodiments, the maintenance dose is 50 mg. In certain embodiments, the maintenance dose is 100 mg. In certain embodiments, the maintenance dose is 150 mg. In certain embodiments, the maintenance dose is 200 mg. In certain embodiments, the maintenance dose is 300 mg. In certain embodiments, the maintenance dose is 350 mg. In certain embodiments, the loading dose is 400 mg and the maintenance dose is 50 mg. In certain embodiments, the loading dose is 400 mg and the maintenance dose is 100 mg. In certain embodiments, the loading dose is 400 mg and the maintenance dose is 150 mg. In certain embodiments, the loading dose is 400 mg and the maintenance dose is 200 mg. In certain embodiments, the loading dose is 400 mg and the maintenance dose is 300 mg. In certain embodiments, the loading dose is 400 mg and the maintenance dose is 350 mg. In certain embodiments, the loading dose is 420 mg and the maintenance dose is 50 mg. In certain embodiments, the loading dose is 420 mg and the maintenance dose is 100 mg. In certain embodiments, the loading dose is 420 mg and the maintenance dose is 150 mg. In certain embodiments, the loading dose is 420 mg and the maintenance dose is 200 mg. In certain embodiments, the loading dose is 420 mg and the maintenance dose is 300 mg. In certain embodiments, the loading dose is 420 mg and the maintenance dose is 350 mg. In certain embodiments, the loading dose is 430 mg and the maintenance dose is 50 mg. In certain embodiments, the loading dose is 430 mg and the maintenance dose is 100 mg. In certain embodiments, the loading dose is 430 mg and the maintenance dose is 150 mg. In certain embodiments, the loading dose is 430 mg and the maintenance dose is 200 mg. In certain embodiments, the loading dose is 430 mg and the maintenance dose is 300 mg. In certain embodiments, the loading dose is 430 mg and the maintenance dose is 350 mg. In certain embodiments, the loading dose is 450 mg and the maintenance dose is 50 mg. In certain embodiments, the loading dose is 450 mg and the maintenance dose is 100 mg. In certain embodiments, the loading dose is 450 mg and the maintenance dose is 150 mg. In certain embodiments, the loading dose is 450 mg and the maintenance dose is 200 mg. In certain embodiments, the loading dose is 450 mg and the maintenance dose is 300 mg. In certain embodiments, the loading dose is 450 mg and the maintenance dose is 350 mg. In certain embodiments the anti-beta7 antibody is rhuMAb Beta7 (etrolizumab).

In another aspect, methods of subcutaneously administering therapeutically-effective amounts of anti-beta7 antibodies at a loading dose followed by one or more maintenance doses are provided. In certain embodiments, the first maintenance dose is administered on the same day as the loading dose. In certain embodiments, the first maintenance dose is administered one day after the loading dose. In certain embodiments, the first maintenance dose is administered one week after the loading dose. In certain embodiments, the first maintenance dose is administered two weeks after the loading dose. In certain embodiments, the first maintenance dose is administered four weeks after the loading dose. In certain embodiments, the second and each subsequent maintenance dose is administered every two weeks. In certain embodiments, the second and each subsequent maintenance dose is administered every four weeks. In certain embodiments, the second and each subsequent maintenance dose is administered every eight weeks. In certain embodiments, the first maintenance dose is administered two weeks after the loading dose, the second maintenance dose is administered two weeks after the first maintenance dose, and each subsequent maintenance dose is administered every four weeks. In certain embodiments, the anti-beta7 antibody is administered for a period of two months. In certain embodiments, the anti-beta7 antibody is administered for a period of three months. In certain embodiments, the anti-beta7 antibody is administered for a period of six months. In certain embodiments, the anti-beta7 antibody is administered for a period of 12 months. In certain embodiments, the anti-beta7 antibody is administered for a period of 18 months. In certain embodiments, the anti-beta7 antibody is administered for a period of 24 months. In certain embodiments, the anti-beta7 antibody is administered for the lifetime of the subject. In certain embodiments the anti-beta7 antibody is rhuMAb Beta7 (etrolizumab).

In yet another aspect, an article of manufacture comprising an integrin beta7 antagonist is provided. In certain embodiments, the article of manufacture comprises a prefilled syringe or an autoinjector device comprising 2 ML (150 mg) integrin beta7 antagonist. In certain embodiments, the article of manufacture comprises a prefilled syringe or an autoinjector device comprising 1 ML (180 mg) integrin beta7 antagonist. In certain embodiments the integrin beta7 antagonist is an anti-beta7 antibody or rhuMAb Beta7 (etrolizumab).

In still yet another aspect, methods of subcutaneously administering therapeutically effective amounts of an integrin beta7 antagonist with at least one additional compound are provided. In certain embodiments, the at least one additional compound is selected from 5-aminosalicylic acid (5-ASA), azathioprine (AZA), 6-mercaptopurine (6-MP), and methotrexate.

In another aspect, methods of subcutaneously administering therapeutically effective amounts of an integrin beta7 antagonist where the subject or the patient previously failed at least one biological agent are provided. In certain embodiments, the biological agent is selected from adalimumab, etanercept, infliximab, golimumab, certolizumab pegol, natalizumab, and vedolizumab. In certain embodiments the integrin beta7 antagonist is an anti-beta7 antibody or rhuMAb Beta7 (etrolizumab).

In yet another aspect, methods of subcutaneously administering therapeutically effective amounts of an integrin beta7 antagonist with a self-inject device are provided. In certain embodiments, the self-inject device is selected from a prefilled syringe, microneedle device, an autoinjector device and needle-free injection device. In certain embodiments the integrin beta7 antagonist is an anti-beta7 antibody or rhuMAb Beta7 (etrolizumab).

In still another aspect, methods of inducing remission in an ulcerative colitis patient are provided. Such methods include administering an anti-beta7 antibody according to any of the doses or dosing regimens indicated above. In certain embodiments, the anti-beta7 antibody is rhuMAb Beta7 (etrolizumab). In certain embodiments, remission in the patient is determined to be induced when the absolute Mayo Clinic Score ≤ 2 and no individual subscore >1, which is also referred to as clinical remission. In certain embodiments remission in the patient is induced at least by week 10 following initiation of treatment with anti-beta7 antibody. In certain embodiments, patients administered anti-beta7 antibody as described above retain clinical remission from induction and continue in remission for a certain period of time, also referred to as sustained remission. In certain such embodiments, sustained remission is for a period of 8 weeks following induction of remission, or a period of 30 weeks following induction of remission, or a period of 50 weeks following induction of remission, or a period of 54 weeks or more following induction of remission. In certain embodiments, patients administered anti-beta7 antibody according to any of the doses or dosing regimens indicated above experience sustained steroid-free remission. In certain embodiments, patients who experience sustained steroid-free remission discontinue corticosteroid use at week 30 following induction of remission and sustain remission through week 50 or week 54 or longer following induction of remission. In certain, embodiments, patients administered anti-beta7 antibody as described above experience a reduced time to flare or a reduced rate of flares over a given period of time. In certain embodiments, patients administered anti-beta7 antibody as described above experience mucosal healing. In certain embodiments, patients who experience mucosal healing are determined to have an endoscopy subscore of 0 or 1 as assessed by flexible sigmoidoscopy. In certain embodiments the anti-beta7 antibody is rhuMAb Beta7 (etrolizumab).

### BRIEF DESCRIPTION OF THE DRAWINGS

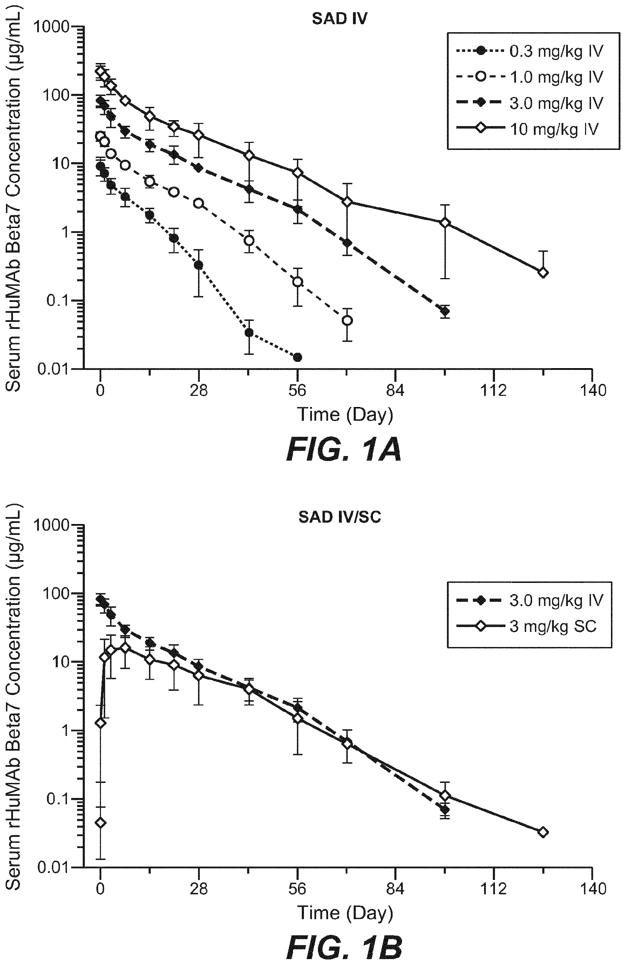

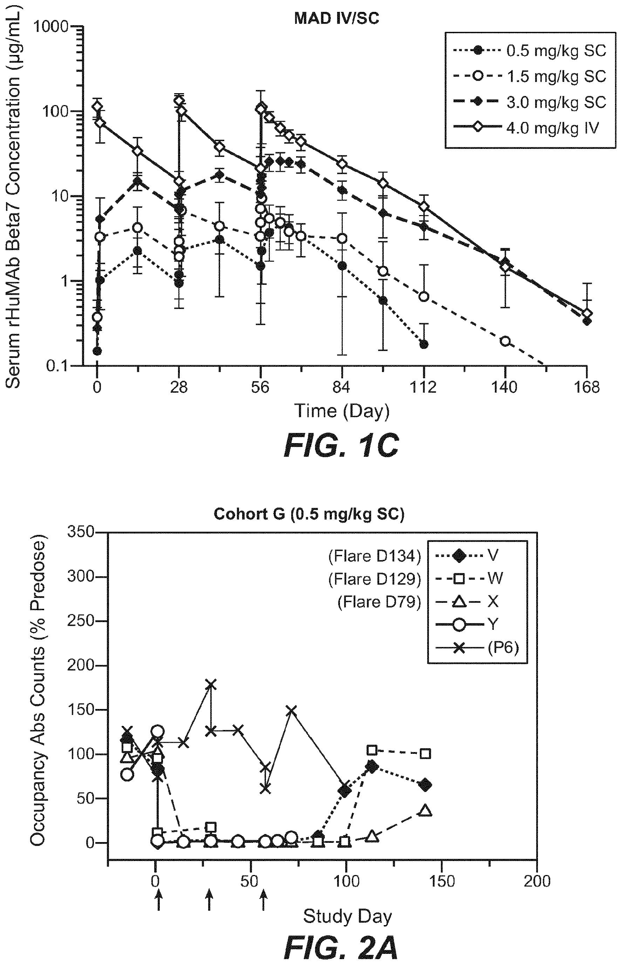

Figure 1 shows group mean serum concentration-time profiles of rhuMAb Beta7 in the Phase I study as described in Example 1. (A) Serum rhuMAb Beta7 in patients in the single ascending dose stage of the study who received study drug IV; (B) Serum rhuMAb Beta7 in patients in the single ascending dose stage of the study who received study drug at 3.0 mg/kg IV compared to patients who received the same dose of study drug SC; (C) Serum rhuMAb Beta7 in patients in the multiple ascending dose stage of the study. Each graph shows serum rhuMAb Beta7 concentration (µg/mL) on the vertical axis and time (days) on the horizontal axis.

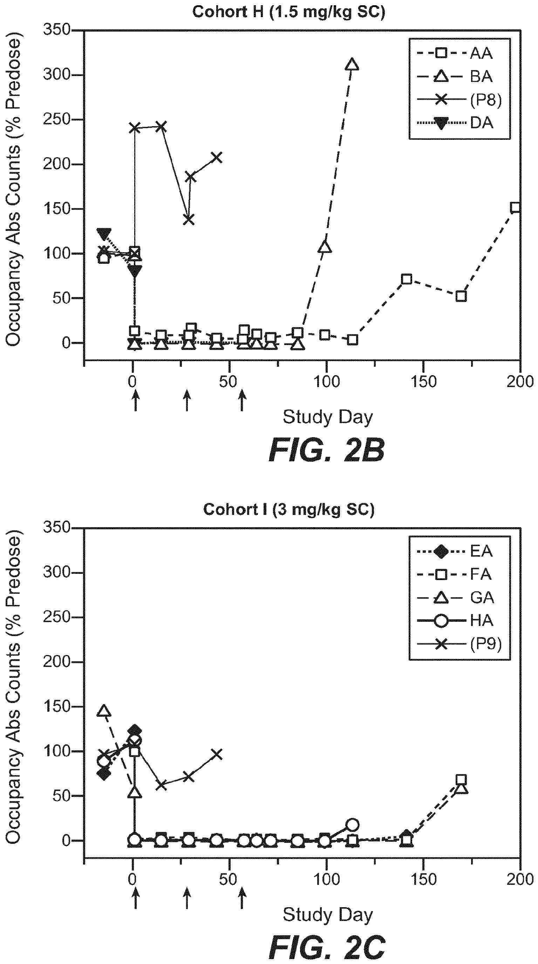

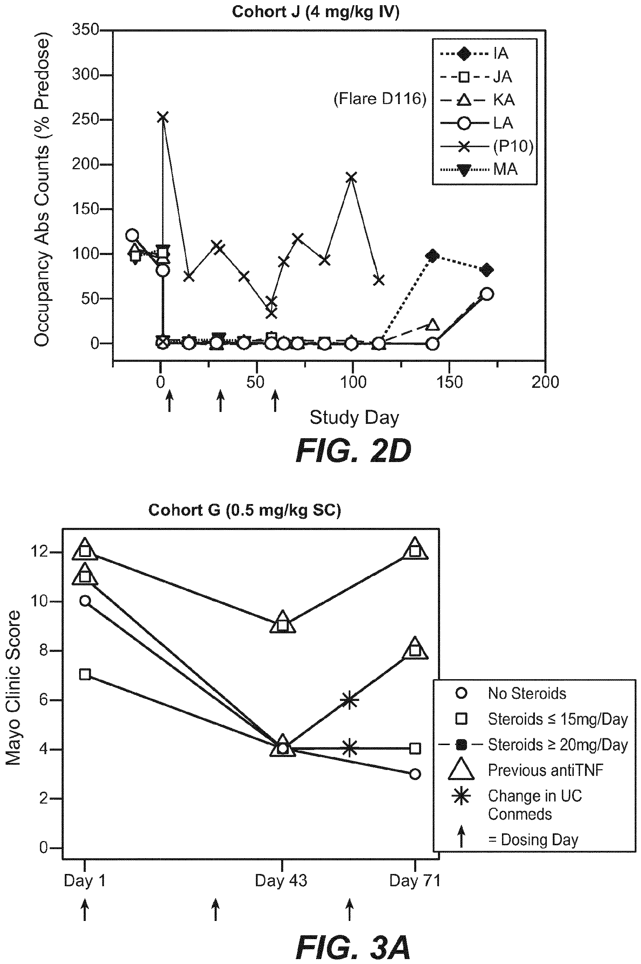

Figure 2 shows integrin beta 7 occupancy as described in Example 1. (A) Occupancy in samples from each patient in cohort G; four patients (V, W, X, Y) received 0.5 mg/kg rhuMAb beta7 SC while one received placebo (P6); (B) Occupancy in samples from each patient in cohort H; three patients (AA, BA, DA) received 1.5 mg/kg rhuMAb beta7 SC while one received placebo (P8); (C) Occupancy in samples from each patient in cohort I; four patients (EA, FA, GA, HA) received 3.0 mg/kg rhuMAb beta7 SC while one received placebo (P9); (D) Occupancy in samples from each patient in cohort J; five patients (IA, JA, KA, LA, MA) received 4.0 mg/kg rhuMAb beta7 IV while one received placebo (P10). Each graph shows occupancy on the vertical axis and study day on the horizontal axis. Certain patients had flares during the study and this is indicated in parentheses by each patient with the day of the flare indicated.

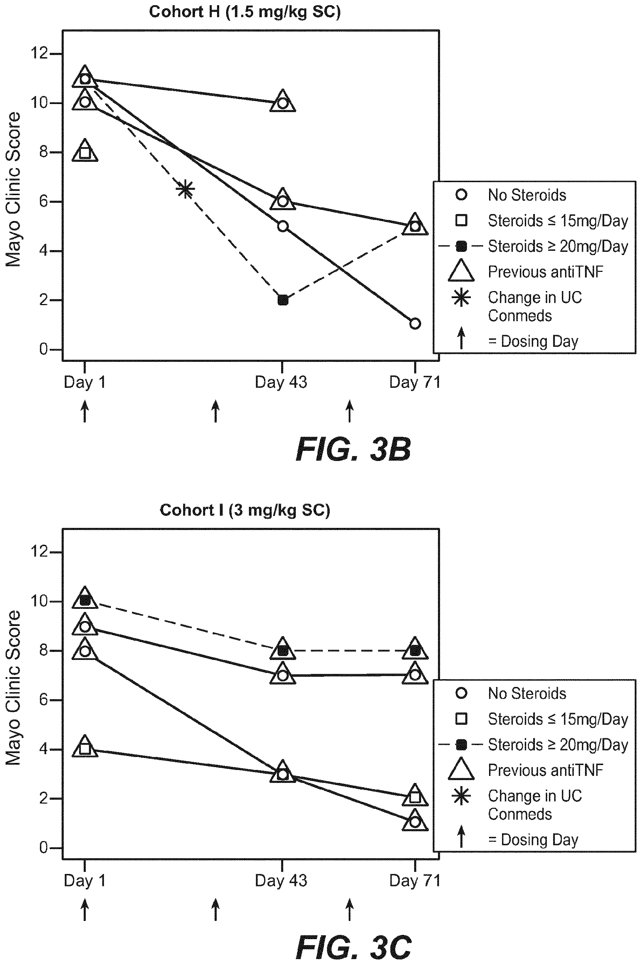

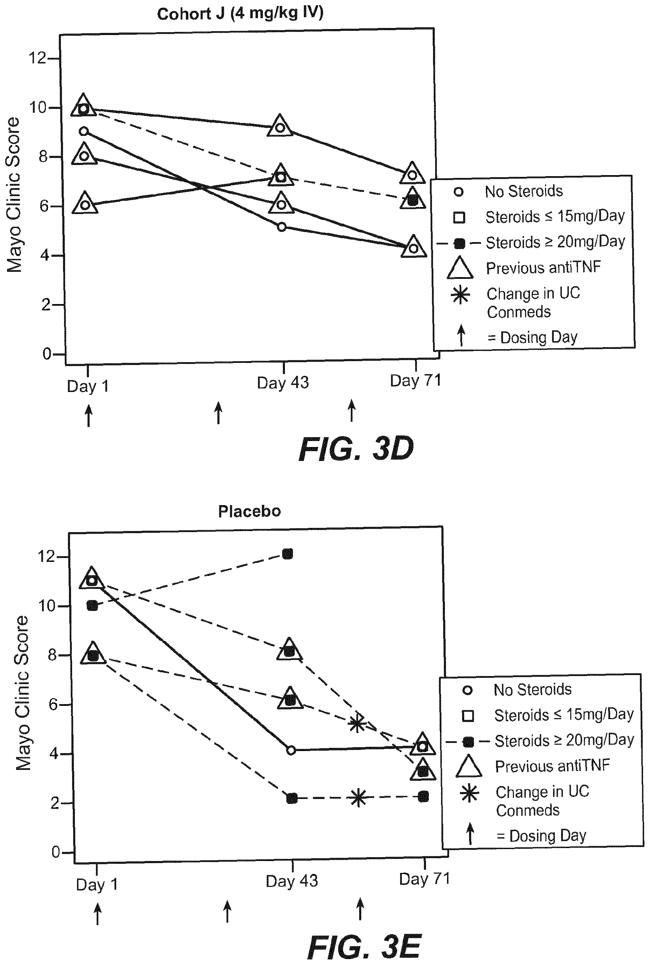

Figure 3 shows individual changes in Mayo Clinic Score as described in Example 1. (A) Cohort G (0.5 mg/kg SC); (B) Cohort H (1.5 mg/kg SC); (C) Cohort I (3 mg/kg SC); (D) Cohort J (4 mg/kg IV); (E) placebo. For each graph, Mayo Clinic Score is shown on the vertical axis and time (days) is shown on the horizontal axis. Arrows indicate days on which study drug (A-D) or placebo (E) was administered.

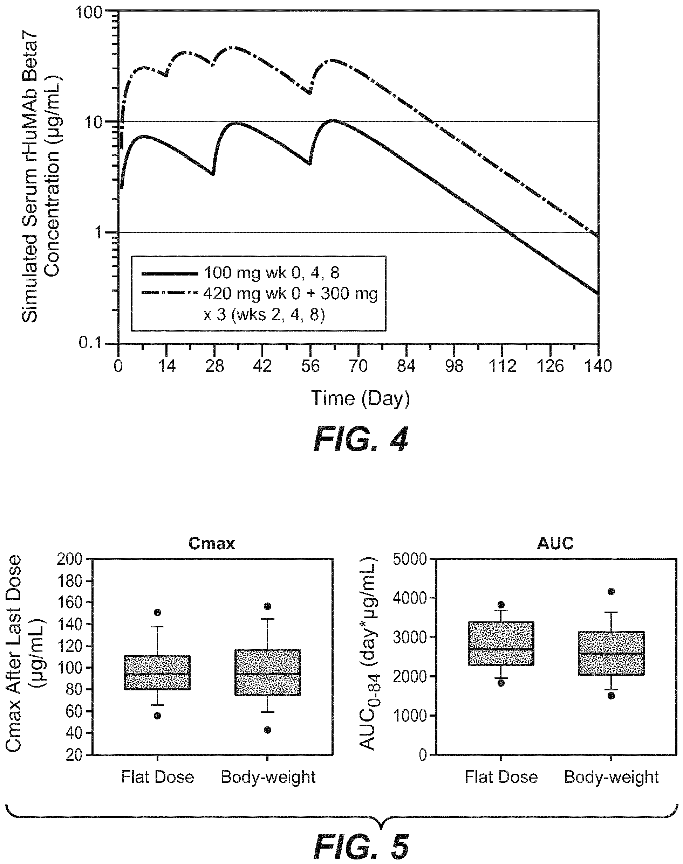

Figure 4 shows the serum rhuMAb Beta7 concentration-time profile (median level) simulated using a pharmacokinetic model derived from preliminary Phase I pharmacokinetic data as described in Example 2. Simulated serum rhuMAb Beta7 concentration (µg/mL) is on the vertical axis; time (days) is on the horizontal axis.
Figure 5 shows a comparison of population simulation-predicted steady-state exposure (Cₘₐₓ and AUC₀₋₈₄) and variability for body weight-based versus flat dosing as described in Example 2.

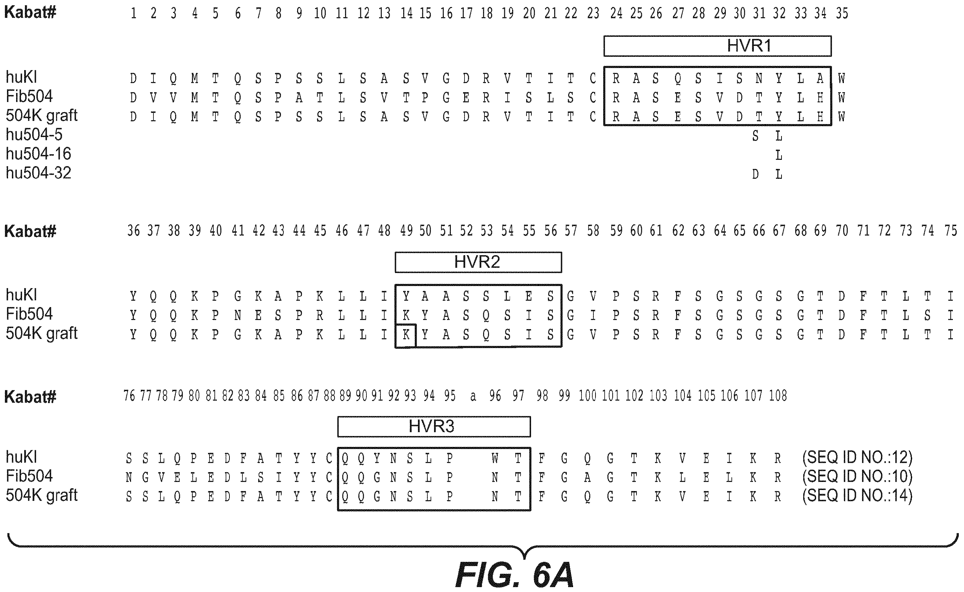

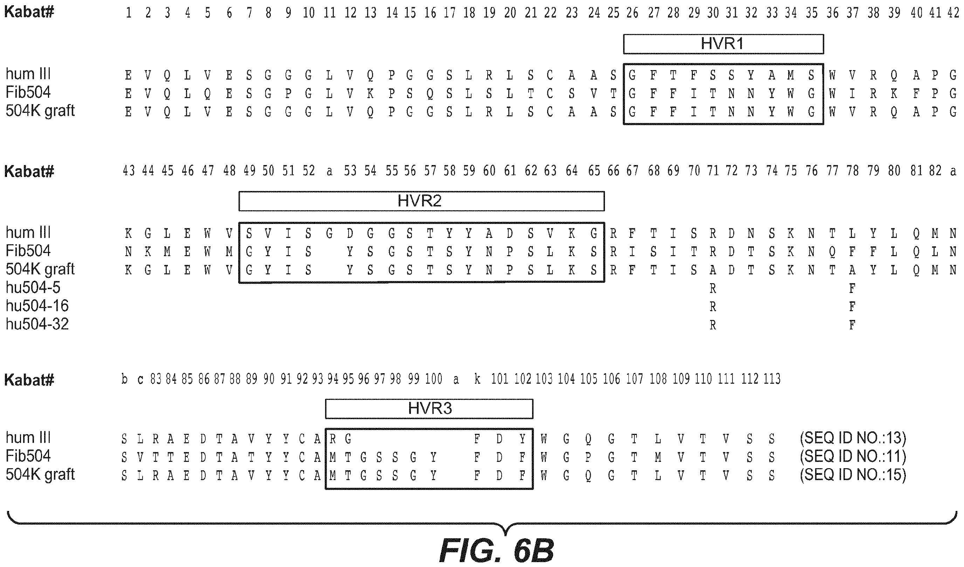

Figure 6A and 6B shows alignment of sequences of the variable light and heavy chains for the following: light chain human subgroup kappa I consensus sequence (FIG. 6A, SEQ ID NO:12), heavy chain human subgroup III consensus sequence (FIG. 6B, SEQ ID NO:13), rat anti-mouse beta7 antibody (Fib504) variable light chain (FIG. 6A, SEQ ID NO:10), rat anti-mouse beta7 antibody (Fib504) variable heavy chain (FIG. 6B, SEQ ID NO:11), and humanized antibody variants: Humanized hu504Kgraft variable light chain (FIG. 6A, SEQ ID NO:14), humanized hu504K graft variable heavy chain (FIG. 6B, SEQ ID NO:15), variants hu504-5, hu504-16, and hu504-32 (amino acid variations from humanized hu504K graft are indicated in FIG. 6A) (light chain) (SEQ ID NOS:22-24, respectively, in order of appearance) and FIG. 6B (heavy chain) for variants hu504-5, hu504-16, and hu504-32 (SEQ ID NO:25).

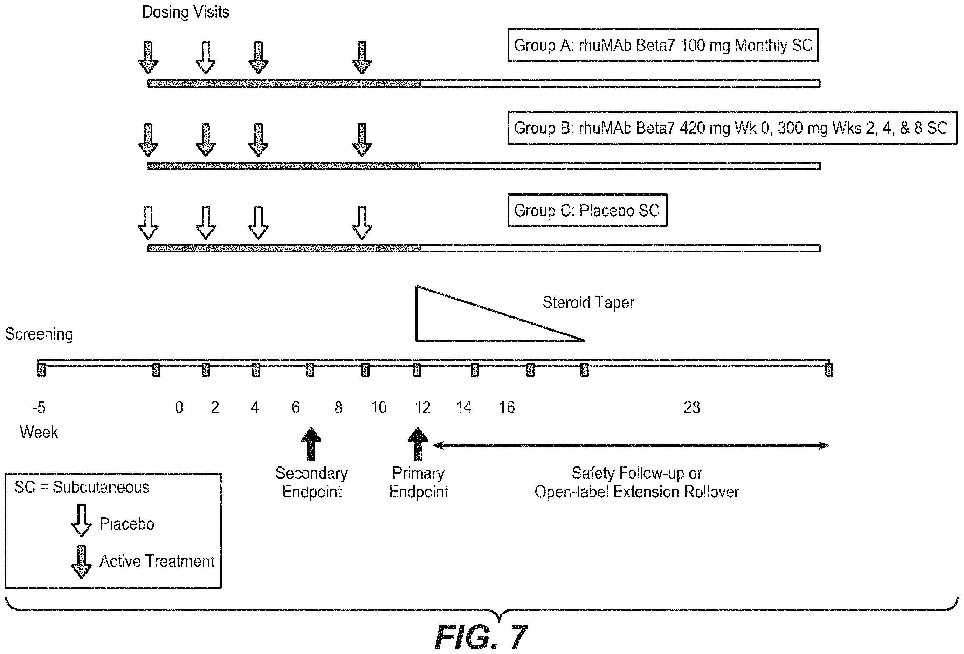

Figure 7 shows the study schema for the Phase II clinical study as described in Example 2.

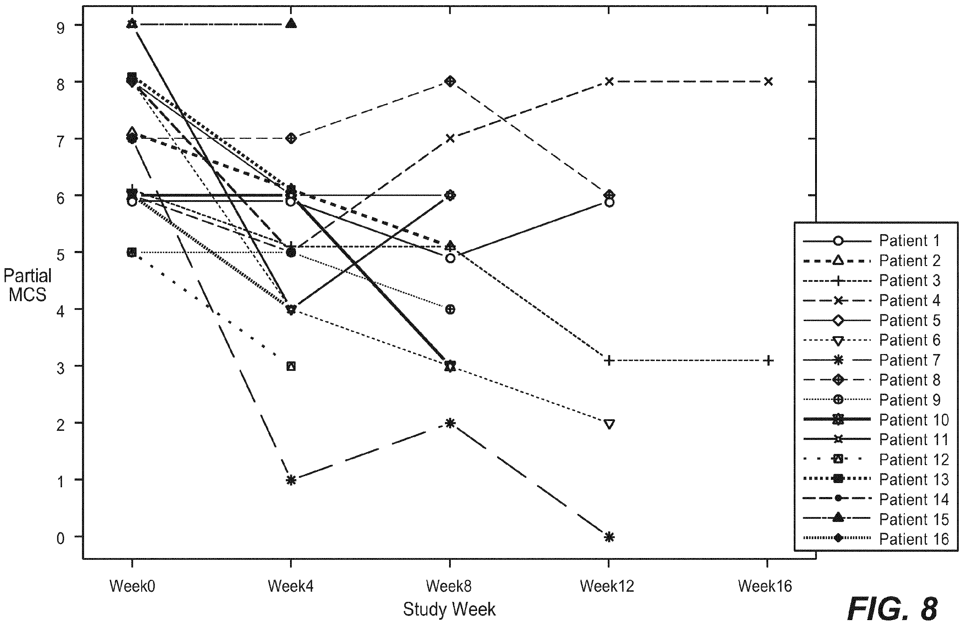

Figure 8 shows the partial Mayo Clinic Score (pMCS) for patients with moderate to severe ulcerative colitis who have entered the open label extension study as described in Example 2.

### DETAILED DESCRIPTION

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application.

### CERTAIN DEFINITIONS

For purposes of interpreting this specification, the following definitions will apply and whenever appropriate, terms used in the singular will also include the plural and vice versa. In the event that any definition set forth below conflicts with any document incorporated herein by reference, the definition set forth below shall control.

As used in this specification and the appended claims, the singular forms "a," "an" and "the" include plural referents unless the context clearly dictates otherwise. Thus, for example, reference to "a protein" includes a plurality of proteins; reference to "a cell" includes mixtures of cells, and the like.

Ranges provided in the specification and appended claims include both end points and all points between the end points. Thus, for example, a range of 2.0 to 3.0 includes 2.0, 3.0, and all points between 2.0 and 3.0.

"Treatment," "treating," and grammatical variations thereof refer to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, decreasing the rate of disease progression, amelioration or palliation of the disease state, and remission or improved prognosis.

"Treatment regimen" refers to a combination of dosage, frequency of administration, or duration of treatment, with or without addition of a second medication.

"Effective treatment regimen" refers to a treatment regimen that will offer beneficial response to a patient receiving the treatment.

"Modifying a treatment" refers to changing the treatment regimen including, changing dosage, frequency of administration, or duration of treatment, and/or addition of a second medication.

"Patient response" or "patient responsiveness" can be assessed using any endpoint indicating a benefit to the patient, including, without limitation, (1) inhibition, to some extent, of disease progression, including slowing down and complete arrest; (2) reduction in the number of disease episodes and/or symptoms; (3) reduction in lesional size; (4) inhibition (*i.e.,* reduction, slowing down or complete stopping) of disease cell infiltration into adjacent peripheral organs and/or tissues; (5) inhibition (*i.e.,* reduction, slowing down or complete stopping) of disease spread; (6) decrease of auto-immune response, which may, but does not have to, result in the regression or ablation of the disease lesion; (7) relief, to some extent, of one or more symptoms associated with the disorder; (8) increase in the length of disease-free presentation following treatment; and/or (9) decreased mortality at a given point of time following treatment. The term "responsiveness" refers to a measurable response, including complete response (CR) and partial response (PR).

As used herein, "complete response" or "CR" means the disappearance of all signs of inflammation or remission in response to treatment. This does not necessarily mean the disease has been cured.

"Partial response" or "PR" refers to a decrease of at least 50% in the severity of inflammation, in response to treatment.

A "beneficial response" of a patient to treatment with an integrin beta7 antagonist and similar wording refers to the clinical or therapeutic benefit imparted to a patient at risk for or suffering from a gastrointestinal inflammatory disorder from or as a result of the treatment with the antagonist, such as an anti-beta7 integrin antibody. Such benefit includes cellular or biological responses, a complete response, a partial response, a stable disease (without progression or relapse), or a response with a later relapse of the patient from or as a result of the treatment with the antagonist.

"A patient maintains responsiveness to a treatment" when the patient' responsiveness does not decrease with time during the course of a treatment.

The term "sample", as used herein, refers to a composition that is obtained or derived from a subject of interest that contains a cellular and/or other molecular entity that is to be characterized and/or identified, for example based on physical, biochemical, chemical and/or physiological characteristics. For example, the phrase "disease sample" and variations thereof refers to any sample obtained from a subject of interest that would be expected or is known to contain the cellular and/or molecular entity that is to be characterized. The sample can be obtained from a tissue for the subject of interest or from peripheral blood of the subject.

"A beta7 integrin antagonist" or "beta7 antagonist" refers to any molecule that inhibits one or more biological activities or blocking binding of beta7 integrin with one or more of its associated molecules. Antagonists of the invention can be used to modulate one or more aspects of beta7 associated effects, including but not limited to association with alpha4 integrin subunit, association with alphaE integrin subunit, binding of alpha4beta7 integrin to MAdCAM, VCAM-1 or fibronectin and binding of alphaEbeta7 integrin to E-cadherin. These effects can be modulated by any biologically relevant mechanism, including disruption of ligand binding to beta7 subunit or to the alpha4beta7 or alphaEbeta7 dimeric integrin, and/or by disrupting association between the alpha and beta integrin subunits such that formation of the dimeric integrin is inhibited. In one embodiment of the invention, the beta7 antagonist is an anti-beta7 integrin antibody (or anti-beta7 antibody). In one embodiment, the anti-beta7 integrin antibody is a humanized anti-beta7 integrin antibody and more particularly a recombinant humanized monoclonal anti-beta7 antibody (or rhuMAb beta7). In some embodiments, the anti-beta7 antibodies of the present invention are anti-integrin beta7 antagonistic antibodies that inhibit or block the binding of beta7 subunit with alpha4 integrin subunit, association with alphaE integrin subunit, binding of alpha4beta7 integrin to MAdCAM, VCAM-1 or fibronectin and binding of alphaEbeta7 integrin to E-cadherin.

By "beta7 subunit" or "β7 subunit" is meant the human β7 integrin subunit (Erle et al., (1991) J. Biol. Chem. 266:11009-11016). The beta7 subunit associates with alpha4 integrin subunit, such as the human .alpha.4 subunit (Kilger and Holzmann (1995) J. Mol. Biol. 73:347-354). The alpha4beta7 integrin is reportedly expressed on a majority of mature lymphocytes, as well as a small population of thymocytes, bone marrow cells and mast cells. (Kilshaw and Murant (1991) Eur. J. Immunol. 21:2591-2597; Gurish *et al.,* (1992) 149: 1964-1972; and Shaw, S. K. and Brenner, M. B. (1995) Semin. Immunol. 7:335). The beta7 subunit also associates with the alphaE subunit, such as the human alphaE integrin subunit (Cepek, K. L, et al. (1993) J. Immunol. 150:3459). The alphaEbeta7 integrin is expressed on intra-intestinal epithelial lymphocytes (iIELs) (Cepek, K. L. (1993) *supra*).

By "alphaE subunit" or "alphaE integrin subunit" or "αE subunit" or "αE integrin subunit" or "CD103" is meant an integrin subunit found to be associated with beta7 integrin on intra-epithelial lymphocytes, which alphaEbeta7 integrin mediates binding of the iELs to intestinal epithelium expressing E-cadherin (Cepek, K. L. et al. (1993) J. Immunol. 150:3459; Shaw, S. K. and Brenner, M. B. (1995) Semin. Immunol. 7:335).

"MAdCAM" or "MAdCAM-1" are used interchangeably in the context of the present invention and refer to the protein mucosal addressin cell adhesion molecule-1, which is a single chain polypeptide comprising a short cytoplasmic tail, a transmembrane region and an extracellular sequence composed of three immunoglobulin-like domains. The cDNAs for murine, human and macaque MAdCAM-1 have been cloned (Briskin, et al., (1993) Nature, 363:461-464; Shyjan et al., (1996) J. Immunol. 156:2851-2857).

"VCAM-1" or "vascular cell adhesion molecule-1" "CD106" refers to a ligand of alpha4beta7 and alpha4beta1, expressed on activated endothelium and important in endothelial-leukocyte interactions such as binding and transmigration of leukocytes during inflammation.

"CD45" refers to a protein of the protein tyrosine phosphatase (PTP) family. PTPs are known to be signaling molecules that regulate a variety of cellular processes including cell growth, differentiation, mitotic cycle, and oncogenic transformation. This PTP contains an extracellular domain, a single transmembrane segment and two tandem intracytoplasmic catalytic domains, and thus belongs to receptor type PTP. This gene is specifically expressed in hematopoietic cells. This PTP has been shown to be an essential regulator of T- and B-cell antigen receptor signaling. It functions through either direct interaction with components of the antigen receptor complexes, or by activating various Src family kinases required for the antigen receptor signaling. This PTP also suppresses JAK kinases, and thus functions as a regulator of cytokine receptor signaling. Four alternatively spliced transcripts variants of this gene, which encode distinct isoforms, have been reported. (Tchilian EZ, Beverley PC (2002). "CD45 in memory and disease." Arch. Immunol. Ther. Exp. (Warsz.) 50 (2): 85-93. Ishikawa H, Tsuyama N, Abroun S, et al. (2004). "Interleukin-6, CD45 and the src-kinases in myeloma cell proliferation." Leuk. Lymphoma 44 (9):1477-81.

Various isoforms of CD45 exist: CD45RA, CD45RB, CD45RC, CD45RAB, CD45RAC, CD45RBC, CD45RO, CD45R (ABC). CD45 is also highly glycosylated. CD45R is the longest protein and migrates at 200 kDa when isolated from T cells. B cells also express CD45R with heavier glycosylation, bringing the molecular weight to 220 kDa, hence the name B220; B cell isoform of 220 kDa. B220 expression is not restricted to B cells and can also be expressed on activated T cells, on a subset of dendritic cells and other antigen presenting cells. Stanton T, Boxall S, Bennett A, et al. (2004). "CD45 variant alleles: possibly increased frequency of a novel exon 4 CD45 polymorphism in HIV seropositive Ugandans." Immunogenetics 56 (2): 107-10.

"Gut-homing lymphocytes" refer to a subgroup of lymphocytes having the characteristic of selectively homing to intestinal lymph nodes and tissues but not homing to peripheral lymph nodes and tissues. This subgroup of lymphocytes are characterized by an unique expression pattern of a combination of multiples cell surface molecules, including, but not limited to, the combination of CD4, CD45RA and Beta7. Typically, at least two subsets of peripheral blood CD4⁺ lymphocytes can be subdivided based on the markers of CD45RA and Beta7, CD45RA⁻β7^{high}, and CD45RA⁻β7^{low} CD4⁺ cells. CD45RA⁻β7^{high} CD4⁺ cells home preferentially to intestinal lymph nodes and tissues, whereas CD45RA⁻β7^{low} CD4⁺ cells home preferentially to peripheral lymph nodes and tissues (Rott *et al.* 1996; Rott *et al.* 1997; Williams *et al.* 1998; Rosé *et al.* 1998; Williams and Butcher 1997; Butcher *et al.* 1999). Gut-homing lymphocytes are therefore a distinctive subgroup of lymphocytes identified as CD45RA⁻β7^{high} CD4⁺ in a flow cytometry assay. The methods of identifying this group of lymphocytes are well-known in the art and also disclosed in detail in Examples of the present application.

As used herein with respect to a cell surface marker, the symbol "+" indicates a positive expression of a cell surface marker. For instance, CD4⁺ lymphocytes are a group of lymphocytes having CD4 expressed on their cell surfaces.

As used herein with respect to a cell surface marker, the symbol "-" indicates a negative expression of a cell surface marker. For instance, CD45RA⁻ lymphocytes are a group of lymphocytes having no CD45RA expressed on their cell surfaces.

As used herein with respect to the expression of a cell surface marker, the symbol "low" indicates a relatively low level of expression of a cell surface marker on lymphocytes, while "high" indicates a relatively high level of expression of a cell surface marker on lymphocytes. In a flow cytometry, the intensity of β7^{high} is at least about 10 or 100 fold higher than that of β7^{low}. Thus, as provided herein in exemplary embodiments, the CD45RA⁻β7^{low} CD4⁺ and CD45RA⁻β7^{high} CD4⁺ lymphocytes locate in distinct portions of a dot plot or histogram of a flow cytometry analysis where X-axis is the intensity of expression of CD45AR and Y-axis is the intensity of the expression of Beta7.

"Peripheral-homing lymphocytes" refer to a subgroup of lymphocytes having the characteristic of homing to peripheral lymph nodes and tissues and not homing to intestinal lymph nodes and tissues. In an exemplary embodiment, as explained above, Peripheral-homing lymphocytes are a distinctive group of lymphocytes identified as CD45RA⁻β7^{low} CD4⁺ cells in a flow cytometry assay. The methods of identifying this group of lymphocytes are known in the art.

"Gastrointestinal inflammatory disorders" are a group of chronic disorders that cause inflammation and/or ulceration in the mucous membrane. These disorders include, for example, inflammatory bowel disease (*e.g*., Crohn's disease, ulcerative colitis, indeterminate colitis and infectious colitis), mucositis (*e.g*., oral mucositis, gastrointestinal mucositis, nasal mucositis and proctitis), necrotizing enterocolitis and esophagitis.

"Inflammatory Bowel Disease" or "IBD" is used interchangeably herein to refer to diseases of the bowel that cause inflammation and/or ulceration and includes without limitation Crohn's disease and ulcerative colitis.

"Crohn's disease (CD)" and "ulcerative colitis (UC)" are chronic inflammatory bowel diseases of unknown etiology. Crohn's disease, unlike ulcerative colitis, can affect any part of the bowel. The most prominent feature Crohn's disease is the granular, reddish-purple edematous thickening of the bowel wall. With the development of inflammation, these granulomas often lose their circumscribed borders and integrate with the surrounding tissue. Diarrhea and obstruction of the bowel are the predominant clinical features. As with ulcerative colitis, the course of Crohn's disease may be continuous or relapsing, mild or severe, but unlike ulcerative colitis, Crohn's disease is not curable by resection of the involved segment of bowel. Most patients with Crohn's disease require surgery at some point, but subsequent relapse is common and continuous medical treatment is usual.

Crohn's disease may involve any part of the alimentary tract from the mouth to the anus, although typically it appears in the ileocolic, small-intestinal or colonic-anorectal regions. Histopathologically, the disease manifests by discontinuous granulomatomas, crypt abscesses, fissures and aphthous ulcers. The inflammatory infiltrate is mixed, consisting of lymphocytes (both T and B cells), plasma cells, macrophages, and neutrophils. There is a disproportionate increase in IgM- and IgG-secreting plasma cells, macrophages and neutrophils.

Anti-inflammatory drugs sulfasalazine and 5-aminosalisylic acid (5-ASA) are used for treating mildly active colonic Crohn's disease and are commonly prescribed in an attempt to maintain remission of the disease. Metroidazole and ciprofloxacin are similar in efficacy to sulfasalazine and are particularly prescribed for treating perianal disease. In more severe cases, corticosteroids are prescribed to treat active exacerbations and can sometimes maintain remission. Azathioprine and 6-mercaptopurine have also been used in patients who require chronic administration of corticosteroids. It has been suggested that these drugs may play a role in the long-term prophylaxis. Unfortunately, there can be a very long delay (up to six months) before onset of action in some patients. Antidiarrheal drugs can also provide symptomatic relief in some patients. Nutritional therapy or elemental diet can improve the nutritional status of patients and induce symptomatic improvement of acute disease, but it does not induce sustained clinical remissions. Antibiotics are used in treating secondary small bowel bacterial overgrowth and in treatment of pyogenic complications.

"Ulcerative colitis (UC)" afflicts the large intestine. The course of the disease may be continuous or relapsing, mild or severe. The earliest lesion is an inflammatory infiltration with abscess formation at the base of the crypts of Lieberkuhn. Coalescence of these distended and ruptured crypts tends to separate the overlying mucosa from its blood supply, leading to ulceration. Symptoms of the disease include cramping, lower abdominal pain, rectal bleeding, and frequent, loose discharges consisting mainly of blood, pus and mucus with scanty fecal particles. A total colectomy may be required for acute, severe or chronic, unremitting ulcerative colitis.

The clinical features of UC are highly variable, and the onset may be insidious or abrupt, and may include diarrhea, tenesmus and relapsing rectal bleeding. With fulminant involvement of the entire colon, toxic megacolon, a life-threatening emergency, may occur. Extraintestinal manifestations include arthritis, pyoderma gangrenoum, uveitis, and erythema nodosum.

Treatment for UC includes sulfasalazine and related salicylate-containing drugs for mild cases and corticosteroid drugs in severe cases. Topical administration of either salicylates or corticosteroids is sometimes effective, particularly when the disease is limited to the distal bowel, and is associated with decreased side effects compared with systemic use. Supportive measures such as administration of iron and antidiarrheal agents are sometimes indicated. Azathioprine, 6-mercaptopurine and methotrexate are sometimes also prescribed for use in refractory corticosteroid-dependent cases.

An "effective dosage" refers to an amount effective, at dosages and for periods of time necessary, to achieve the desired therapeutic or prophylactic result.

As used herein, the term "patient" refers to any single subject for which treatment is desired. In certain embodiments, the patient herein is a human.

A "subject" herein is typically a human. In certain embodiments, a subject is a non-human mammal. Exemplary non-human mammals include laboratory, domestic, pet, sport, and stock animals, e.g., mice, cats, dogs, horses, and cows. Typically, the subject is eligible for treatment, e.g., treatment of a gastrointestinal inflammatory disorder.

As used herein, "lifetime" of a subject refers to the remainder of the life of the subject after starting treatment.

The terms "antibody" and "immunoglobulin" are used interchangeably in the broadest sense and include monoclonal antibodies (for example, full length or intact monoclonal antibodies), polyclonal antibodies, multivalent antibodies, multispecific antibodies (e.g., bispecific antibodies so long as they exhibit the desired biological activity) and may also include certain antibody fragments (as described in greater detail herein). An antibody can be human, humanized and/or affinity matured.

"Antibody fragments" comprise only a portion of an intact antibody, wherein the portion preferably retains at least one, and typically most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an *in vivo* half life substantially similar to an intact antibody. For example, such an antibody fragment may comprise on antigen binding arm linked to an Fc sequence capable of conferring *in vivo* stability to the fragment.

The term "monoclonal antibody" as used herein refers to an antibody obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (*e.g*., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin lo sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1: 105-115 (1998); Harris, Biochem. Soc. Transactions 23:1035-1038 (1995); Hurle and Gross, Curr. Op. Biotech. 5:428-433 (1994).

A "human antibody" is one which comprises an amino acid sequence corresponding to that of an antibody produced by a human and/or has been made using any of the techniques for making human antibodies as disclosed herein. Such techniques include screening human-derived combinatorial libraries, such as phage display libraries (see, *e.g.,* Marks et al., J. Mol. Biol., 222: 581-597 (1991) and Hoogenboom et al., Nucl. Acids Res., 19: 4133-4137 (1991)); using human myeloma and mouse-human heteromyeloma cell lines for the production of human monoclonal antibodies (see, *e.g.,* Kozbor J. Immunol., 133: 3001 (1984); Brodeur et al., Monoclonal Antibody Production Techniques and Applications, pp. 55-93 (Marcel Dekker, Inc., New York, 1987); and Boerner et al., J. Immunol., 147: 86 (1991)); and generating monoclonal antibodies in transgenic animals (*e.g.*, mice) that are capable of producing a full repertoire of human antibodies in the absence of endogenous immunoglobulin production (see, *e.g.,* Jakobovits et al., Proc. Natl. Acad. Sci USA, 90: 2551 (1993); Jakobovits et al., Nature, 362: 255 (1993); Bruggermann et al., Year in Immunol., 7: 33 (1993)). This definition of a human antibody specifically excludes a humanized antibody comprising antigen-binding residues from a non-human animal.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In certain embodiments, the antibody will be purified (1) to greater than 95% by weight of antibody as determined by the Lowry method, and often more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or silver stain. Isolated antibody includes the antibody in situ within recombinant cells since at least one component of the antibody's natural environment will not be present. Ordinarily, however, isolated antibody will be prepared by at least one purification step.

The term "hypervariable region," "HVR," or "HV," when used herein refers to the regions of an antibody variable domain which are hypervariable in sequence and/or form structurally defined loops. Generally, antibodies comprise six hypervariable regions; three in the VH (HI, H2, H3), and three in the VL (L1, L2, L3). A number of hypervariable region delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, Md. (1991)). Chothia refers instead to the location of the structural loops (Chothia and Lesk J. Mol. Biol. 196:901-917 (1987)). The AbM hypervariable regions represent a compromise between the Kabat CDRs and Chothia structural loops, and are used by Oxford Molecular's AbM antibody modeling software. The "contact" hypervariable regions are based on an analysis of the available complex crystal structures. The residues from each of these HVRs are noted below.

| Loop | Kabat | AbM | Chothia | Contact | |
|---|---|---|---|---|---|
| L1 | L24-L34 | L24-L34 | L26-L32 | L30-L36 | |
| L2 | L50-L56 | L50-L56 | L50-L52 | L46-L55 | |
| L3 | L89-L97 | L89-L97 | L91-L96 | L89-L96 | |
| H1 | H31-H35B | H26-H35B | H26-H32 | H30-H35B | (Kabat Numbering) |
| H1 | H31-H35 | H26-H35 | H26-H32 | H30-H35 | (Chothia Numbering) |
| H2 | H50-H65 | H50-H58 | H53-H55 | H47-H58 | |
| H3 | H95-H102 | H95-H102 | H96-H101 | H93-H101 | |

Hypervariable regions may comprise "extended hypervariable regions" as follows: 24-36 or 24-34 (L1), 46-56 or 49-56 or 50-56 or 52-56 (L2) and 89-97 (L3) in the VL and 26-35 (H1), 50-65 or 49-65 (H2) and 93-102, 94-102 or 95-102 (H3) in the VH. The variable domain residues are numbered according to Kabat *et al., supra* for each of these definitions.

"Framework" or "FR" residues are those variable domain residues other than the hypervariable region residues as herein defined.

A "human consensus framework" is a framework which represents the most commonly occurring amino acid residue in a selection of human immunoglobulin VL or VH framework sequences. Generally, the selection of human immunoglobulin VL or VH sequences is from a subgroup of variable domain sequences. Generally, the subgroup of sequences is a subgroup as in Kabat *et al.* In one embodiment, for the VL, the subgroup is subgroup kappa I as in Kabat *et al.* In one embodiment, for the VH, the subgroup is subgroup III as in Kabat *et al.*

An "affinity matured" antibody is one with one or more alterations in one or more CDRs thereof which result in an improvement in the affinity of the antibody for antigen, compared to a parent antibody which does not possess those alteration(s). In certain embodiments, affinity matured antibodies will have nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1996); Yelton et al. J. Immunol. 155:1994-2004 (1995); Jackson et al., J. Immunol. 154(7):3310-9 (1995); and Hawkins et al. J. Mol. Biol. 226:889-896 (1992).

The phrase "substantially similar," or "substantially the same," as used herein, denotes a sufficiently high degree of similarity between two numeric values (generally one associated with an antibody of the invention and the other associated with a reference/comparator antibody) such that one of skill in the art would consider the difference between the two values to be of little or no biological and/or statistical significance within the context of the biological characteristic measured by said values (*e.g.,* Kd values). The difference between said two values is less than about 50%, less than about 40%, less than about 30%, less than about 20%, less than about 10% as a function of the value for the reference/comparator antibody.

"Binding affinity" generally refers to the strength of the sum total of noncovalent interactions between a single binding site of a molecule *(e.g.,* an antibody) and its binding partner (*e.g*., an antigen). Unless indicated otherwise, as used herein, "binding affinity" refers to intrinsic binding affinity which reflects a 1:1 interaction between members of a binding pair (*e.g.*, antibody and antigen). The affinity of a molecule X for its partner Y can generally be represented by the dissociation constant (Kd). Affinity can be measured by common methods known in the art, including those described herein. Low-affinity antibodies generally bind antigen slowly and tend to dissociate readily, whereas high-affinity antibodies generally bind antigen faster and tend to remain bound longer. A variety of methods of measuring binding affinity are known in the art, any of which can be used for purposes of the present invention.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-binding sites and is still capable of cross-linking antigen.

"Fv" is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab= fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')₂ antibody fragments originally were produced as pairs of Fab' fragments which have hinge cysteines between them. Other chemical couplings of antibody fragments are also known.

The "light chains" of antibodies from any vertebrate species can be assigned to one of two clearly distinct types, called kappa (κ) and lambda (λ), based on the amino acid sequences of their constant domains.

Depending on the amino acid sequences of the constant domains of their heavy chains, antibodies (immunoglobulins) can be assigned to different classes. There are five major classes of immunoglobulins: IgA, IgD, IgE, IgG, and IgM, and several of these may be further divided into subclasses (isotypes), *e.g.,* IgG₁, IgG₂, IgG₃, IgG₄, IgA₁, and IgA₂. The heavy-chain constant domains that correspond to the different classes of immunoglobulins are called α, δ, ε, γ, and µ, respectively. The subunit structures and three-dimensional configurations of different classes of immunoglobulins are well known and described generally in, for example, Abbas et al. Cellular and Mol. Immunology, 4th ed. (W. B. Saunders, Co., 2000). An antibody may be part of a larger fusion molecule, formed by covalent or non-covalent association of the antibody with one or more other proteins or peptides.

The terms "full-length antibody," "intact antibody," and "whole antibody" are used herein interchangeably to refer to an antibody in its substantially intact form, not antibody fragments as defined below. The terms particularly refer to an antibody with heavy chains that contain an Fc region.

A "naked antibody" for the purposes herein is an antibody that is not conjugated to a cytotoxic moiety or radiolabel.

The term "Fc region" herein is used to define a C-terminal region of an immunoglobulin heavy chain, including native sequence Fc regions and variant Fc regions. Although the boundaries of the Fc region of an immunoglobulin heavy chain might vary, the human IgG heavy chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue.

Unless indicated otherwise, herein the numbering of the residues in an immunoglobulin heavy chain is that of the EU index as in Kabat et al., Sequences of Proteins of Immunological Interest, 5th Ed. Public Health Service, National Institutes of Health, Bethesda, MD (1991), expressly incorporated herein by reference. The "EU index as in Kabat" refers to the residue numbering of the human IgG1 EU antibody.

A "functional Fc region" possesses an "effector function" of a native sequence Fc region. Exemplary "effector functions" include C1q binding; complement dependent cytotoxicity; Fc receptor binding; antibody-dependent cell-mediated cytotoxicity (ADCC); phagocytosis; down regulation of cell surface receptors *(e.g.,* B cell receptor; BCR), etc. Such effector functions generally require the Fc region to be combined with a binding domain *(e.g.,* an antibody variable domain) and can be assessed using various assays as herein disclosed, for example.

A "native sequence Fc region" comprises an amino acid sequence identical to the amino acid sequence of an Fc region found in nature. Native sequence human Fc regions include a native sequence human IgG1 Fc region (non-A and A allotypes); native sequence human IgG2 Fc region; native sequence human IgG3 Fc region; and native sequence human IgG4 Fc region as well as naturally occurring variants thereof.

A "variant Fc region" comprises an amino acid sequence which differs from that of a native sequence Fc region by virtue of at least one amino acid modification. In certain embodiments, the variant Fc region has at least one amino acid substitution compared to a native sequence Fc region or to the Fc region of a parent polypeptide, e.g., from about one to about ten amino acid substitutions, and in certain embodiments from about one to about five amino acid substitutions in a native sequence Fc region or in the Fc region of the parent polypeptide. In certain embodiments, the variant Fc region herein will possess at least about 80% homology with a native sequence Fc region and/or with an Fc region of a parent polypeptide, or at least about 90% homology therewith, or at least about 95% homology therewith.

Depending on the amino acid sequence of the constant domain of their heavy chains, intact antibodies can be assigned to different "classes." There are five major classes of intact antibodies: IgA, IgD, IgE, IgG, and IgM, and several of these may be further divided into "subclasses" (isotypes), *e.g.,* IgG1, IgG2, IgG3, IgG4, IgA, and IgA2. The heavy-chain constant domains that correspond to the different classes of antibodies are called α, δ, ε, γ, and µ, respectively. The subunit structures and three-dimensional configurations of different classes of immunoglobulins are well known.

"Antibody-dependent cell-mediated cytotoxicity" and "ADCC" refer to a cell-mediated reaction in which nonspecific cytotoxic cells that express Fc receptors (FcRs) (*e.g.* Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcyRI, FcyRII and FcγRIII. FcR expression on hematopoietic cells in summarized is Table 3 on page 464 of Ravetch and Kinet, Annu. Rev. Immunol 9:457-92 (1991). To assess ADCC activity of a molecule of interest, an *in vitro* ADCC assay, such as that described in U.S. Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. PNAS (USA) 95:652-656 (1998).

"Human effector cells" are leukocytes which express one or more FcRs and perform effector functions. In certain embodiments, the cells express at least FcγRIII and perform ADCC effector function. Examples of human leukocytes which mediate ADCC include peripheral blood mononuclear cells (PBMC), natural killer (NK) cells, monocytes, cytotoxic T cells and neutrophils. The effector cells may be isolated from a native source thereof, *e.g*., from blood or PBMCs as described herein.

The terms "Fc receptor" or "FcR" are used to describe a receptor that binds to the Fc region of an antibody. In certain embodiments, FcR is a native sequence human FcR. Moreover, FcR is one which binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain (see review M. in Daëron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)), and regulates homeostasis of immunoglobulins. Antibodies with improved binding to the neonatal Fc receptor (FcRn), and increased half-lives, are described in WO00/42072 (Presta, L.) and US2005/0014934A1 (Hinton et al.). These antibodies comprise an Fc region with one or more substitutions therein which improve binding of the Fc region to FcRn. For example, the Fc region may have substitutions at one or more of positions 238, 250, 256, 265, 272, 286, 303, 305, 307, 311, 312, 314, 317, 340, 356, 360, 362, 376, 378, 380, 382, 413, 424, 428 or 434 (Eu numbering of residues). In certain embodiments, the Fc region-comprising antibody variant with improved FcRn binding comprises amino acid substitutions at one, two or three of positions 307, 380 and 434 of the Fc region thereof (Eu numbering of residues).

"Single-chain Fv" or "scFv" antibody fragments comprise the V_{H} and V_{L} domains of antibody, wherein these domains are present in a single polypeptide chain. In certain embodiments, the Fv polypeptide further comprises a polypeptide linker between the V_{H} and V_{L} domains which enables the scFv to form the desired structure for antigen binding. For a review of scFv see Plückthun in The Pharmacology of Monoclonal Antibodies, vol. 113, Rosenburg and Moore eds., Springer-Verlag, New York, pp. 269-315 (1994). HER2 antibody scFv fragments are described in WO93/16185; U.S. Patent No. 5,571,894; and U.S. Patent No. 5,587,458.

The term "diabodies" refers to small antibody fragments with two antigen-binding sites, which fragments comprise a variable heavy domain (V_{H}) connected to a variable light domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "affinity matured" antibody is one with one or more alterations in one or more hypervariable regions thereof which result an improvement in the affinity of the antibody for antigen, compared to a parent antibody which does not possess those alteration(s). In certain embodiments, affinity matured antibodies will have nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

An "amino acid sequence variant" antibody herein is an antibody with an amino acid sequence which differs from a main species antibody. In certain embodiments, amino acid sequence variants will possess at least about 70% homology with the main species antibody, or they will be at least about 80%, or at least about 90% homologous with the main species antibody. The amino acid sequence variants possess substitutions, deletions, and/or additions at certain positions within or adjacent to the amino acid sequence of the main species antibody. Examples of amino acid sequence variants herein include an acidic variant (e.g., deamidated antibody variant), a basic variant, an antibody with an amino-terminal leader extension (*e.g*. VHS-) on one or two light chains thereof, an antibody with a C-terminal lysine residue on one or two heavy chains thereof, etc, and includes combinations of variations to the amino acid sequences of heavy and/or light chains. The antibody variant of particular interest herein is the antibody comprising an amino-terminal leader extension on one or two light chains thereof, optionally further comprising other amino acid sequence and/or glycosylation differences relative to the main species antibody.

A "glycosylation variant" antibody herein is an antibody with one or more carbohydrate moieties attached thereto which differ from one or more carbohydrate moieties attached to a main species antibody. Examples of glycosylation variants herein include antibody with a G1 or G2 oligosaccharide structure, instead a G0 oligosaccharide structure, attached to an Fc region thereof, antibody with one or two carbohydrate moieties attached to one or two light chains thereof, antibody with no carbohydrate attached to one or two heavy chains of the antibody, etc, and combinations of glycosylation alterations. Where the antibody has an Fc region, an oligosaccharide structure may be attached to one or two heavy chains of the antibody, *e.g.* at residue 299 (298, Eu numbering of residues).

The term "cytotoxic agent" as used herein refers to a substance that inhibits or prevents the function of cells and/or causes destruction of cells. The term is intended to include radioactive isotopes (*e.g*. At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, and toxins such as small molecule toxins or enzymatically active toxins of bacterial, fungal, plant or animal origin, including fragments and/or variants thereof.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-a and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon-α, -β, and -y; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1α, IL-2, IL-3, IL-4, IL-5, IL-6, IL-7, IL-8, IL-9, IL-10, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

The term "immunosuppressive agent" as used herein for adjunct therapy refers to substances that act to suppress or mask the immune system of the subject being treated herein. This would include substances that suppress cytokine production, down-regulate or suppress self-antigen expression, or mask the MHC antigens. Examples of such agents include 2-amino-6-aryl-5-substituted pyrimidines (see U.S. Patent No. 4,665,077); non-steroidal anti-inflammatory drugs (NSAIDs); ganciclovir; tacrolimus; glucocorticoids such as cortisol or aldosterone; anti-inflammatory agents such as a cyclooxygenase inhibitor; a 5-lipoxygenase inhibitor; or a leukotriene receptor antagonist; purine antagonists such as azathioprine or mycophenolate mofetil (MMF); alkylating agents such as cyclophosphamide; bromocryptine; danazol; dapsone; glutaraldehyde (which masks the MHC antigens, as described in U.S. Patent No. 4,120,649); anti-idiotypic antibodies for MHC antigens and MHC fragments; cyclosporine; 6 mercaptopurine; steroids such as corticosteroids or glucocorticosteroids or glucocorticoid analogs, e.g., prednisone, methylprednisolone, including SOLU-MEDROL.RTM. methylprednisolone sodium succinate, and dexamethasone; dihydrofolate reductase inhibitors such as methotrexate (oral or subcutaneous); anti-malarial agents such as chloroquine and hydroxychloroquine; sulfasalazine; leflunomide; cytokine or cytokine receptor antibodies or antagonists including anti-interferon-alpha, -beta, or -gamma antibodies, anti-tumor necrosis factor(TNF)-alpha antibodies (infliximab (REMICADE.RTM.) or adalimumab), anti-TNF-alpha immunoadhesin (etanercept), anti-TNF-beta antibodies, anti-interleukin-2 (IL-2) antibodies and anti-IL-2 receptor antibodies, and anti-interleukin-6 (IL-6) receptor antibodies and antagonists; anti-LFA-1 antibodies, including anti-CDlla and anti-CD18 antibodies; anti-L3T4 antibodies; heterologous anti-lymphocyte globulin; pan-T antibodies, anti-CD3 or anti-CD4/CD4a antibodies; soluble peptide containing a LFA-3 binding domain (WO 90/08187 published Jul. 26, 1990); streptokinase; transforming growth factor-beta (TGF-beta); streptodomase; RNA or DNA from the host; FK506; RS-61443; chlorambucil; deoxyspergualin; rapamycin; T-cell receptor (Cohen et al., U.S. Patent No. 5,114,721); T-cell receptor fragments (Offner et al., Science, 251: 430-432 (1991); WO 90/11294; Ianeway, Nature, 341: 482 (1989); and WO 91/01133); BAFF antagonists such as BAFF or BR3 antibodies or immunoadhesins and zTNF4 antagonists (for review, see Mackay and Mackay, Trends Immunol., 23:113-5 (2002) and see also definition below); biologic agents that interfere with T cell helper signals, such as anti-CD40 receptor or anti-CD40 ligand (CD154), including blocking antibodies to CD40-CD40 ligand. (*e.g*., Durie etal., Science, 261: 1328-30 (1993); Mohan et al., J. Immunol., 154: 1470-80 (1995)) and CTLA4-Ig (Finck et al., Science, 265: 1225-7 (1994)); and T-cell receptor antibodies (EP 340,109) such as T10B9.

The term "ameliorates" or "amelioration" as used herein refers to a decrease, reduction or elimination of a condition, disease, disorder, or phenotype, including an abnormality or symptom.

A "symptom" of a disease or disorder (e.g., inflammatory bowel disease, e.g., ulcerative colitis or Crohn's disease) is any morbid phenomenon or departure from the normal in structure, function, or sensation, experienced by a subject and indicative of disease.

The expression "therapeutically effective amount" refers to an amount that is effective for preventing, ameliorating, or treating a disease or disorder (e.g., inflammatory bowel disease, e.g., ulcerative colitis or Crohn's disease). For example, a "therapeutically effective amount" of an antibody refers to an amount of the antibody that is effective for preventing, ameliorating, or treating the specified disease or disorder. Similarly, a "therapeutically effective amount" of a combination of an antibody and a second compound refers to an amount of the antibody and an amount of the second compound that, in combination, is effective for preventing, ameliorating, or treating the specified disease or disorder.

It is to be understood that the terminology "a combination of' two compounds does not mean that the compounds have to be administered in admixture with each other. Thus, treatment with or use of such a combination encompasses a mixture of the compounds or separate administration of the compounds, and includes administration on the same day or different days. Thus the terminology "combination" means two or more compounds are used for the treatment, either individually or in admixture with each other. When an antibody and a second compound, for example, are administered in combination to a subject, the antibody is present in the subject at a time when the second compound is also present in the subject, whether the antibody and second compound are administered individually or in admixture to the subject. In certain embodiments, a compound other than the antibody is administered prior to the antibody. In certain embodiments, a compound other than the antibody is administered after the antibody.

For the purposes herein, "tumor necrosis factor-alpha (TNF-alpha)" refers to a human TNF-alpha molecule comprising the amino acid sequence as described in Pennica et al., Nature, 312:721 (1984) or Aggarwal et al., JBC, 260:2345 (1985).

A "TNF-alpha inhibitor" herein is an agent that inhibits, to some extent, a biological function of TNF-alpha, generally through binding to TNF-alpha and neutralizing its activity. Examples of TNF inhibitors specifically contemplated herein are etanercept (ENBREL®), infliximab (REMICADE®), adalimumab (HUMIRA®), golimumab (SIMPONITM), and certolizumab pegol (CIMZIA®).

"Corticosteroid" refers to any one of several synthetic or naturally occurring substances with the general chemical structure of steroids that mimic or augment the effects of the naturally occurring corticosteroids. Examples of synthetic corticosteroids include prednisone, prednisolone (including methylprednisolone), dexamethasone triamcinolone, and betamethasone.

An "antagonist" refers to a molecule capable of neutralizing, blocking, inhibiting, abrogating, reducing or interfering with the activities of a particular or specified protein, including its binding to one or more receptors in the case of a ligand or binding to one or more ligands in case of a receptor. Antagonists include antibodies and antigen-binding fragments thereof, proteins, peptides, glycoproteins, glycopeptides, glycolipids, polysaccharides, oligosaccharides, nucleic acids, bioorganic molecules, peptidomimetics, pharmacological agents and their metabolites, transcriptional and translation control sequences, and the like. Antagonists also include small molecule inhibitors of the protein, and fusion proteins, receptor molecules and derivatives which bind specifically to the protein thereby sequestering its binding to its target, antagonist variants of the protein, antisense molecules directed to the protein, RNA aptamers, and ribozymes against the protein.

A "self-inject device" refers to a medical device for self-administration, e.g., by a patient or in-home caregiver, of a therapeutic agent. Self-inject devices include autoinjector devices and other devices designed for self-administration.

A variety of additional terms are defined or otherwise characterized herein.

### COMPOSITIONS AND METHODS

### A. Beta7 integrin Antagonists

Methods of treating a gastrointestinal inflammatory disorder in a subject, e.g., a human, by administering beta7 integrin antagonists are provided. Examples of potential antagonists include an oligonucleotide that binds to the fusions of immunoglobulin with beta7 integrin, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the beta7 integrin that recognizes the ligand but imparts no effect, thereby competitively inhibiting the action of the beta7 integrin.

Another potential beta7 integrin antagonist is an antisense RNA or DNA construct prepared using antisense technology, where, *e.g.,* an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the beta7 integrin herein, is used to design an antisense RNA oligonucleotide of from about 10 to 40 base pairs in length. A DNA oligonucleotide is designed to be complementary to a region of the gene involved in transcription (triple helix--see Lee et al., Nucl. Acids Res., 6:3073 (1979); Cooney et al., Science, 241: 456 (1988); Dervan et al., Science, 251:1360 (1991)), thereby preventing transcription and the production of the beta7 integrin. The antisense RNA oligonucleotide hybridizes to the mRNA *in vivo* and blocks translation of the mRNA molecule into beta7 integrin protein (antisense--Okano, Neurochem., 56:560 (1991); Oligodeoxynucleotides as Antisense Inhibitors of Gene Expression (CRC Press: Boca Raton, Fla., 1988). The oligonucleotides described above can also be delivered to cells such that the antisense RNA or DNA may be expressed *in vivo* to inhibit production of the PRO polypeptide. When antisense DNA is used, oligodeoxyribonucleotides derived from the translation-initiation site, e.g., between about -10 and +10 positions of the target gene nucleotide sequence, are typical.

Other potential antagonists include small molecules that bind to the active site, the ligand or binding molecule binding site, thereby blocking the normal biological activity of the beta7 integrin. Examples of small molecules include, but are not limited to, small peptides or peptide-like molecules, typically soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can-be identified by known techniques. For further details see, *e.g.,* Rossi, Current Biology, 4:469-471 (1994), and PCT Publication No. WO 97/33551 (published Sep. 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, *e.g.,* PCT Publication No. WO 97/33551. These small molecules can be identified by any one or more of the screening assays discussed hereinabove and/or by any other screening techniques well known for those skilled in the art.

Screening assays for antagonists are designed to identify compounds that bind or complex with the beta7 integrin encoded by the genes identified herein, or otherwise interfere with the interaction of the encoded polypeptides with other cellular proteins. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

### B. Anti-Beta7 integrin Antibodies

In one embodiment, the beta7 integrin antagonists are anti-beta7 antibodies. Exemplary antibodies include polyclonal, monoclonal, humanized, human, bispecific, and heteroconjugate antibodies, etc., as described below.

### 1. Polyclonal Antibodies

Polyclonal antibodies can be raised in animals by multiple subcutaneous (SC) or intraperitoneal (IP) injections of the relevant antigen and an adjuvant. It may be useful to conjugate the relevant antigen to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals are immunized against the antigen, immunogenic conjugates, or derivatives by combining, *e.g*., 100 µg or 5 µg of the protein or conjugate (for rabbits or mice, respectively) with 3 volumes of Freund's complete adjuvant and injecting the solution intradermally at multiple sites. One month later the animals are boosted with 1/5 to 1/10 the original amount of peptide or conjugate in Freund's complete adjuvant by subcutaneous injection at multiple sites. Seven to 14 days later the animals are bled and the serum is assayed for antibody titer. Animals are boosted until the titer plateaus. In certain embodiments, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

### 2. Monoclonal Antibodies

Monoclonal antibodies may be made using the hybridoma method first described by Kohler et al., Nature, 256:495 (1975), or may be made by recombinant DNA methods (see, e.g., U.S. Patent No. 4,816,567).

In the hybridoma method, a mouse or other appropriate host animal, such as a hamster, is immunized as described above to elicit lymphocytes that produce or are capable of producing antibodies that will specifically bind to the protein used for immunization. Alternatively, lymphocytes may be immunized *in vitro.* After immunization, lymphocytes are isolated and then fused with a myeloma cell line using a suitable fusing agent, such as polyethylene glycol, to form a hybridoma cell (Goding, Monoclonal Antibodies: Principles and Practice, pp. 59-103 (Academic Press, 1986)).

The hybridoma cells thus prepared are seeded and grown in a suitable culture medium which medium may contain one or more substances that inhibit the growth or survival of the unfused, parental myeloma cells (also referred to as fusion partner). For example, if the parental myeloma cells lack the enzyme hypoxanthine guanine phosphoribosyl transferase (HGPRT or HPRT), the selective culture medium for the hybridomas typically will include hypoxanthine, aminopterin, and thymidine (HAT medium), which substances prevent the growth of HGPRT-deficient cells.

In certain embodiments, fusion partner myeloma cells are those that fuse efficiently, support stable high-level production of antibody by the selected antibody-producing cells, and are sensitive to a selective medium that selects against the unfused parental cells. In certain embodiments, myeloma cell lines are murine myeloma lines, such as those derived from MOPC-21 and MPC-11 mouse tumors available from the Salk Institute Cell Distribution Center, San Diego, Calif. USA, and SP-2 and derivatives *e.g.*, X63-Ag8-653 cells available from the American Type Culture Collection, Manassas, Va., USA. Human myeloma and mouse-human heteromyeloma cell lines also have been described for the production of human monoclonal antibodies (Kozbor, J. Immunol., 133:3001 (1984); and Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. In certain embodiments, the binding specificity of monoclonal antibodies produced by hybridoma cells is determined by immunoprecipitation or by an *in vitro* binding assay, such as radioimmunoassay (RIA) or enzyme-linked immunosorbent assay (ELISA).

The binding affinity of the monoclonal antibody can, for example, be determined by the Scatchard analysis described in Munson et al., Anal. Biochem., 107:220 (1980). Once hybridoma cells that produce antibodies of the desired specificity, affinity, and/or activity are identified, the clones may be subcloned by limiting dilution procedures and grown by standard methods (Goding, Monoclonal Antibodies: Principles and Practice, pp. 59-103 (Academic Press, 1986)). Suitable culture media for this purpose include, for example, D-MEM or RPMI-1640 medium. In addition, the hybridoma cells may be grown *in vivo* as ascites tumors in an animal *e.g.*, by i.p. injection of the cells into mice. The monoclonal antibodies secreted by the subclones are suitably separated from the culture medium, ascites fluid, or serum by conventional antibody purification procedures such as, for example, affinity chromatography (*e.g*., using protein A or protein G-Sepharose) or ion-exchange chromatography, hydroxylapatite chromatography, gel electrophoresis, dialysis, etc.

DNA encoding the monoclonal antibodies is readily isolated and sequenced using conventional procedures (*e.g*., by using oligonucleotide probes that are capable of binding specifically to genes encoding the heavy and light chains of murine antibodies). The hybridoma cells serve as a source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce antibody protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Pluckthun, Immunol. Revs. 130:151-188 (1992).

In a further embodiment, monoclonal antibodies or antibody fragments can be isolated from antibody phage libraries generated using e.g., the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res. 21:2265-2266 (1993)). Thus, these techniques are viable alternatives to traditional monoclonal antibody hybridoma techniques for isolation of monoclonal antibodies.

The DNA that encodes the antibody may be modified to produce chimeric or fusion antibody polypeptides, for example, by substituting human heavy chain and light chain constant domain (CH and CL) sequences for the homologous murine sequences (U.S. Patent No. 4,816,567; and Morrison, et al., Proc. Natl. Acad. Sci. USA, 81:6851 (1984)), or by fusing the immunoglobulin coding sequence with all or part of the coding sequence for a non-immunoglobulin polypeptide (heterologous polypeptide). The non-immunoglobulin polypeptide sequences can substitute for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

Exemplary anti-beta7 antibodies are Fib504, Fib 21, 22, 27, 30 (Tidswell, M. J Immunol. 1997 Aug 1; 159(3): 1497-505) or humanized derivatives thereof. Humanized antibodies of Fib504 was disclosed in detail in U.S. Patent Publication No. 20060093601 (issued as U.S. Patent No. 7,528,236), the content of which is incorporated by reference in its entirety (also see discussion below).

### 3. Human and Humanized Antibodies

The anti-beta7 integrin antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies. The choice of human variable domains, both light and heavy, to be used in making the humanized antibodies is very important to reduce antigenicity and HAMA response (human anti-mouse antibody) when the antibody is intended for human therapeutic use. According to the so-called "best-fit" method, the sequence of the variable domain of a rodent antibody is screened against the entire library of known human variable domain sequences. The human V domain sequence which is closest to that of the rodent is identified and the human framework region (FR) within it accepted for the humanized antibody (Sims et al., J. Immunol. 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol. 151:2623 (1993)). It is further important that antibodies be humanized with retention of high binding affinity for the antigen and other favorable biological properties. To achieve this goal, according to certain embodiments, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e.,* the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

Various forms of a humanized Anti-beta7 integrin antibody are contemplated. For example, the humanized antibody may be an antibody fragment, such as a Fab, which is optionally conjugated with one or more cytotoxic agent(s) in order to generate an immunoconjugate. Alternatively, the humanized antibody may be an intact antibody, such as an intact IgG1 antibody.

Exemplary humanized anti-beta7 antibodies include, but are not limited to rhuMAb Beta7, which is a humanized monoclonal antibody against the integrin subunit β7 and was derived from the rat anti-mouse/human monoclonal antibody FIB504 (Andrew et al., 1994 J Immunol 1994;153:3847-61). It has been engineered to include human immunoglobulin IgG1 heavy chain and κ1 light chain frameworks and is produced by Chinese hamster ovary cells. This antibody binds to two integrins, α4β7 (Holzmann et al. 1989 Cell, 1989;56:37-46; Hu et al., 1992, Proc Natl Acad Sci USA 1992;89:8254-8) and αEβ7 (Cepek et al., 1993 J Immunol 1993; 150:3459-70), which regulate trafficking and retention of lymphocyte subsets in the gastrointestinal tract and are involved in inflammatory bowel diseases (IBD) such as ulcerative colitis (UC) and Crohn's disease (CD). rhuMAb Beta7 is a potent *in vitro* blocker of the cellular interaction between α4β7 and its ligands (mucosal addressin cell adhesion molecule-1 [MAdCAM]-1, vascular cell adhesion molecule [VCAM]-1, and fibronectin) as well as the interaction between αEβ7 and its ligand (E-cadherin). rhuMAb Beta7 binds reversibly, with similar high affinity, to β7 on lymphocytes from rabbits, cynomolgus monkeys, and humans. It also binds to mouse β7 with high affinity. The amino acid sequence as well as the making and using of rhuMAb Beta7 and its variants are disclosed in detail in e.g., U.S. Patent Application Publication No. 20060093601 (issued as U.S. Patent No. 7,528,236), the content of which is incorporated in its entirety.

FIGS. 6A and 6B depict alignment of sequences of the variable light and heavy chains for the following: light chain human subgroup kappa I consensus sequence (FIG. 6A, SEQ ID NO:12), heavy chain human subgroup III consensus sequence (FIG. 6B, SEQ ID NO:13), rat anti-mouse beta7 antibody (Fib504) variable light chain (FIG. 6A, SEQ ID NO:10), rat anti-mouse beta7 antibody (Fib504) variable heavy chain (FIG. 6B, SEQ ID NO:11), and humanized antibody variants: Humanized hu504Kgraft variable light chain (FIG. 6A, SEQ ID NO:14), humanized hu504K graft variable heavy chain (FIG. 6B, SEQ ID NO:15), variants hu504-5, hu504-16, and hu504-32 (amino acid variations from humanized hu504K graft are indicated in FIG. 6A (light chain) (SEQ ID NOS:22-24, respectively, in order of appearance) and FIG. 6B (heavy chain) for variants hu504-5, hu504-16, and 504-32 (SEQ ID NO:25).

### 4. Human Antibodies

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array into such germ-line mutant mice will result in the production of human antibodies upon antigen challenge. See, *e.g.,* Jakobovits et al., Proc. Natl. Acad. Sci. USA, 90:2551 (1993); Jakobovits et al., Nature, 362:255-258 (1993); Bruggemann et al., Year in Immuno. 7:33 (1993); U.S. Patent Nos. 5,545,806, 5,569,825, 5,591,669 (all of GenPharm); U.S. Patent No. 5,545,807; and WO 97/17852.

Alternatively, phage display technology (McCafferty et al., Nature 348:552-553 [1990]) can be used to produce human antibodies and antibody fragments *in vitro,* from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats, reviewed in, e.g., Johnson, Kevin S. and Chiswell, David J., Current Opinion in Structural Biology 3:564-571 (1993). Several sources of V-gene segments can be used for phage display. Clackson et al., Nature, 352:624-628(1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). See, also, U.S. Patent Nos. 5,565,332 and 5,573,905.

As discussed above, human antibodies may also be generated by *in vitro* activated B cells (see U.S. Patent Nos. 5,567,610 and 5,229,275).

### 5. Antibody Fragments

In certain circumstances there are advantages of using antibody fragments, rather than whole antibodies. The smaller size of the fragments allows for rapid clearance, and may lead to improved access to solid tumors.

Various techniques have been developed for the production of antibody fragments. Traditionally, these fragments were derived via proteolytic digestion of intact antibodies (see, *e.g.,* Morimoto et al., Journal of Biochemical and Biophysical Methods 24:107-117 (1992) and Brennan et al., Science, 229:81 (1985)). However, these fragments can now be produced directly by recombinant host cells. For example, the antibody fragments can be isolated from the antibody phage libraries discussed above. Alternatively, Fab'-SH fragments can be directly recovered from *E. coli* and chemically coupled to form F(ab')₂ fragments (Carter et al., Bio/Technology 10:163-167 (1992)). According to another approach, F(ab')₂ fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; US Patent No. 5,571,894; and US Patent No. 5,587,458. The antibody fragment may also be a "linear antibody," *e.g*., as described in US Patent No. 5,641,870 for example. Such linear antibody fragments may be monospecific or bispecific.

### 6. Bispecific Antibodies

Bispecific antibodies are antibodies that have binding specificities for at least two different epitopes. Exemplary bispecific antibodies may bind to two different epitopes of beta7 integrin as described herein. Other such antibodies may combine a TAT binding site with a binding site for another protein. Alternatively, an anti-Beta7 integrin arm may be combined with an arm which binds to a triggering molecule on a leukocyte such as a T-cell receptor molecule (*e.g.,* CD3), or Fc receptors for IgG (Fc.γ.R), such as Fc.γRI (CD64), Fc.γRII (CD32) and Fc. γ.RIII (CD16), so as to focus and localize cellular defense mechanisms to the TAT-expressing cell. Bispecific antibodies may also be used to localize cytotoxic agents to cells which express TAT. These antibodies possess a TAT-binding arm and an arm which binds the cytotoxic agent (e.g., saporin, anti-interferon-.alpha., vinca alkaloid, ricin A chain, methotrexate or radioactive isotope hapten). Bispecific antibodies can be prepared as full length antibodies or antibody fragments (*e.g*., F(ab')₂ bispecific antibodies).

Methods for making bispecific antibodies are known in the art. Traditional production of full length bispecific antibodies is based on the co-expression of two immunoglobulin heavy chain-light chain pairs, where the two chains have different specificities (Millstein et al., Nature 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J. 10:3655-3659 (1991).

According to a different approach, antibody variable domains with the desired binding specificities (antibody-antigen combining sites) are fused to immunoglobulin constant domain sequences. In certain embodiments, the fusion is with an Ig heavy chain constant domain, comprising at least part of the hinge, C_{H2}, and C_{H3} regions. In certain embodiments, the first heavy-chain constant region (C_{H1}) containing the site necessary for light chain bonding, present in at least one of the fusions. DNAs encoding the immunoglobulin heavy chain fusions and, if desired, the immunoglobulin light chain, are inserted into separate expression vectors, and are co-transfected into a suitable host cell. This provides for greater flexibility in adjusting the mutual proportions of the three polypeptide fragments in embodiments when unequal ratios of the three polypeptide chains used in the construction provide the optimum yield of the desired bispecific antibody. It is, however, possible to insert the coding sequences for two or all three polypeptide chains into a single expression vector when the expression of at least two polypeptide chains in equal ratios results in high yields or when the ratios have no significant affect on the yield of the desired chain combination.

In certain embodiments, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology 121:210 (1986).

According to another approach described in U.S. Patent No. 5,731,168, the interface between a pair of antibody molecules can be engineered to maximize the percentage of heterodimers which are recovered from recombinant cell culture. In certain embodiments, the interface comprises at least a part of the C_{H3} domain. In this method, one or more small amino acid side chains from the interface of the first antibody molecule are replaced with larger side chains (*e.g*., tyrosine or tryptophan). Compensatory "cavities" of identical or similar size to the large side chain(s) are created on the interface of the second antibody molecule by replacing large amino acid side chains with smaller ones (*e.g*., alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in U.S. Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science 229:81 (1985) describe a procedure wherein intact antibodies are proteolytically cleaved to generate F(ab').sub.2 fragments. These fragments are reduced in the presence of the dithiol complexing agent, sodium arsenite, to stabilize vicinal dithiols and prevent intermolecular disulfide formation. The Fab' fragments generated are then converted to thionitrobenzoate (TNB) derivatives: One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E*. *coli,* which can be chemically coupled to form bispecific antibodies. Shalaby et al., J. Exp. Med. 175: 217-225 (1992) describe the production of a fully humanized bispecific antibody F(ab')₂ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets. Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a V.sub.H connected to a V.sub.L by a linker which is too short to allow pairing between the two domains on the same chain. Accordingly, the V.sub.H and V.sub.L domains of one fragment are forced to pair with the complementary V.sub.L and V.sub.H domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al., J. Immunol. 147:60 (1991).

### 7. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 8. Multivalent Antibodies

A multivalent antibody may be internalized (and/or catabolized) faster than a bivalent antibody by a cell expressing an antigen to which the antibodies bind. The antibodies of the present invention can be multivalent antibodies (which are other than of the IgM class) with three or more antigen binding sites (*e.g*., tetravalent antibodies), which can be readily produced by recombinant expression of nucleic acid encoding the polypeptide chains of the antibody. The multivalent antibody can comprise a dimerization domain and three or more antigen binding sites. In certain embodiments, the dimerization domain comprises (or consists of) an Fc region or a hinge region. In this scenario, the antibody will comprise an Fc region and three or more antigen binding sites amino-terminal to the Fc region. In certain embodiments, the multivalent antibody herein comprises (or consists of) three to about eight, but typically four, antigen binding sites. The multivalent antibody comprises at least one polypeptide chain (and typically two polypeptide chains), wherein the polypeptide chain(s) comprise two or more variable domains. For instance, the polypeptide chain(s) may comprise VD1-(X1).sub.n-VD2-(X2).sub.n-Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CHl-Fc region chain; or VH-CH1-VH-CH1-Fc region chain. The multivalent antibody herein may further comprise at least two (and typically four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

### 9. Effector Function Engineering

It may be desirable to modify the antibody of the invention with respect to effector function, *e.g*., so as to enhance antigen-dependent cell-mediated cytotoxicity (ADCC) and/or complement dependent cytotoxicity (CDC) of the antibody. This may be achieved by introducing one or more amino acid substitutions in an Fc region of the antibody. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992). Homodimeric antibodies with enhanced anti-tumor activity may also be prepared using heterobifunctional cross-linkers as described in Wolff et al., Cancer Research 53:2560-2565 (1993). Alternatively, an antibody can be engineered which has dual Fc regions and may thereby have enhanced complement lysis and ADCC capabilities. See Stevenson et al., AntiCancer Drug Design 3:219-230 (1989). To increase the serum half life of the antibody, one may incorporate a salvage receptor binding epitope into the antibody (especially an antibody fragment) as described in U.S. Patent No. 5,739,277, for example. As used herein, the term "salvage receptor binding epitope" refers to an epitope of the Fc region of an IgG molecule (*e.g.,* IgG₁, IgG₂, IgG₃, or IgG₄) that is responsible for increasing the *in vivo* serum half-life of the IgG molecule.

### 10. Immunoconjugates

The antagonist or antibody used in the methods herein is optionally conjugated to another agent, such as a cytotoxic agent, or cytokine.

Conjugation will ordinarily be achieved through a covalent linkage, the precise nature of which will be determined by the targeting molecule and the linking site on the integrin beta7 antagonist or antibody polypeptide. Typically, a non-peptidic agent is modified by the addition of a linker that allows conjugation to anti-beta7 integrin antibody through its amino acid side chains, carbohydrate chains, or reactive groups introduced on antibody by chemical modification. For example, a drug may be attached through the .epsilon.-amino group of a lysine residue, through a free .alpha.-amino group, by disulfide exchange to a cysteine residue, or by oxidation of the 1,2- diols in a carbohydrate chain with periodic acid to allow attachment of drugs containing various nucleophiles through a Schiff-base linkage. See, for example, U.S. Patent No. 4,256,833. Protein modifying agents include amine-reactive reagents (*e.g*., reactive esters, isothiocyanates, aldehydes, and sulfonyl halides), thiol-reactive reagents (*e.g*., haloacetyl derivatives and maleimides), and carboxylic acid- and aldehyde-reactive reagents. Integrin beta7 antagonist or antibody polypeptides can be covalently joined to peptidic agents through the use of bifunctional cross-linking reagents. Heterobifunctional reagents are more commonly used and permit the controlled coupling of two different proteins through the use of two different reactive moieties (*e.g*., amine-reactive plus thiol, iodoacetamide, or maleimide). The use of such linking agents is well known in the art. See, for example, Brinkley, *supra,* and U.S. Patent No. 4,671,958. Peptidic linkers can also be employed. In the alternative, an anti-beta7 integrin antibody polypeptide can be linked to a peptidic moiety through preparation of a fusion polypeptide.

Examples of further bifunctional protein coupling agents include N-succinimidyl-3-(2-pyridyldithiol) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene).

### 11. Immunoliposomes

The anti-beta7 integrin antibodies disclosed herein may also be formulated as immunoliposomes. A "liposome" is a small vesicle composed of various types of lipids, phospholipids and/or surfactant which is useful for delivery of a drug to a mammal. The components of the liposome are commonly arranged in a bilayer formation, similar to the lipid arrangement of biological membranes. Liposomes containing the antibody are prepared by methods known in the art, such as described in Epstein et al., Proc. Natl. Acad. Sci. USA 82:3688 (1985); Hwang et al., Proc. Natl Acad. Sci. USA 77:4030 (1980); U.S. Patent Nos. 4,485,045 and 4,544,545; and WO97/38731 published Oct. 23, 1997. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter.

Fab' fragments of the antibody of the present invention can be conjugated to the liposomes as described in Martin et al., J. Biol. Chem. 257:286-288 (1982) via a disulfide interchange reaction. A chemotherapeutic agent is optionally contained within the liposome. See Gabizon et al., J. National Cancer Inst. 81(19):1484 (1989).

### 12. Vectors, Host Cells and Recombinant Methods for Antibody Production

Also provided are isolated nucleic acids encoding the anti-beta7 antibodies or polypeptide agents described herein, vectors and host cells comprising the nucleic acids and recombinant techniques for the production of the antibodies.

For recombinant production of the antibody, the nucleic acid encoding it may be isolated and inserted into a replicable vector for further cloning (amplification of the DNA) or for expression. In another embodiment, the antibody may be produced by homologous recombination, *e.g*., as described in U.S. Patent No. 5,204,244, specifically incorporated herein by reference. DNA encoding the monoclonal antibody is readily isolated and sequenced using conventional procedures (*e.g*., by using oligonucleotide probes that are capable of binding specifically to genes encoding the heavy and light chains of the antibody). Many vectors are available. The vector components generally include, but are not limited to, one or more of the following: a signal sequence, an origin of replication, one or more marker genes, an enhancer element, a promoter, and a transcription termination sequence, *e.g.,* as described in U.S. Patent No. 5,534,615 issued Jul. 9, 1996 and specifically incorporated herein by reference.

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as Escherichia, *e.g., E. coli,* Enterobacter, Erwinia, Klebsiella, Proteus, Salmonella, *e.g.,* Salmonella typhimurium, Serratia, *e.g.,* Serratia marcescans, and Shigella, as well as Bacilli such as B. subtilis and B. licheniformis (*e.g*., B. licheniformis 41P disclosed in DD 266,710 published 12 Apr. 1989), Pseudomonas such as P. aeruginosa, and Streptomyces. One *E. coli* cloning host is *E. coli* 294 (ATCC 31,446), although other strains such as *E. coli* B, *E. coli* X1776 (ATCC 31,537), and *E. coli* W3110 (ATCC 27,325) are suitable. These examples are illustrative rather than limiting.

In addition to prokaryotes, eukaryotic microbes such as filamentous fungi or yeast are suitable cloning or expression hosts for anti-beta7 integrin antibody-encoding vectors. Saccharomyces cerevisiae, or common baker's yeast, is the most commonly used among lower eukaryotic host microorganisms. However, a number of other genera, species, and strains are commonly available and useful herein, such as Schizosaccharomyces pombe; Kluyveromyces hosts such as, *e.g.,* K. lactis, K. fragilis (ATCC 12,424), K. bulgaricus (ATCC 16,045), K. wickeramii (ATCC 24,178), K. waltii (ATCC 56,500), K. drosophilarum (ATCC 36,906), K. thermotolerans, and K. marxianus; yarrowia (EP 402,226); Pichia pastoris (EP 183,070); Candida; Trichoderma reesia (EP 244,234); Neurospora crassa; Schwanniomyces such as Schwanniomyces occidentalis; and filamentous fungi such as, *e.g.,* Neurospora, Penicillium, Tolypocladium, and Aspergillus hosts such as A. nidulans and A. niger.

Suitable host cells for the expression of glycosylated anti-Beta7 antibody are derived from multicellular organisms. Examples of invertebrate cells include plant and insect cells. Numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruitfly), and Bombyx mori have been identified. A variety of viral strains for transfection are publicly available, *e.g.,* the L-1 variant of Autographa californica NPV and the Bm-5 strain of Bombyx mori NPV, and such viruses may be used as the virus herein according to the present invention, particularly for transfection of Spodoptera frugiperda cells. Plant cell cultures of cotton, corn, potato, soybean, petunia, tomato, and tobacco can also be utilized as hosts.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR(CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for anti-beta7 integrin antibody production and cultured in conventional nutrient media modified as appropriate for inducing promoters, selecting transformants, or amplifying the genes encoding the desired sequences.

The host cells used to produce the anti-beta7 integrin antibody of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem. 1 02:255 (1980), U.S. Patent Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN.TM.drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

When using recombinant techniques, the antibody can be produced intracellularly, in the periplasmic space, or directly secreted into the medium. If the antibody is produced intracellularly, as a first step, the particulate debris, either host cells or lysed fragments, is removed, for example, by centrifugation or ultrafiltration. Carter et al., Bio/Technology 10:163-167 (1992) describe a procedure for isolating antibodies which are secreted to the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being the typical purification technique. The suitability of protein A as an affinity ligand depends on the species and isotype of any immunoglobulin Fc domain that is present in the antibody. Protein A can be used to purify antibodies that are based on human .gamma.1, .gamma.2, or .gamma.4 heavy chains (Lindmark et al., J. Immunol. Meth. 62:1-13 (1983)). Protein G is recommended for all mouse isotypes and for human .gamma.3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a C.sub.H3 domain, the Bakerbond ABX.TM.resin (J. T. Baker, Phillipsburg, N.J.) is useful for purification. Other techniques for protein purification such as fractionation on an ion-exchange column, ethanol precipitation, Reverse Phase HPLC, chromatography on silica, chromatography on heparin SEPHAROSE.TM. chromatography on an anion or cation exchange resin (such as a polyaspartic acid column), chromatofocusing, SDS-PAGE, and ammonium sulfate precipitation are also available depending on the antibody to be recovered. Following any preliminary purification step(s), the mixture comprising the antibody of interest and contaminants may be subjected to low pH hydrophobic interaction chromatography using an elution buffer at a pH between about 2.5-4.5, typically performed at low salt concentrations (*e.g.,* from about 0-0.25 M salt).

### C. Pharmaceutical Formulations

Therapeutic formulations comprising the therapeutic agents, antagonists or antibodies of the invention are prepared for storage by mixing the antibody having the desired degree of purity with optional physiologically acceptable carriers, excipients or stabilizers (Remington's Pharmaceutical Sciences 16th edition, Osol, A. Ed. (1980)), in the form of aqueous solutions, lyophilized or other dried formulations. Acceptable carriers, excipients, or stabilizers are nontoxic to recipients at the dosages and concentrations employed, and include buffers such as phosphate, citrate, histidine and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*., Zn-protein complexes); and/or non-ionic surfactants such as TWEEN.TM., PLURONICS.TM. or polyethylene glycol (PEG).

The formulation herein may also contain more than one active compound as necessary for the particular indication being treated, typically those with complementary activities that do not adversely affect each other. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the immunoglobulin of the invention, which matrices are in the form of shaped articles, *e.g.,* films, or microcapsule. Examples of sustained-release matrices include polyesters, hydrogels (for example, poly(2-hydroxyethyl-methacrylate), or poly(vinylalcohol)), polylactides (U.S. Patent No. 3,773,919), copolymers of L-glutamic acid and .gamma. ethyl-L-glutamate, non-degradable ethylene-vinyl acetate, degradable lactic acid-glycolic acid copolymers such as the LUPRON DEPOT.TM. (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated immunoglobulins remain in the body for a long time, they may denature or aggregate as a result of exposure to moisture at 37°C., resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

### D. Administration

The physician administering treatment will be able to determine the appropriate dose for the individual subject for weight-based dosing or, for flat dosing, will follow the instructions on the label. Preparation and dosing schedules for commercially available second therapeutic and other compounds administered in combination with the integrin beta7 antagonists may be used according to manufacturers' instructions or determined empirically by the skilled practitioner.

For the prevention or treatment of disease, the appropriate dosage of the integrin beta7 antagonist and any second therapeutic or other compound administered in combination with the non-depleting antibody will depend on the type of gastrointestinal inflammatory disorder to be treated, e.g., IBD, UC, CD, the severity and course of the disease, whether the integrin beta7 antagonist or combination is administered for preventive or therapeutic purposes, previous therapy, the patient's clinical history and response to the integrin beta7 antagonist or combination, and the discretion of the attending physician. The integrin beta7 antagonist or combination is suitably administered to the patient at one time or more typically over a series of treatments. In certain embodiments, the integrin beta7 antagonist is administered once every week, or once every two weeks, or once every four weeks, or once every six weeks, or once every eight weeks for a period of one month (4 weeks), or two months, three months, or six months, or 12 months, or 18 months, or 24 months, or chronically for the lifetime of the patient. In certain embodiments, the treatment is self-administered by the patient.

Depending on the type and severity of the disease, about 0.5 mg/kg to 4.0 mg/kg of anti-beta7 antibody is an initial candidate dosage for administration to the patient, whether, for example, by one or more separate administrations, or by continuous infusion. For repeated administrations over several days or longer, depending on the condition, the treatment is sustained until a desired suppression of disease symptoms occurs. However, other dosage regimens may be useful.

For example, in certain embodiments, a flat dose of anti-beta7 antibody is administered to the patient. A flat dose is a particular amount of anti-beta7 antibody that is administered to every patient regardless of weight. Depending on the type and severity of the disease, a flat dose of between about 50 mg and 450 mg of anti-beta7 antibody is administered to the patient, which may be one or more separate injections or infusions or administrations. Such flat dose can be administered intravenously or subcutaneously or by other routes as described herein. In certain embodiments, the flat dose is 50 mg, or 100 mg, or 150 mg, or 200 mg, or 300 mg, or 400 mg, or 420 mg, or 450 mg.

In certain embodiments, an initial flat loading dose of anti-beta7 antibody is followed by one or more flat maintenance doses of anti-beta7 antibody. The loading dose is a larger quantity of anti-beta7 antibody than the maintenance dose. In certain embodiments, the loading dose is between about 400 mg and 450 mg and the maintenance dose is between about 50 mg and 350 mg. In certain embodiments, the loading dose is 400 mg, or 420 mg, or 430 mg, or 450 mg. In certain embodiments, the maintenance dose is 50 mg, or 100 mg, or 150 mg, or 200 mg, or 300 mg, or 350 mg. In certain embodiments, the loading dose is 400 mg and the maintenance dose is 50 mg. In certain embodiments, the loading dose is 400 mg and the maintenance dose is 100 mg. In certain embodiments, the loading dose is 400 mg and the maintenance dose is 150 mg. In certain embodiments, the loading dose is 400 mg and the maintenance dose is 200 mg. In certain embodiments, the loading dose is 400 mg and the maintenance dose is 300 mg. In certain embodiments, the loading dose is 400 mg and the maintenance dose is 350 mg. In certain embodiments, the loading dose is 420 mg and the maintenance dose is 50 mg. In certain embodiments, the loading dose is 420 mg and the maintenance dose is 100 mg. In certain embodiments, the loading dose is 420 mg and the maintenance dose is 150 mg. In certain embodiments, the loading dose is 420 mg and the maintenance dose is 200 mg. In certain embodiments, the loading dose is 420 mg and the maintenance dose is 300 mg. In certain embodiments, the loading dose is 420 mg and the maintenance dose is 350 mg. In certain embodiments, the loading dose is 430 mg and the maintenance dose is 50 mg. In certain embodiments, the loading dose is 430 mg and the maintenance dose is 100 mg. In certain embodiments, the loading dose is 430 mg and the maintenance dose is 150 mg. In certain embodiments, the loading dose is 430 mg and the maintenance dose is 200 mg. In certain embodiments, the loading dose is 430 mg and the maintenance dose is 300 mg. In certain embodiments, the loading dose is 430 mg and the maintenance dose is 350 mg. In certain embodiments, the loading dose is 450 mg and the maintenance dose is 50 mg. In certain embodiments, the loading dose is 450 mg and the maintenance dose is 100 mg. In certain embodiments, the loading dose is 450 mg and the maintenance dose is 150 mg. In certain embodiments, the loading dose is 450 mg and the maintenance dose is 200 mg. In certain embodiments, the loading dose is 450 mg and the maintenance dose is 300 mg. In certain embodiments, the loading dose is 450 mg and the maintenance dose is 350 mg.

In certain embodiments, the first maintenance dose is administered the same day as the loading dose. In certain embodiments, the first maintenance dose is administered one day after the loading dose. In certain embodiments, the first maintenance dose is administered one week after the loading dose. In certain embodiments, the first maintenance dose is administered two weeks after the loading dose. In certain embodiments, the first maintenance dose is administered four weeks after the loading dose. In certain embodiments, the second and each subsequent maintenance dose is administered every two weeks. In certain embodiments, the second and each subsequent maintenance dose is administered every four weeks. In certain embodiments, the second and each subsequent maintenance dose is administered every eight weeks. In certain embodiments, the first maintenance dose is administered two weeks after the loading dose, the second maintenance dose is administered two weeks after the first maintenance dose, and each subsequent maintenance dose is administered every four weeks. In certain embodiments, the anti-beta7 antibody is administered for a period of two months. In certain embodiments, the anti-beta7 antibody is administered for a period of three months. In certain embodiments, the anti-beta7 antibody is administered for a period of six months. In certain embodiments, the anti-beta7 antibody is administered for a period of 12 months. In certain embodiments, the anti-beta7 antibody is administered for a period of 18 months. In certain embodiments, the anti-beta7 antibody is administered for a period of 24 months. In certain embodiments, the anti-beta7 antibody is administered for the lifetime of the subject.

Typically, the clinician will administer an antibody (alone or in combination with a second compound) of the invention until a dosage(s) is reached that provides the required biological effect. The progress of the therapy of the invention is easily monitored by conventional techniques and assays.

The integrin beta7 antagonist can be administered by any suitable means, including parenteral, topical, intravenous, subcutaneous, intraperitoneal, intrapulmonary, intranasal, and/or intralesional administration. Parenteral infusions include intramuscular, intravenous, intraarterial, intraperitoneal, or subcutaneous administration. Intrathecal administration is also contemplated (see, e.g., U.S. Patent Publication No. 2002/0009444 by Grillo-Lopez). In addition, the integrin beta7 antagonist may suitably be administered by pulse infusion, e.g., with declining doses of the antibody. In certain embodiments, the dosing is given intravenously or subcutaneously. Each exposure may be provided using the same or a different administration means. In one embodiment, each exposure to anti-beta7 antibody is by subcutaneous administration. In one embodiment, the first exposure to anti-beta7 antibody, e.g., the loading dose, is by intravenous administration and each subsequent exposure is by subcutaneous administration.

In certain embodiments, an anti-beta7 antibody is administered using, for example, a self-inject device, autoinjector device, or other device designed for self-administration. Various self-inject devices, including autoinjector devices, are known in the art and are commercially available. Exemplary devices include, but are not limited to, prefilled syringes (such as BD HYPAK SCF®, READYFILLTM, and STERIFILL SCFTM from Becton Dickinson; CLEARSHOTTM copolymer prefilled syringes from Baxter; and Daikyo Seiko CRYSTAL ZENITH® prefilled syringes available from West Pharmaceutical Services); disposable pen injection devices such as BD Pen from Becton Dickinson; ultra-sharp and microneedle devices (such as INJECT-EASETM and microinfuser devices from Becton Dickinson; and H-PATCHTM available from Valeritas) as well as needle-free injection devices (such as BIOJECTOR® and IJECT® available from Bioject; and SOF-SERTER® and patch devices available from Medtronic). In certain embodiments, rhuMAb Beta7 is an article of manufacture comprising a prefilled syringe comprising 2 ML (150 mg) rhuMAb Beta7. In certain embodiments, rhuMAb Beta7 is an article of manufacture comprising a prefilled syringe comprising 1 ML (180 mg) rhuMAb Beta7.

As noted, the integrin beta7 antagonist can be administered alone or in combination with at least a second therapeutic compound. These second therapeutic compounds are generally used in the same dosages and with administration routes as used heretofore, or about from 1 to 99% of the heretofore-employed dosages. If such second compounds are used, they are used in certain embodiments in lower amounts than if the integrin beta7 antagonist were not present, so as to eliminate or reduce side effects caused thereby.

Also as noted (e.g., see below), a variety of suitable second therapeutic compounds for the treatment of IBD, e.g., ulcerative colitis and Crohn's disease are known in the art, and dosages and administration methods for such second therapeutic compounds have likewise been described.

Administration of the integrin beta7 antagonist and any second therapeutic compound can be done simultaneously, e.g., as a single composition or as two or more distinct compositions using the same or different administration routes. Alternatively, or additionally, the administration can be done sequentially, in any order. In certain embodiments, intervals ranging from minutes to days, to weeks to months, can be present between the administrations of the two or more compositions. For example, the integrin beta7 antagonist may be administered first, followed by the second therapeutic compound. However, simultaneous administration or administration of the second therapeutic compound prior to the integrin beta7 antagonist is also contemplated.

The standard of care for subjects with active moderate-severe active UC involves therapy with standard doses of: an aminosalicylate, an oral corticosteroid, 6-mercaptopurine (6-MP) and/or azathioprine. Therapy with an integrin beta7 antagonist, such as an anti-beta7 integrin antibody as disclosed herein will result in an improvement in disease remission (rapid control of disease and/or prolonged remission), and/or clinical response, superior to that achieved with the standard of care for such subjects.

In one embodiment, the treatment of the present invention for inflammatory bowel disease (IBD) in a human subject with IBD comprises administering to the subject an effective amount of an therapeutic agent, such as an anti-beta7 integrin antibody, and further comprising administering to the subject an effective amount of a second medicament, that is an immunosuppressant, a pain-control agent, an antidiarrheal agent, an antibiotic, or a combination thereof.

In an exemplary embodiment, said secondary medicine is selected from the group consisting of an aminosalicylate, an oral corticosteroid, 6-mercaptopurine (6-MP) and azathioprine. In another exemplary embodiment, said secondary medicine is another integrin beta7 antagonist, such as another anti-beta7 integrin antibody or an antibody against a cytokine.

All these second medicaments may be used in combination with each other or by themselves with the first medicament, so that the expression "second medicament" as used herein does not mean it is the only medicament besides the first medicament, respectively. Thus, the second medicament need not be one medicament, but may constitute or comprise more than one such drug.

Combined administration herein includes co-administration, using separate formulations or a single pharmaceutical formulation, and consecutive administration in either order, wherein generally there is a time period while both (or all) active agents simultaneously exert their biological activities.

The combined administration of a second medicament includes co-administration (concurrent administration), using separate formulations or a single pharmaceutical formulation, and consecutive administration in either order, wherein generally there is a time period while both (or all) active agents (medicaments) simultaneously exert their biological activities.

### E. Design Treatment Regimens

Drug development is a complex and expensive process. The cost of bringing a new drug to market is estimated to be between $800 million and $1 billion. Less than 10% of drugs in phase I clinical trials make it to the approval phase. Two key reasons why drugs fail at late stages are a lack of understanding of the relationship between dose-concentration response and unanticipated safety events. Given this scenario, it is important to have enabling tools that help predict how a drug will perform *in vivo* and assist in the success of a clinical therapeutic candidate (Lakshmi Kamath, Drug Discovery and Development; Modeling Success in PK/PD Testing Drug Discovery & Development (2006)).

Pharmacokinetics (PK) characterizes the absorption, distribution, metabolism, and elimination properties of a drug. Pharmacodynamics (PD) defines the physiological and biological response to the administered drug. PK/PD modeling establishes a mathematical and theoretical link between these two processes and helps better predict drug action. Integrated PK/PD modeling and computer-assisted trial design via simulation are being incorporated into many drug development programs and are having a growing impact (Lakshmi Kamath, Drug Discovery and Development; Modeling Success in PK/PD Testing Drug Discovery & Development (2006)).

PK/PD testing is typically performed at every stage of the drug development process. Because development is becoming increasingly complex, time consuming, and cost intensive, companies are looking to make better use of PK/PD data to eliminate flawed candidates at the beginning and identify those with the best chance of clinical success. (Lakshmi Kamath, *supra*).

PK/PD modeling approaches are proving useful in determining relationships between biomarker response, drug levels, and dosing regimens. The PK/PD profile of a drug candidate and the ability to predict a patient's response to it are critical to the success of clinical trials. Recent advances in molecular biology techniques and a better understanding of targets for various diseases have validated biomarkers as a good clinical indicator of a drug's therapeutic efficacy. Biomarker assays help identify a biological response to a drug candidate. Once a biomarker is clinically validated, trial simulations can be effectively modeled. Biomarkers have the potential to achieve surrogate status that may someday substitute for clinical outcomes in drug development. (Lakshmi Kamath, *supra*).

The amount of biomarkers in the peripheral blood can be used in identifying the biological response to a treatment with integrin beta7 antagonists and can therefore function as a good clinical indicator for the therapeutic efficacy of a candidate treatment.

Traditional PK/PD modeling in drug development defines parameters such as drug dose concentration, drug exposure effects, drug half-life, drug concentrations against time, and drug effects against time. When used more broadly, quantitative techniques such as drug modeling, disease modeling, trial modeling, and market modeling can support the entire development process, which results in better decisions through explicit consideration of risk and better utilization of knowledge. A variety of PK/PD modeling tools are available to drug development researchers, for example, WinNonlin and the Knowledgebase Server (PKS) developed by Pharsight, Inc. Mountain View, California.

The foregoing written specification and following examples are considered to be sufficient to enable one skilled in the art to practice the invention. Various modifications of the invention in addition to those shown and described herein will become apparent to those skilled in the art from the foregoing description and following examples and fall within the scope of the appended claims.

It is understood that the application of the teachings of the present invention to a specific problem or situation will be within the capabilities of one having ordinary skill in the art in light of the teachings contained herein.

Further details of the invention are illustrated by the following non-limiting Examples. The disclosures of all citations in the specification are expressly incorporated herein by reference.

### EXAMPLES

### Example 1

### A PHASE I, MULTICENTER, RANDOMIZED, PLACEBO-CONTROLLED, DOUBLE-BLIND STUDY TO ASSESS THE SAFETY, PHARMACOKINETICS, PHARMACODYNAMICS, AND IMMUNOGENICITY OF INTRAVENOUS AND SUBCUTANEOUS rhuMAb Beta7 ADMINISTERED IN A SINGLE-DOSE, DOSE-ESCALATION STAGE FOLLOWED BY A MULTIDOSE, PARALLEL-TREATMENT STAGE IN PATIENTS WITH ULCERATIVE COLITIS

### Description of the Study

### Description of rhuMAb Beta7 (etrolizumab)

RhuMAb Beta7 (etrolizumab) is a humanized monoclonal antibody based on the human IgG1 subgroup III V_{H}, κ subgroup-I V_{L} consensus sequences and is directed specifically against the β7 subunit of the integrin heterodimer. It has been shown to bind with high affinity to α4β7 (K_{d} of about 116 pM) and αEβ7 (K_{d} of about 1800 pM).

This recombinant antibody has two heavy chains (446 residues) and two light chains (214 residues) that are covalently linked by interchain and intrachain disulfide bonds typical of IgG1 antibodies. For the work described herein, it was produced in Chinese hamster ovary (CHO) cells. The molecular mass of the intact, non glycosylated rhuMAb Beta7 molecule was approximately 144 kDa. Each heavy chain of rhuMAb Beta7 has one conserved N linked glycosylation site at Asn297. The oligosaccharides present at this site were typical of those observed in recombinant antibodies expressed in CHO cells, with the predominant glycoforms being the asialo, biantennary GO, and G1 glycans. The mass of the most prevalent rhuMAb Beta7 form containing two GO glycans and no C terminal lysine residues was approximately 147 kDa.

RhuMAb Beta7 was supplied by Genentech as a clear to slightly opalescent, colorless to slightly yellow aqueous solution in a physiologically acceptable buffer.

### Overall Design of the Study

This first-in-human Phase I study consisted of a randomized (within dose cohort), double-blind, placebo-controlled, single-dose, dose-escalation stage followed by a randomized (across dose cohorts), double-blind, placebo controlled, multidose, parallel-treatment stage. Patients who participated in the single-dose stage of the study who received rhuMAb Beta7 were not permitted to participate in the multidose stage. However, patients who received placebo in the single-dose stage of the study were allowed to take part in the multidose stage. Forty-eight patients with ulcerative colitis took part in this study. Patients were required to have had a diagnosis of ulcerative colitis for 12 weeks. The study was conducted in patients 18-70 years of age who had a diagnosis of moderate to severe ulcerative colitis, were outpatients, and had a Mayo Clinic score (MCS) of ≥ 5 points at baseline. The range for the MCS is 0 to 12, and higher scores indicate more severe disease activity (Schroeder et al., N. Engl. J. Med. 317:1625-1629 (1987); see also Table 1 below). Patients who were systemically unwell and required hospitalization or surgery because of their ulcerative colitis were not included in the study. In addition, eligible patients were considered by the investigator to be candidates for biologic therapy, in that they had moderate to severe disease that had not responded to standard therapy with 5-ASA, immunosuppressives (azathioprine or 6-mercaptopurine), or steroids.

**Table 1. Mayo Clinic Scoring System for Assessment of Ulcerative Colitis Activity.**

| | **Assessment Category** | | | |
|---|---|---|---|---|
| **Score** | **Stool frequency^{a}** | **Rectal bleeding^{b}** | **Findings on Endoscopy** | **Physician's global assessment^{c}** |
| 0 | normal no. of stools for this patient | no blood seen | normal or inactive disease | normal |
| 1 | 1 to 2 stools more than normal | streaks of blood with stool less than half the time | mild disease (erythema, decreased vascular pattern, mild friability) | mild disease |
| 2 | 3 to 4 stools more than normal | obvious blood with stool most of the time | moderate disease (marked erythema, lack of vascular pattern, friability, erosions) | moderate disease |
| 3 | 5 or more stools more than normal | blood alone passes | severe disease (spontaneous bleeding, ulceration) | severe disease |
| | subscore: 0 to 3 | subscore: 0 to 3 | subscore: 0 to 3 | subscore: 0 to 3 |

| | | | | |
|---|---|---|---|---|
| ^{a} Each patient serves as his or her own control to establish the degree of abnormality of the stool frequency. ^{b} The daily bleeding score represents the most severe bleeding of the day. ^{c} The physician's global assessment acknowledges the three other criteria, the patient's daily recollection of abdominal discomfort and general sense of well-being, and other observations, such as physical findings and the patient's performance status. | | | | |

The objective of the single-dose dose-escalation stage of the study was to assess safety and tolerability of rhuMAb Beta7 administration in patients with UC. In this stage, patients were enrolled sequentially into five ascending dose cohorts (Cohorts A-F; note that an originally-planned Cohort E that was to receive a dose of 1.0 mg/kg SC was omitted, but the designation A-F has been kept here) and treated with single intravenous (IV) or subcutaneous (SC) doses at one of four dose levels (0.3, 1.0, 3.0, or 10.0 mg/kg) (see Table 2).

Five patients were planned for enrollment per cohort. Within each cohort, patients were randomly assigned to receive rhuMAb Beta7 or placebo. For the single-dose stage, dose escalation decisions were based on clinical safety evaluation of cohorts receiving study drug through the IV route (Cohorts A, B, C, and D). On the basis of previous experience with SC administration of other monoclonal antibodies in humans, the exposure after SC administration was expected to be lower than the exposure achieved after IV administration.

Any potential acute toxicities (e.g., hypersensitivity reactions, infusion reactions, or serum sickness-type reactions) were expected to manifest and likely resolve during the 14-day safety follow-up period. Following completion of dosing in the single-dose stage, safety and pharmacokinetic (PK) data was evaluated prior to commencing the multidose stage.

The multidose parallel-treatment stage (Cohorts G-J) commenced approximately 10 weeks after dosing was completed for the last patient treated in Cohort F (3.0 mg/kg SC) of the single-dose stage. During these 10 weeks, the PK and clinical safety data were reviewed for the single-dose stage of the study. The parallel design and proposed dose range for the multidose stage were conditional on a maximum tolerated dose (MTD) of > 10 mg/kg from the single dose stage and was confirmed upon review of the PK data in the single dose stage to ensure that projected human pharmacokinetics, based on the cynomolgus study, were supported (see below). The maximum dose of 4.0 mg/kg IV administered every 4 weeks for three cycles in the multidose stage was within the safety factors determined from the cynomolgus monkey toxicology data (Intn'l Patent Pub. No. WO2009/140684; Stefanich et al., Br. J. Pharmacol., Accepted Article, doi: 10.1111/j.1476-5381.2011.01205.x), with safety factors of 16-fold on the basis of the human equivalent dose (HED) and 21 fold on the basis of exposure as measured by area under the curve (AUC) (see below). The primary objective of the multidose stage was to characterize the safety of multiple dosing across a proposed dose range (0.5-4 mg/kg) and to evaluate the potential for immunogenicity with repeat dosing in a patient group with ulcerative colitis. In this part of the study, patients were randomly assigned to receive rhuMAb Beta7 or placebo in one of four dose cohorts (see Table 2). RhuMAb Beta7 or placebo was administered subcutaneously (0.5 mg/kg, 1.5 mg/kg, or 3.0 mg/kg) or intravenously (4.0 mg/kg) every 4 weeks for three cycles, at Days 1, 29, and 57 (see Table 2). The assessment of exposure safety at the 4.0 mg/kg level was important for considering administration of rhuMAb Beta7 at flat dosing.

The incidence and nature of adverse events, serious adverse events, and laboratory abnormalities were assessed. Safety was assessed by the incidence of adverse events, graded according to the National Cancer Institute Common Toxicity Criteria for Adverse Events (NCI CTCAE), v3.0. Patients were followed for safety for 18 weeks (through Day 127) during the single-dose stage of the study and for 28 weeks (through Day 197) during the multidose stage. Blood samples were collected for PK, exploratory PD, and anti-therapeutic antibody (ATA) assessments.

**Table 2. Study Cohorts**

| Cohort | Dosage (mg/kg) | Route |
|---|---|---|
| A | 0.3 | IV |
| B | 1 | IV |
| C | 3 | IV |
| D | 10 | IV |
| E | Omitted | |
| F | 3 | SC |
| G | 0.5×3 ^{a} | SC |
| H | 1.5×3 ^{a} | SC |
| I | 3.0×3 ^{a} | SC |
| J | 4.0×3 ^{a} | IV |

| | | |
|---|---|---|
| IV = intravenous; SC = subcutaneous. ^{a}Study drug administered once every 4 weeks for three cycles, at Days 1, 29, and 57. | | |

### Dose-Limiting Toxicities (DLT)

A DLT was defined as any NCI CTCAE Grade ≥ 3 adverse event occurring within 14 days of study drug administration during the single-dose stage of the study that was determined by the investigator or Sponsor to have any reasonable possible relationship to rhuMAb Beta7. Within this category, Grade 3 infusion related toxicities (e.g., allergic reaction/hypersensitivity, fever, pain, bronchospasm, wheezing, or hypoxia) occurring during an infusion, or within 24 hours after completing an infusion, was considered DLTs. Exacerbation of ulcerative colitis was not considered a DLT.

The MTD was defined as the dose level below the one at which 2 or more patients treated with rhuMAb Beta7 experienced a DLT as defined above. If no more than 1 patient has experienced a DLT in each of the six single-dose cohorts, the MTD has not been defined and the highest tested dose is 10 mg/kg.

### Rationale for Study Design

This study evaluated the safety, tolerability, PK profile, immunogenicity, and exploratory PD markers of rhuMAb Beta7 over short and intermediate periods of time.

### Rationale for Two-Stage Design

The single-dose stage was primarily designed to evaluate acute toxicity. Examples of this type of potential toxicity include hypersensitivity reactions, serum sickness, and local tissue irritation after SC administration. This type of toxicity can occur with any protein therapeutic irrespective of its route of administration. Although the target for rhuMAb Beta7 is the β7^{high} gut-homing subpopulation of lymphocytes, which account for a small proportion (approximately 2%-10%) of the total lymphocyte population in humans (Rott et al., J Immunol 156:3727-36, 1996), any infusion reactions were monitored closely.

Multiple dose administration allowed for evaluation of toxicity with repeat SC and IV doses of rhuMAb Beta7 with a follow up of 28 weeks. To better evaluate the potential for immunogenicity in an IBD population, study drug was administered to patients once every 4 weeks for three cycles in the multidose stage. In addition, evaluation of multidose pharmacokinetics, exploratory PD markers, and evidence of biological activity was enabled during the multi-dose phase of the study and facilitated the selection of doses and dose intervals for the Phase II studies (see below).

### Rationale for Study Population

Patients in this study were required to have ulcerative colitis with an MCS of ≥ 5 at baseline and be considered by the investigator to be eligible for biologic therapy, in that they had moderate to severe disease that had demonstrated an inadequate response to standard therapy with 5-ASA drugs, immunosuppressives (azathioprine or 6 mercaptopurine), or steroids. Any patient with a history of clinically significant opportunistic infection, chronic or latent infection, active systemic infection, or suppression of WBC counts was excluded from taking part in the study. This patient population was selected to best approximate the eventual target population for this therapeutic, while minimizing the potential risk of increased susceptibility to infections introduced by concomitant treatment with high-dose corticosteroids and immunosuppressive drugs.

### Rationale for Starting Dose, Dose Range, and Dosing Regimen

Selection of Phase I doses was based on a combination of nonclinical safety factors, projected human pharmacokinetics, and exploratory nonclinical PD data. Multiple dosing was incorporated into this study to determine potential immunogenicity of repeat dosing and to assess the safety of cumulative exposure of rhuMAb Beta7. The dose levels and frequency proposed provided an opportunity to evaluate the relationship between the PK profile, exploratory PD profile, and any evidence of biological activity in patients with ulcerative colitis. This facilitated the selection of the appropriate dose range and dose frequency for a Phase II proof-of-concept study (see below).

RhuMAb Beta7 PK data from cynomolgus monkeys was used to determine saturation of β7 receptors (Intn'l Patent Pub. No. WO2009/140684; Stefanich et al., Br. J. Pharmacol., Accepted Article, doi: 10.1111/j.1476-5381.2011.01205.x). Data collected following single dose administration of 1, 3, and 10 mg/kg rhuMAb Beta7 suggested nonlinear pharmacokinetics below 3 mg/kg, indicating that doses of 3 mg/kg or above are needed to fully saturate β7 receptor-mediated clearance. Therefore, the dose of 1 mg/kg in the cynomolgus monkey appeared to be a non-receptor-saturating dose; the corresponding mean observed maximum serum concentration at this dose was 25.1 µg/mL. The human equivalent dose (HED) for the 1 mg/kg non-saturating dose in cynomolgus monkeys is approximately 0.3 mg/kg; this proposed starting single dose of 0.3 mg/kg would not be expected to result in serum concentrations of > 10 µg/mL, and therefore should not be a saturating dose.

The proposed starting dose in humans, 0.3 mg/kg rhuMAb Beta7 administered by IV infusion, was selected to evaluate any acute reactions as a result of study drug administration and was supported by a nonclinical toxicology study in cynomolgus monkeys (Intn'l Patent Pub. No. WO2009/140684; Stefanich et al., Br. J. Pharmacol., Accepted Article, doi: 10.1111/j.1476-5381.2011.01205.x). Single-dose safety factors for the starting dose were estimated on the basis of the NOAEL of ≥ 50 mg/kg, with doses given once weekly for 12 weeks (Intn'l Patent Pub. No. WO2009140684), the projected human clearance (CL) and the projected human volume of central compartment (V₁). The safety factors are 53.7 on the basis of HED, 76.2 on the basis of exposure (AUC), 167 on the basis of dose, and 175 on the basis of expected maximum concentration (Cmax) (see Table 3). At the highest proposed multidose regimen of 4 mg/kg IV for a total of three doses, the safety factors are 16.1 on the basis of HED, 21.1 on the basis of AUC, 50 on the basis of dose, and 34.4 on the basis of Cmax (see Table 3).

The rationale for selecting a starting dose of 0.3 mg/kg using NOAEL rather than MABEL is based on several factors. First, targeting integrins is not novel, as α4β7 has been targeted in the clinic previously by both natalizumab and MLN02 (also referred to as MLN0002 or vedolizumab) with no acute serious adverse events (Feagan et al., N Engl J Med 352:2499-507 (2005); Sandborn et al., N Engl J Med 353:1912-25 (2005)). Second, the proposed mechanism of action of rhuMAb Beta7 is to block lymphocyte trafficking to the gut and possibly to block retention of lymphocytes in the epithelium. This mechanism of action has not been shown to be associated with acute adverse events with previous integrin antagonists. Furthermore, rhuMAb Beta7 has not demonstrated any apparent agonistic properties, nor has it induced cytokine release in vitro. Finally, rhuMAb Beta7 has not demonstrated any antibody-dependent cell mediated cytotoxicity or complement-dependent cytotoxicity activity in vitro up to concentrations of 10 µg/mL. There has also been no evidence of change in peripheral cell subsets in the nonclinical studies completed in mice and cynomolgus monkeys. Therefore, 0.3 mg/kg was considered to be a reasonable starting dose for this first in human trial conducted in ulcerative colitis.

**Table 3. Single-Dose and Repeat-Dose Safety Factor Estimates for rhuMAb Beta7.**

| Proposed Human Dose | | | | Safety Factors | |
|---|---|---|---|---|---|
| Dose Level^{a} | Dose (mg/kg) | No. of Doses | Total Dose (mg/kg) | Single Dose | Repeat Dose |
| | | | | HED (mg/kg) ^{b} | |
| Single dose, low | 0.3 | 1 | 0.3 | 53.7 | 644 |
| Single dose, high | 10 | 1 | 10 | 1.61 | 19.3 |
| Repeat dose | 4 | 3 | 12 | 1.34 | 16.1 |

| | | | | Dose (mg/kg)^{c} | |
|---|---|---|---|---|---|
| Single dose, low | 0.3 | 1 | 0.3 | 167 | 2000 |
| Single dose, high | 10 | 1 | 10 | 5.00 | 60.0 |
| Repeat dose | 4 | 3 | 12 | 4.17 | 50.0 |

| | | | | Exposure (AUC) ^{d} | |
|---|---|---|---|---|---|
| Single dose, low | 0.3 | 1 | 0.3 | 76.2 | 843 |
| Single dose, high | 10 | 1 | 10 | 2.29 | 25.3 |
| Repeat dose | 4 | 3 | 12 | 1.91 | 21.1 |

| | | | | Concentration (*C*ₘₐₓ)^{e} | |
|---|---|---|---|---|---|
| Single dose, low | 0.3 | 1 | 0.3 | 175 | 459 |
| Single dose, high | 10 | 1 | 10 | 5.26 | 13.8 |
| Repeat dose | 4 | 3 | 12 | 13.2 | 34.4^{f} |

| | | | | | |
|---|---|---|---|---|---|
| AUC = area under the serum concentration-time curve; AUC_{inf} = area under the serum concentration-time curve from Time 0 to infinity; CL=clearance; *C*ₘₐₓ = maximum concentration observed; HED = human equivalent dose; IV=intravenous; NOAEL = no observed adverse effect level. ^{a}Lowest single dose is 0.3 mg/kg IV. Highest single dose is 10 mg/kg IV. Highest repeat dose is 4 mg/kg IV every 4 weeks for three total doses IV (parallel enrollment). ^{b}Safety factor= HED/Doseₕ, where h is human. The conversion of the cynomolgus monkey NOAEL to the HED was based on body weight and was calculated by dividing the NOAEL by 3.1. Assuming a NOAEL of ≥ 50 mg/kg×12, the HED is 16.1 mg/kg for single-dose safety factors and 193 mg/kg (16.1 mg/kg×12) for repeat-dose safety factors. ^{c}Safety factor= Dose_{c}/Doseₕ, where c is cynomolgus monkey and h is human. Assuming a NOAEL of ≥ 50 mg/kg×12, Dose_{c} is 50 mg/kg for single-dose safety factors and 600 mg/kg (50 mg/kg×12) for repeat-dose safety factors. ^{d}Safety factor= AUC_{c}/AUCₕ, where c is cynomolgus monkey and h is human. For single-dose safety factors, AUC_{c} was calculated using the relationship AUC_{c} = Dose_{c}/CL_{c}. CL_{c} of 2.93 mL/day/kg was determined by calculating the mean CL following four weekly doses of 25 mg/kg in cynomolgus monkeys. Assuming a NOAEL of ≥ 50 mg/kg, the AUC_{c} was 17065 day•µg/mL for single-dose safety factors. For repeat-dose safety factors, an AUC_{c} of 188846 day•µg/mL was determined by calculating the geometric mean AUC_{inf} for the 50 mg/kg×12 IV dose group. AUCₕ was estimated using the relationship AUCₕ = doseₕ/(CLₕ). The most conservative estimate of CLₕ was used to calculate exposure-based safety factors. A CLₕ was estimated using the species invariant-time method (Dedrick 1973). The estimated AUCₕ for IV administration of a 0.3 mg/kg single dose, a 10 mg/kg single dose, and three 4 mg/kg doses was 224, 7463, and 8955 day•µg/mL, respectively. ^{e}Safety factor = *C*_{max-c}/*C*ₘₐₓ₋ₕ, where c is cynomolgus monkey and h is human. *C*_{max-c} is the geometric mean of the maximum observed concentration in cynomolgus monkeys. Assuming a NOAEL of ≥ 50 mg/kg, the geometric mean *C*_{0-c} observed following a single IV administration of 50 mg/kg was 1315 µg/mL. The geometric mean *C*_{max-c} observed following 12 IV doses at 50 mg/kg was 3444 µg/mL. *C*ₘₐₓ₋ₕ was estimated and determined to be similar to that observed for other IgG1 antibodies in humans. The estimated *C*ₘₐₓ₋ₕ for IV administration of a 0.3 mg/kg single dose, a 10 mg/kg single dose, and three 4 mg/kg doses was 7.5, 250, and 100 µg/mL, respectively. ^{f}Safety factor is based on a predicted *C*ₘₐₓ₋ₕ estimated as dose/V₁, which assumes no accumulation following repeat dose of 4 mg/kg, given every 4 weeks for total of three doses. | | | | | |

Selection of a target dose range and dosing regimen were estimated on the basis of simulated serum rhuMAb Beta7 concentration-time profiles projected to maintain steady state trough levels above the target concentration of 1-10 µg/mL. This target concentration is based on results from in vivo studies of rhuMAb Beta7 in mice and cynomolgus monkeys, which showed that a trough concentration of 1-10 µg/mL was necessary to maintain occupation of β7 receptors on circulating peripheral blood T-cells. The occupation of β7 receptors on circulating peripheral blood T-cells is an exploratory PD marker that was assessed in the Phase I study. Until it is known how this marker will correlate with clinical activity, the relevance of the 1-10 µg/mL target concentration to clinical efficacy is also unknown.

The planned dose-escalation scheme was expected to optimize the ability to evaluate the safety of rhuMAb Beta7 when administered over a broad range of doses and exposures. Undue or unexpected accumulation of study drug following IV administration every 4 weeks was not anticipated.

### Outcome Measures

The primary outcome measures for this study were as follows: (1) Incidence, nature, and severity of adverse events (graded according to the NCI CTCAE, Version 3.0); and (2) incidence and nature of laboratory abnormalities, based on hematology, clinical chemistry, and urinalysis test results.

The secondary outcome measures for this study were PK profile and parameters derived from the serum concentration-time profile following administration of rhuMAb Beta7, including those parameters listed: Cmax; CL, or apparent CL (CL/F) for drugs given subcutaneously; Volume of distribution (V) or apparent volume of distribution (V/F) for drugs given subcutaneously; AUC; Elimination half-life (t1/2); Dose proportionality; and SC bioavailability. The secondary outcome measures also include incidence of antibodies directed against rhuMAb Beta7, based on samples obtained at multiple timepoints before and after dosing for each patient.

Certain exploratory outcome measures were also investigated. These include change from baseline in PD markers (which may include receptor occupancy by rhuMAb Beta7, relative cell surface β7 receptor level, and/or β7 integrin expressing T-cell subsets), as measured by flow cytometry of lymphocyte subsets; and change from baseline in modified MCS.

### Study Population

Outpatients with moderate to severe ulcerative colitis and an MCS of ≥ 5 were selected to take part in the study. If patients had received high-dose corticosteroids for acute management of their disease, the dose was reduced to ≤ 20 mg/day of prednisone, or prednisone equivalent, for a minimum of 2 weeks prior to study drug administration.

At any time during the study, patients who had a flare of their ulcerative colitis were permitted to have rescue therapy with steroids (IV, oral, and/or topical). Addition of or increases in doses of 5-ASA (oral or topical) and/or immunosuppressants (i.e., azathioprine, 6 mercaptopurine, or methotrexate) were also allowed as rescue therapy. Use of anti-TNF agents (e.g., infliximab, adalimumab, certolizumab), Tysabri® (natalizumab), cyclosporine, tacrolimus, or any investigational agents was not allowed as rescue therapy during the course of the study.

Patients in the multidose stage of the study who received rescue therapy for ulcerative colitis flare did not receive further doses of study drug but did continue to undergo follow-up for safety, PK, exploratory PD, and ATA assessments.

Any patient with suspected clinically significant immune suppression, based on frequency and severity of previous opportunistic infections and evidence of diminished WBC counts in the peripheral blood at screening were not be included in the study. Because a proportion of patients may have received prior treatment with infliximab, patients were required to have discontinued anti-TNF therapy at least 12 weeks prior to initiating treatment in this study. Infliximab has a half life of 8-9 days; therefore, a washout period of 12 weeks (approximately 10 half lives) was considered adequate for drug clearance of the biologic, ensuring patient safety and fair evaluation of any rhuMAb Beta7-associated adverse events.

Prior to taking part in the study, patients were screened specifically for any evidence of infection, in particular JC virus (measured by plasma JCV DNA), cytomegalovirus (CMV) infection of the colon (by colonic biopsy), respiratory, helminthic, or parasitic infection. Patients were excluded if any active infection was found.

### Inclusion Criteria

Patients must have met the following criteria to be eligible for study entry: (1) Able and willing to provide written informed consent; (2) 18-70 years of age; (3) Males and females with reproductive potential: Willing to use a reliable method of contraception (e.g., hormonal contraceptive, patch, vaginal ring, intrauterine device, or physical barrier) from study start to a minimum of 4 months (approximately 5 half-lives) after the last dose of study drug; (4) Diagnosis of moderate to severe ulcerative colitis with an MCS ≥ 5 points at screening (see above); (5) Chest X-ray without clinically significant abnormalities within 6 months prior to screening; (6) Eligible to receive biologic therapy in the opinion of the investigator, in that they have moderate to severe disease which has demonstrated an inadequate response to standard therapy with 5-ASA drugs, immunosuppressives (azathioprine, 6 mercaptopurine, or methotrexate), or steroids. In the United States, patients who are taking part in the MD stage must have demonstrated an inadequate response, or intolerance, to both immunosuppressant and anti-TNF therapies; (7) Disease duration at time of screening of ≥ 12 weeks (after first diagnosis by a physician); (8) Patients who are on high-dose corticosteroids must have the dose reduced to ≤ 20 mg/day of prednisone, or prednisone equivalent, for at least 2 weeks prior to dosing with study drug on Day 1. Topical corticosteroids and topical 5 ASA preparations must be withdrawn for at least 1 week prior to dosing with study drug on Day 1; (9) Patients who are on oral 5-ASA must be on a stable dose or discontinue for at least 4 weeks prior to dosing with study drug on Day 1; (10) Patients who previously received anti-TNF therapy must have discontinued therapy for at least 12 weeks prior to dosing with study drug on Day 1; (11) Immunosuppressant therapy for the single-dose stage: In the United States: patients must discontinue immunosuppressant therapy (i.e. azathioprine, 6-mercaptopurine, or methotrexate) at the time of dosing with study drug on Day 1. Outside the United States: at the investigator's discretion, patients may discontinue immunosuppressant therapy (i.e., azathioprine, 6 mercaptopurine, or methotrexate) at the time of dosing with study drug on Day 1, or may continue to receive these therapies at a stable dose during the study; (12) Immunosuppressant therapy for the multiple-dose stage: In the United States: At the investigator's discretion, patients may discontinue immunosuppressant therapy (azathioprine, 6 mercaptopurine, or methotrexate) at the time of first dosing with study drug on Day 1, or may continue to receive these therapies at a stable dose for up to 6 weeks (Day 43) during the study. At Day 43, however, all patients must have their immunosuppressant therapy discontinued for the remainder of the MD stage of the study (through Day 197). In addition, for patients who are receiving steroids, tapering must commence at Day 57 in patients who have achieved clinical response, or at the subsequent visit when clinical response is achieved. Clinical response will be judged by the investigator and will take into account the Day 43 flexible sigmoidoscopy. Outside the United States: At the investigator's discretion, patients may discontinue immunosuppressant therapy (azathioprine, 6 mercaptopurine, or methotrexate) at the time of first dosing with study drug on Day 1, or may continue to receive these therapies at a stable dose during the study.

### Exclusion Criteria

Patients who met any of the following criteria were excluded from study entry. The exclusion criteria related to UC included: (1) Requirement for hospitalization due to severity of ulcerative colitis; (2) Moderate to severe anemia (hemoglobin < 10 g/dL); (3) Any manifestation of ulcerative colitis likely to require, in the investigator's judgment, treatment with > 20 mg/day of prednisone, or prednisone equivalent, during the course of the study.

The exclusion criteria related to general health included: (1) Pregnant or lactating; (2) Lack of peripheral venous access; (3) Inability to comply with study protocol, in the opinion of the investigator; (4) Positive stool test for ova or parasites, positive stool culture for pathogens, or positive stool toxin assay for Clostridium difficile at screening; (5) Significant uncontrolled disease, such as neurological, cardiac, pulmonary, renal, hepatic, endocrine, or GI disorders; (6) Significant screening ECG abnormalities, including evidence of acute myocardial infarction, complete left bundle branch block, second-degree heart block, or complete heart block; (7) Poorly controlled diabetes (glycosylated hemoglobin [HbA1c] > 8.0%); (8) Conditions other than ulcerative colitis (e.g., asthma) that could require treatment with > 20 mg/day of prednisone, or prednisone equivalent, during the course of the study; (9) History of malignancy except completely excised basal cell carcinoma or squamous cell carcinoma of the skin; (10) Impaired renal function (creatinine > 1.5 × upper limit of normal [ULN]); (11) Impaired hepatic function (transaminases > 2.5 × ULN, or abnormalities in synthetic function tests judged by the investigator to be clinically significant).

The exclusion criteria related to risk factors for infections included: (1) Congenital immune deficiency; (2) Serum immunoglobulins IgA and IgG (including IgG subclasses 2 and 3) below the laboratory lower limit of normal; (3) Positive test for serum JCV DNA; (4) Positive tests for antibodies indicating active or prior infection with HIV or hepatitis B or C; (5) Positive IgM antibody titers in the presence of negative IgG titers to Epstein Barr virus (EBV); (6) Intestinal mucosa biopsy positive for CMV; (7) Positive screening test for latent mycobacterium tuberculosis infection Purified protein derivative (PPD) skin test or lab assays such as QuantiFERON®-TB Gold (QFT-G, or equivalent blood test) are acceptable screening assays. QFT-G (or equivalent blood test) should be used in patients previously vaccinated with Bacille Calmette-Guérin (BCG). If a negative PPD test has been documented within 3 months prior to screening, it does not need to be repeated; (8) History of severe systemic bacterial, fungal, or parasitic infections (more than two hospitalizations per year or more than two courses of IV antibiotics per year); (9) History of invasive fungal infections such as Candida or Aspergillus (excluding thrush or other superficial fungal infections) within 6 months prior to initiation of study treatment; (10) History of severe herpes (simplex type 1, simplex type 2, or zoster) infection or reactivation within 12 weeks prior to initiation of study treatment, or frequent recurrence of herpes (more than 2 times per year); (11) History of any other opportunistic infections within 12 weeks prior to initiation of study treatment; (12) History of PML; (13) Any current or recent (within 4 weeks prior to initiation of study treatment) signs or symptoms of infection; (14) Received oral antibiotics within 4 weeks prior to initiation of study treatment, or IV antibiotics within 8 weeks prior to initiation of study treatment; (15) Received a live attenuated vaccine within 4 weeks prior to screening; (16) Hospitalized within 4 weeks prior to screening.

The exclusion criteria related to medications included: (1) Received any investigational treatment within 12 weeks prior to screening (or within 5 half lives of the investigational product, whichever is greater); (2) Patients who completed the SD stage of this study but did not receive rhuMAb Beta7 (i.e., received placebo) will not be excluded from participating in the MD stage; (3) Received any biologic therapy within 12 weeks prior to screening; (4) Thrombocytopenia (platelet count < 75,000/µL), Neutropenia (absolute neutrophil count < 1500/µL), or Lymphopenia (absolute lymphocyte count < 800/µL); (5) Received cyclosporine or tacrolimus (FK506) within 3 months prior to initiation of study treatment

### Assay Methods

### JCV DNA Assessment

Patients had JCV DNA tested in the serum at baseline and then every 4 weeks during the study, using the JCV DNA DetectR™ Qualitative Assay (#8145), with a positive test classified as 80 copies/mL or above. A quantitative JCV DNA assay (JCV DNA Ultraquant™ Quantitative Assay [#8147]) was performed for patients who become JCV DNA-positive during the study. This assay has a lower detection limit of 80 copies/mL.

### Pharmacokinetic and Anti-Therapeutic Antibody (ATA) Assessments

Serum samples were obtained from all patients for determination and characterization of the pharmacokinetics of rhuMAb Beta7 and for assessment of ATAs. Serum levels of rhuMAb Beta7 and ATAs for the clinical samples were examined by immunometric methods as part of the testing program described below.

The testing program for ATAs followed the overall guidance for ATA tests (Mire-Sluis et al. J Immunol Methods 289:1-16 (2004)). The testing program for ATAs had two tiers: a screening tier and a characterization tier. The screening tier had an initial screening test of serum samples, a confirmatory test for those who tested positive in the initial screen, and titering of confirmed positive samples. Positive sera were banked for possible future use. Screening tier assays were validated. Because high serum levels of rhuMAb Beta7 were expected to interfere with detection of ATAs, washout samples were obtained after administration of the final dose of rhuMAb Beta7, when drug levels were anticipated to fall below levels expected to interfere with the assay.

### Pharmakokinetic Assay

A sandwich ELISA with a colorimetric detection system was validated to quantify rhuMAb Beta7 (PRO145223) in human serum. Microtiter plates were coated with an anti-rhuMAb Beta7 antibody at 1.0 µg/mL to capture rhuMAb Beta7. Diluted samples, standards, and controls were added to the plate and incubated. Subsequently, biotinylated anti-rhuMAb Beta7 and streptavidin conjugated to horseradish peroxidase (HRP) were added for detection and incubated. A peroxidase substrate (tetramethyl benzidine) was added to develop color, and the reaction was stopped by adding 1 M phosphoric acid. The plates were read at 450 nm for detection absorbance and at 620 or 630 nm for reference absorbance. The minimum quantifiable concentration of this assay was 12.5 ng/mL.

### Anti-therapeutic Antibody Assay

An antibody bridging Electrochemiluminescence Assay (ECLA) was validated to detect antibodies to PRO145223 (rhuMAb Beta7) in human serum. The assay used PRO145223 conjugated to biotin and PRO145223 conjugated to Sulfo-TAG to capture antibodies direct against PRO145223. The biotinylated rhuMAb Beta7 conjugate at 2 µg/mL and the Sulfo-TAG rhuMAb Beta7 conjugate at 2 µg/mL were added to a 96-well polypropylene microplate (Corning). Subsequently, diluted samples and controls were added to the microplate and co-incubated with the conjugates overnight. A streptavidin coated Meso Scale Discovery® (MSD; Gaithersburg, MD) plate was also blocked with assay diluent overnight at 2°C -8°C. The samples from the polypropylene plate were then transferred to the blocked MSD plate and incubated at room temperature for 2 hours. The MSD plate was then washed three times with wash buffer. MSD 2x Read Buffer T was added to the MSD plate, and the plate was read on the MSD Sector Imager 6000 reader. Antibody titer values were determined by a log titer data reduction program.

During the assay validation, the minimum reportable titer was determined to be: Log₁₀(Minimum Dilution) = 1.30 titer units.

### Pharmacodynamic Assessments

Peripheral blood samples were drawn from patients and exploratory PD markers were measured, in real time, by fluorescence-activated cell sorting (FACS) analysis of peripheral blood lymphocyte subsets using standard flow cytometry methods. The data was analyzed for each cohort after all samples were measured.

The beta7 assay was designed to assess beta7 occupancy and expression using PE (Phycoerythrin) conjugated rhuMAbBeta7 (competing antibody) to detect the free sites available pre- and post-dosing along with a PE conjugated anti-beta7 antibody (non-competing antibody), which was not inhibited by the presence of rhuMAbBeta7 and provided assessment of total beta7 expression during dosing.

Breifly, 100mL Sodium Heparin anticoagulated whole blood was added to appropriately labeled staining tubes on ice and incubated for 30 minutes. Post incubation, a cocktail containing appropriate antibody cocktails was added to the appropriate tubes and incubated 30 minutes in the dark on ice. The cells were then washed with phosphate-buffered saline containing 2% normal calf serum and then finally resuspended in 1% paraformaldehyde and stored at 2-8 °C until acquisition on a Becton Dickinson FACSCalibur flow cytometer. The tubes were acquired with a stop count of 50,000 total events.

### RESULTS

The patient baseline characterstics for both the single dose stage and the multidose stage are shown in the following two tables.

**Table 4. Patient baseline characteristics for the SAD stage.**

| | Cohort A (0.3 mg/kg IV) n=4 | Cohort B (1 mg/kg IV) n=4 | Cohort C (3 mg/kg SC) n=4 | Cohort D (10 mg/kg IV) n=4 | Cohort F (3 mg/kg SC) n=4 | Placebo n=5 | Total n=25 |
|---|---|---|---|---|---|---|---|
| Age Median (Range) | 39 (36-53) | 45 (25-64) | 46 (34-47) | 41 (29-48) | 35 (19-41) | 26 (21-49) | 38 (19-64) |
| Sex (M:F) | 4:0 | 4:0 | 3:1 | 3:1 | 1:3 | 2:3 | 17:8 |
| Baseline MCS Median (Range) | 10 (6-11) | 9 (8-9) | 10 (8-11) | 10 (8-12) | 8 (5-9) | 9 (7-10) | 9 (5-12) |
| On Steroids | 3 | 2 | 1 | 2 | 2 | 3 | 13 |
| On Immunosuppressants | 1 | 1 | 2 | 1 | 2 | 0 | 7 |
| On 5-ASA | 3 | 1 | 2 | 3 | 3 | 3 | 15 |
| Disease Duration (years) Median (Range) | 6.5 (4-9.2) | 12.9 (5.7-24) | 14.9 (6.9-22.8) | 8.9 (2.7-19.6) | 6.7 (2.4-8.3) | 6 (4.6-29.9) | 7.5 (2.4,29.9) |

**Table 5. Patient baseline characteristics for the MAD stage.**

| | Cohort G (0.5 mg/kg SC) n=4 | Cohort H (1.5 mg/kg SC) n=5 | Cohort I (3 mg/kg SC) n=4 | Cohort J (4 mg/kg IV) n=5 | Placebo n=5 | Total n=23 |
|---|---|---|---|---|---|---|
| Age Median (Range) | 49 (31-57) | 56 (33-63) | 39 (25-57) | 41 (20-55) | 31 (20-62) | 46 (20-63) |
| Sex (M:F) | 2:2 | 3:2 | 3:1 | 5:0 | 3:2 | 16:7 |
| Baseline MCS Median (Range) | 10.5 (7-12) | 11 (8-11) | 8.5 (4-10) | 9 (6-10) | 10 (8-11) | 10 (4-12) |
| On Steroids | 3 | 2 | 2 | 1 | 4 | 12 |
| On Immunosuppressants | 0 | 2 | 0 | 3 | 1 | 6 |
| On 5-ASA | 4 | 3 | 2 | 3 | 3 | 15 |
| Previous anti-TNF therapy | 4 | 3 | 2 | 3 | 3 | 15 |
| Disease Duration (years) Median (Range) | 6 (4-17.2) | 8.2 (1.6-23.9) | 10.1 (4-29.9) | 5.2 (2.7-19.7) | 5.3 (1.6-25.2) | 6 (1.6,29.9) |

As indicated above, the primary outcome measures of this Phase I study were safety and tolerability. For the single dose stage, we did not observe any dose limiting toxicities and no infusion or injection site reactions. Seven patients experienced serious adverse events (six receiving rhuMAb Beta7 and one receiving placebo) in the single-dose stage. There were two serious adverse events related to impaired wound healing in two patients receiving active treatment who underwent urgent colectomy for exacerbation of UC, however, there were significant confounding factors. There were no clinically significant injection site reactions in the multiple dose stage of the study. There was one serious adverse event in the multidose stage of the study.

There were no concerning safety signals identified for rhuMAb Beta7 at the doses (single and multiple) tested in the Phase I study. The protocol-defined criteria for DLT were not met in any of the dose cohorts. The overall incidence of AEs was generally comparable between cohorts, with no apparent trend toward a dose-dependent increase in the incidence of AEs. The majority of AEs were NCI CTCAE Grade 1 or 2. Seven patients who received rhuMAb Beta7 in the Phase I study had SAEs reported: six were in the SD stage, and one was in the MD stage. There was no apparent dose-related increase in the incidence of infection related events in the SD stage of the study. No severe infusion or injection-related AEs were reported in either stage of the study. There did appear to be an increased incidence of AEs that were reported within 24 hours after study drug administration among rhuMAb Beta7-treated patients (45% of patients in the SD stage and 55.6% of patients in the MD stage) compared with placebo (20% of patients in both the SD and MD stage). However, all AEs occurring within 24 hours after study drug administration were NCI CTCAE Grade 1 or 2.

Blood samples from each of the patients were collected at certain time points throughout the study and serum rhuMAb Beta7 concentration was measured to assess PK. The PK assessment was performed for available serum concentration-time data through use of standard non-compartmental (NCA) PK methods and the computer software WinNonlin Pro version 5.1.1 (Pharsight). Actual blood sampling times were used. The results are shown in Fig.1. Fig. 1A shows the results for the SAD stage of the study, Fig. 1B shows the PK profile of a single dose of rhuMAb Beta7 administered at 3.0 mg/kg IV compared to 3.0 mg/kg SC, and Fig. 1C shows the results for the MAD stage of the study.

Analysis of the data presented in Fig. 1A shows that serum rhuMAb-Beta7 concentrations exhibited a biphasic disposition with an initial rapid distribution phase followed by a slow elimination phase. Upon IV administration, the Cₘₐₓ increased in a dose-proportional manner and averaged at 9.33 µg/mL for the 0.3-mg/kg dose group and 239 µg/mL for the 10-mg/kg dose group. Similarly, the group mean area under the concentration-time curve (AUC_{0-inf}) ranged from 76.9 to 2500 day×µg/mL and was approximately dose proportional, as evidenced by the similar dose-normalized AUC values across the dose range. The terminal elimination half-life was approximately 2 weeks (∼14 days). The PK profiles of lower doses (0.3 mg/kg and 1 mg/kg) suggest a slight trend of nonlinear PK, possibly because of the contribution of a target-mediated clearance at lower concentrations.

Analysis of the data presented in Fig. 1B shows that the elimination of the SC cohort was generally consistent with the IV cohort. The preliminary assessment comparing AUC_{0-inf} values of single 3 mg/kg SC dose cohort to AUC_{0-inf} from single 3 mg/kg IV dose cohort estimated an approximate bioavailablility of 66-67%.

Analysis of the data presented in Fig. 1C shows that, consistent with the single-dose PK data, rhuMAb Beta7 serum exposure was generally dose proportional. The once-every-4-week dosing schedule resulted in minimal or moderate drug accumulation over time. The highest dose of 4 mg/kg IV cohort had an average (± standard deviation) Cₘₐₓ of 117 ± 31 and 136 ± 24 µg/mL after the first and the final dose, respectively. Similar to the single-dose kinetics, the terminal elimination half-lives in multidose cohorts were approximately 2 weeks.

A preliminary population PK assessment was performed for data from all single dose cohorts using NONMEM VI (ICON). A univariate exploratory analysis was performed by plotting inter-individual variability (IIV) for clearance (CL) or central volume of distribution (V₁) against body weight (BW) as the covariate. The CL or V₁ of rhuMAb Beta7 did not appear to correlate with BW in this assessment, suggesting that flat dosing could be substituted for BW based dosing.

In summary, the analysis described above indicates general linear pharmacokinetics with a slight trend of non-linear PK at doses < 3 mg/kg. The elimination half-life following single IV doses of 3 and 10 mg/kg was approximately 2 weeks (∼14 days) and the SC bioavailability was approximately 66-67%.

The results of the anti-therapeutic antibody (ATA) analysis revealed that one patient in the SAD cohort that received 0.3 mg/kg IV was positive for ATA by day 29 and remained positive through day 127. This ATA positivity had no impact on PK (data not shown).

Occupancy of integrin beta7 was measured in samples from each of the patients at various times. For the SAD stage of the study, occupancy was maintained at 1-10 µg/mL (data not shown). Fig. 2 shows the results of the occupancy analysis from the MAD stage of the study. The data suggests a dose relationship in duration of beta7 receptor saturation with full occupancy retained on monthly dosing as low as 0.5 mg/kg SC.

We also analyzed the data for evidence of possible clinical activity. One parameter that was investigated is individual changes in Mayo Clinic Score (MCS). These results are shown in Fig. 3. For all patients in the multidose stage, the mean (± sd) baseline MCS was 9.3 + 1.9. Clinical response was defined as a decrease in MCS of 3 points plus 30% reduction from baseline and a ≥ 1 point decrease in rectal bleeding or absolute bleeding score of 0/1. As shown in Figs. 3A-E, at day 71, 12/18 patients treated with rhuMAb Beta7 and 4/5 patients treated with placebo demonstrated clinical response by these criteria. In addition, clinical remission was defined as an absolute MCS ≤ 2 plus no individual subscore >1. Figs. 3A-E show that 3/18 patients treated with rhuMAb Beta7 and 1/5 patients treated with placebo demonstrated clinical remission by these criteria. Of note, clinical activity was observed in rhuMAb Beta7-treated patients who were not on steroids and the clinical activity was more consistent in Cohorts H and I.

### Example 2

### PHASE II RANDOMIZED DOUBLE-BLIND PLACEBO-CONTROLLED STUDY TO EVALUATE THE EFFICACY AND SAFETY OF rhuMAb BETA7 IN PATIENTS WITH MODERATE TO SEVERE ULCERATIVE COLITIS AND OPEN LABEL EXTENSION STUDY

### Study Design

### Description of the Study

This Phase II study is a randomized, double-blind, placebo-controlled multicenter study to evaluate the efficacy and safety across two rhuMAb Beta7 dose levels compared with placebo in patients with moderate to severe UC. This target patient population is the same as that studied in the Phase I study described above. The primary efficacy endpoint will be evaluated at Week 10 (2 weeks after the final dose of study drug is administered) with a secondary efficacy endpoint at Week 6.

Patients will be randomized in a 1:1:1 ratio across a dose range of rhuMAb Beta7 100 mg SC (flat dose) at Weeks 0, 4, and 8 and 420 mg SC (flat loading dose) at Week 0 followed by 300 mg SC at Weeks 2, 4, and 8 or matching placebo SC. The study schema is shown in Figure 7. The study will be divided into a screening period of 0-35 days, a double-blind treatment period of 10 weeks, a safety follow-up period of 18 weeks, and a progressive multifocal leukoencephalopathy (PML) follow up period of 17 months (2 years after randomization).

To be eligible, patients must have a minimum of a 12-week duration of UC diagnosed according to the American College of Gastroenterology (ACG) practice guidelines; that is, clinical and endoscopic evidence corroborated by a histopathology report, with evidence of moderate to severe disease as evidenced by, in certain instances an MCS of ≥ 5, or in certain instances, an MCS of ≥ 6, including an endoscopy subscore of ≥ 2; a rectal bleeding subscore of ≥ 1 (see Table 1); and endoscopic evidence of disease activity a minimum of 25 cm from the anal verge.

Prior to randomization, patients must be on stable doses of concomitant medications for UC. Oral 5-aminosalicylic acid (5-ASA) and immunosuppressant (azathioprine [AZA], 6-mercaptopurine [6-MP], or methotrexate) doses must be kept stable for at least 4 weeks prior to randomization on Day 1. Patients who are receiving topical 5-ASA or corticosteroids must discontinue 2 weeks before randomization on Day 1. Oral corticosteroid doses must be kept stable for 2 weeks prior to randomization on Day 1. Patients receiving high-dose steroids must have the dose reduced to ≤ 20 mg/day for 2 weeks prior to randomization on Day 1. For patients receiving oral corticosteroids during the study treatment period, tapering of steroids must be commenced at Week 10 at a rate of a 5-mg prednisone or prednisone equivalent per week for 2 weeks and then at a rate of 2.5 mg prednisone or prednisone equivalent per week to discontinuation. For patients receiving oral immunosuppressants (other than oral corticosteroids), tapering of immunosuppressants must be commenced at Week 8, and patients must completely discontinue immunosuppressants by Week 10. Patients who have previously received anti-TNF therapy must have discontinued therapy for a minimum of 8 weeks prior to randomization to receive study drug on Day 1. If patients experience persisting or increasing disease activity at any time during the study, rescue therapy in the form of an increase in steroids and or immunosuppressant dose may be increased or initiated according to the investigator's clinical judgment. Patients who require rescue therapy will be permitted to remain in the study but will discontinue study treatment and, during data analysis, will be classified as having experienced treatment failure.

Patients will be assessed to determine whether they failed to respond to conventional therapy, including at least one anti-TNF agent. As used herein, loss of response and/or intolerance to anti-TNF agents and immunosuppressants means the following. With respect to anti-TNF agents, loss of response and/or intolerance means that symptoms of active disease persist despite previous treatment with one or more of (a) infliximab: 5 mg/kg IV, 3 doses over 6 weeks with assessment at 8 weeks; (b) adalimumab: one 160-mg SC does at week 0, followed by one 80-mg dose at week 2 and then 40 mg at 4 and 6 weeks, with assessment at 8 weeks; or recurrent active symptoms during regularly scheduled maintenance dosing following a previous response (elective discontinuation of treatment by patient who has responded and did not lose response does not qualify); or history of intolerance to at least one ant-TNF antibody (including but not exclusive of or limited to infusion-related reaction or injection-site reaction, infection, congestive heart failure, demyelination). With respect to immunosuppressant agents, loss of response and/or intolerance means that symptoms of active disease persist despite previous treatment with one or more of azathioprine (≥ 1.5 mg/kg) or equivalent dose of 6-mercaptopurine mg/kg (≥ 0.75 mg/kg) or methotrexate, 25 mg SC/intramuscular (or as indicated) per week for at least 8 weeks; or history of intolerance of at least one immunosuppressive (including, but not exclusive of pancreatitis, drug fever, rash, nausea/vomiting, liver function test elevation, thiopurine S-methyltransferase genetic mutation, infection).

Randomization to study treatment will be stratified by concomitant treatment with corticosteroids (yes/no), concomitant treatment with immunosuppressants (yes/no), previous anti-TNF exposure (yes/no) (except for patients randomized in the United States), and study site.

UC disease activity will be assessed using the MCS at Screening (and this will be considered the baseline MCS), Week 6 (2 weeks after dosing at Week 4), and Week 10 (2 weeks after the final dose of study drug). Biopsies of the colon will be obtained during the flexible sigmoidoscopy conducted at these same time points. Partial MCS will also be collected throughout the study. Patient Reported Outcomes (PROs) will also be assessed by using a Short Inflammatory Bowel Disease Questionnaire (SIBDQ) and MCS, which will be completed by patients at Day 1 and at Weeks 6 and 10. In addition, disease activity, daily symptoms, and impact of UC will be collected in a patient diary, completed daily by patients from screening (approximately 7 days prior to and up to Day 1) and at least 7 days prior to and up to the study visits at Weeks 6 and 10. Serum and fecal samples will also be obtained for biomarker analysis. Stool will be obtained at screening and Weeks 6, 10, and 28 for measurement of biomarkers. Exemplary biomarkers that will be measured include, but are not limited to, lipocalin, calprotectin, and lactorferrin. Serum and plasma will be obtained at screening, at Day 1, and at Weeks 4, 6, 10, 16, and 28 for measurement of exploratory biomarkers.

The primary efficacy endpoint for this study is the proportion of patients who achieve clinical remission, defined as an absolute reduction in MCS to ≤ 2 with no individual subscore exceeding 1 point, by Week 10. Additional secondary endpoints are listed in the study outcome measures.

### Open Label Extension Study

This study will be an open label extension (OLE) trial to evaluate the safety and tolerability of rhuMAb Beta7 in patients with moderate to severe UC who were enrolled in the Phase II Study described above and who meet the eligibility criteria for entry in the OLE study. This study will enroll approximately 120 patients.

All patients in this OLE study will receive treatment with rhuMAb Beta7 at a dose of 300 mg SC every 4 weeks. The study will be divided into an open-label treatment period of 104 weeks, a post-treatment 12-week safety follow-up, and an extended progressive multifocal leukoencephalopathy (PML) monitoring period of 104 weeks following the last dose of rhuMAb Beta7.

All patients (placebo or active) taking part in the Phase II study described above may enter this OLE study if they fulfill any of the following criteria:
- Have not obtained a clinical response in the Phase II study by Week 10; patients may enter at any time after Week 10 and up to and including Week 28; clinical response is defined as in the Phase II study: at least a 3-point decrease and 30% reduction from baseline in MCS and ≥ 1-point decrease in rectal bleeding subscore or absolute rectal bleeding score of 0 or 1.
- Have had a clinical response (as defined above) in the Phase II study at Week 10 but have had a flare of UC between Week 10 and Week 28; flare of UC is defined as in the Phase II study: a 2-point increase in the partial MCS with 3 days of continuous rectal bleeding and an endoscopy score of 2 on flexible sigmoidoscopy
- Have completed the Phase II study through Week 28 of follow-up.

Patients in the U.S. must also have concomitant immunosuppressive therapy discontinued before entry into the OLE study and must have completely tapered off oral corticosteroids within 24 weeks of enrollment to the OLE study.

### Rationale for Study Design

### Dose Rationale

The rationale for the proposed regimen is summarized below.

The proposed flat doses to be administered SC in the Phase II study-100, 300, and 420 mg-are equivalent to 1.25, 3.75, and 5.25 mg/kg, respectively, for an 80 kg median weight patient. Doses chosen for the Phase II study are expected to provide observed exposure levels that demonstrated preliminary clinical activity in the Phase Ib study (the multidose stage described above). In addition, as illustrated in the serum rhuMAb Beta7-time profile predicted by the simulation with use of preliminary Phase I PK data (Figure 4), the proposed 100- and 420/300-mg dose arms provide an approximate 4.4 fold dose range and a clear exposure separation, after accounting for the interindividual variability (IIV) in exposure. Moreover, the 100 mg dose arm is expected to reach a steady state trough concentration level of 1-10 µg/mL (minimal trough concentration required for receptor occupancy) in all patients, and the 420/300 mg dose arm is anticipated to offer a steady-state trough concentration level above 10 µg/mL in > 90% of the patients. The first loading dose of 420 mg and an additional Week 2 dose of 300 mg in this dose arm are intended to maximize the rhuMAb Beta7 exposure earlier in the treatment course to explore whether an early higher drug exposure will induce a more rapid onset of clinical response. Compared with observed group mean exposure data from Phase I multiple dose 4 mg/kg IV cohort, the predicted exposure within the first 2 or 4 weeks (Day 0-14 or Day0-28) for the proposed high-dose arm has a safety margin of 2.5 (Cₘₐₓ based) or 1.3-2.2 (AUC based) (Table 6).

**Table 6. Safety margin for proposed high dose arm comparing to phase I multiple dose IV cohort exposure.**

| | | | | | **Safety Margin** | | |
|---|---|---|---|---|---|---|---|
| | | **C_{max,1} (ug/mL)** | **AUC₀₋₁₄ (day*ug/mL)** | **AUC₀₋₂₈ (day*ug/mL)** | **C_{max,1} Based** | **AUC₀₋₁₄ based** | **AUC₀₋₂₈ based** |
| **Observed Ph I** | **PhI MAD IV 4 mg/kg (q4wkx3)** | 117.1 | 785.9 | 1128.1 | | | |
| **Simulated PhII** | **420 mg + 300 mg (wks 2, 4, 8)** | 46.4 | 356 | 883.6 | **2.5** | **2.2** | **1.3** |

The flat dose was proposed and justified on the basis of the following considerations. First, a univariate exploratory analysis was conducted in which the interindividual variability (IIV) for clearance (CL) or central volume of distribution (V₁) (obtained from the preliminary base population IV PK model) was plotted against weight as the covariate. The plots of IIV of CL or IIV of V₁ versus weight showed little or no dependence of CL or V₁ on body weight. Second, the variability was compared in the population PK model-predicted steady state maximum serum concentration (C_{max.ss}) and area under the serum concentration-time curve from Day 0 to Day 84 (AUC₀₋₈₄) for patients receiving either a flat dose (300 mg) or an equivalent body weight-based dose (3.75 mg/kg). As demonstrated in Figure 5, median C_{max.ss} and AUC₀₋₈₄ were similar between the flat and body weight-based doses. The dose scenarios simulated and shown in Figure 5 were bodyweight based dose of 3.75 mg/kg and a flat dose of 300 mg. Figure 5 also shows that the IIV of the exposure is similar or slightly smaller in the flat-dose scenario compared with the body weight-based dose scenario.

In summary, the proposed Phase II dosage regimens of 100 mg SC at Weeks 0, 4, and 8 and 420 mg SC at Week 0 followed by 300 mg SC at Weeks 2, 4, and 8 are based on the integrated clinical and PK/PD data and balance the need to satisfy safety and dose ranging, as well as potentially demonstrate clinical activity. The flat dose is supported and justified by the known PK characteristic of rhuMAb Beta7 and the outcome of the exposure variability assessment as described herein.

### Outcome Measures

### Primary Efficacy Outcome Measure

The primary efficacy outcome measure is clinical remission at Week 10. Clinical remission is defined by an MCS ≤ 2 with no individual subscore exceeding 1 point (see Table 1).

### Secondary Efficacy Outcome Measures

The secondary efficacy outcome measures for this study are (1) Clinical response at Week 6 and Week 10 where clinical response is defined by at least a 3-point decrease and 30% reduction from baseline in MCS and a ≥ 1-point decrease in rectal bleeding subscore or absolute rectal bleeding score of 0 or 1; (2) Clinical remission (defined above) at Week 6; and (3) An indicator for endoscopic score and rectal bleeding score of 0 at Week 6 and Week 10.

### Exploratory Outcome Measures

The exploratory outcome measure for this study is the time to flare of UC in patients who have achieved response or remission. For this outcome measure, a flare is defined as a 2 point increase in partial MCS accompanied by 3 days of continuous rectal bleeding and an endoscopy score of 2 on flexible sigmoidoscopy.

### Safety Outcome Measures

The safety and tolerability of rhuMAb Beta7 will be assessed using the following measures: (1) Incidence of adverse events and serious adverse events graded according to National Cancer Institute Common Terminology Criteria for Adverse Events (NCI CTCAE) Version 4.0; (2) Clinically significant changes in vital signs and safety laboratory measures; (3) Discontinuation due to adverse event(s); (4) Incidence and nature of injection-site reactions and hypersensitivity; (5) Incidence of infectious complications; and (6) Immunogenicity as measured by the incidence of ATAs.

### Pharmacokinetic Outcome Measures

The pharmacokinetic outcome measures include the following: (1) Cₘₐₓ after the first and final doses; (2) Time to maximum concentration (Tₘₐₓ) after the first and final doses; (3) Area under the serum concentration-time curve (AUC) within a dose interval after the final dose; (4) AUC from time 0 to time of the last detectable observation (AUCₗₐₛₜ) or to infinity (AUC_{inf}); (5) Apparent clearance (CL/F); (6) Apparent volume of distribution (V/F); and (7) Elimination half-life (t_{1/2}).

### Inclusion Criteria

Patients must meet the following criteria to be eligible for study entry: (1) Able and willing to provide written informed consent; (2) 18-75 years of age; (3) Males and females with reproductive potential must be willing to use a highly effective method of contraception (e.g., hormonal contraceptive [oral or patch], vaginal ring, intrauterine device, physical barrier, or vasectomized partner) from study start to a minimum of 4 months (approximately 5 half lives) after the final dose of the study drug; (4) Diagnosis of moderate to severe UC outpatient with, in certain instances an MCS of ≥ 5, or in certain instances, an MCS of ≥ 6, including an endoscopy subscore ≥ 2; a rectal bleeding subscore ≥ 1 (see Table 1) and disease activity a minimum of 25 cm from the anal verge; (5) Disease duration at time of screening of ≥ 12 weeks (i.e., after first diagnosis by a physician according to ACG guidelines); (6) Patients who are receiving corticosteroids must have been on a stable dose of no more than 20 mg/day of prednisone or prednisone equivalent for at least 2 weeks prior to dosing with study drug on Day 1; (7) Topical corticosteroids and topical 5 ASA preparations must have been withdrawn for at least 2 weeks prior to dosing with study drug on Day 1; (8) Patients who are on oral 5-ASA must be on a stable dose for at least 4 weeks prior to dosing with study drug on Day 1; (9) Patients who are receiving immunosuppressants in the form of AZA, 6-MP, or methotrexate should be on a stable dose for 4 weeks prior to dosing with study drug on Day 1. Patients taking methotrexate should be recommended to also take 1 mg/day folic acid (or equivalent) supplementation if no contraindication; (10) Patients on probiotics (e.g., Culturelle, Saccharomyces boulardii) must be on stable doses for 2 weeks prior to dosing with study drug on Day 1; (11) Patients on antidiarrheals (e.g., loperamide, diphenoxylate with atropine) must be on stable doses for 2 weeks prior to dosing with study drug on Day 1; (12) Patients who have previously received anti-TNF therapy must have discontinued therapy for a minimum of 8 weeks prior to dosing with study drug on Day 1; (13) Patients previously treated with cyclosporine or tacrolimus (FK506) must have discontinued therapy for a minimum of 4 weeks prior to initiation of study drug on Day 1; (14) Patients previously treated with tube feeding, defined formula diets, or parenteral alimentation/nutrition must have discontinued these 3 weeks prior to initiation of study drug on Day 1; (15) Patients with a diagnosis of moderate to severe UC need to be nonresponsive or intolerant of immunosuppressants (for example, AZA, 6-MP, or methotrexate) and at least one anti-TNF agent.

### Exclusion Criteria

Patients who meet any of the following criteria will be excluded from study entry. The exclusion criteria related to UC include: (1) Extensive colonic resection or subtotal or total colectomy; (2) Presence of an ileostomy or colostomy; (3) Moderate to severe anemia (hemoglobin < 9 g/dL); (4) A history or evidence of colonic mucosal dysplasia.

The exclusion criteria related to general health include: (1) Pregnant or lactating; (2) Lack of peripheral venous access; (3) Inability to comply with study protocol, in the opinion of the investigator; (4) Significant uncontrolled co-morbidity, such as neurological, cardiac, pulmonary, renal, hepatic, endocrine, or gastrointestinal (GI) disorders; (5) Significant screening ECG abnormalities, including evidence of acute myocardial infarction, complete left bundle branch block, second-degree heart block, or complete heart block; (6) Poorly controlled diabetes (glycosylated hemoglobin [HbA1c] > 8.0%); (7) Active alcohol abuse; (8) Conditions other than UC (e.g., moderate to severe asthma) that could require treatment with > 20 mg/day of prednisone or prednisone equivalent during the course of the study; (9) History of malignancy except completely excised basal cell carcinoma or squamous cell carcinoma of the skin; (10) Impaired renal function (serum creatinine > 1.5 x upper limit of normal [ULN]); (11) Impaired hepatic function in the absence of a diagnosis of primary sclerosing cholangitis (serum transaminases > 2.5 × ULN, alkaline phosphatase > 2.5 × ULN, total bilirubin > 1.5 x ULN, or abnormalities in synthetic liver function tests judged by the investigator to be clinically significant). If the patient has a diagnosis of primary sclerosing cholangitis, serum transaminases > 3 × ULN, alkaline phosphatase > 3 ×ULN, total bilirubin > 2.5 x ULN, or abnormalities in synthetic liver function tests judged by the investigator to be clinically significant; (12) Hospitalized (other than for elective reasons) within 4 weeks prior to screening; (13) Thrombocytopenia (platelet count < 75,000/µL); (14) significant abnormalities on baseline neurologic examination (including baseline abnormalities in the Mini-Mental State Examination (MMSE) and PML assessment).

The exclusion criteria related to risk factors for infections include: (1) Congenital immune deficiency; (2) Positive tests for antibodies indicating active or prior infection with HIV or hepatitis B (HBV) or C (HCV); (3) Positive IgM antibody titers in the presence of negative IgG titers to Epstein Barr virus; (4) Positive stool test for ova or parasites, positive stool culture for pathogens, or positive stool toxin assay for Clostridium difficile at screening; (5) Intestinal mucosa biopsy positive for cytomegalovirus (CMV) at screening; (6) Positive screening test for latent mycobacterium tuberculosis (TB) infection; (7) Purified protein derivative (PPD) skin test or laboratory assays such as QuantiFERON®-TB Gold (QFT-G) and equivalent blood test are acceptable screening assays; (8) QFT-G (or equivalent blood test) should be used in patients previously vaccinated with Bacille Calmette-Guérin (BCG); (9) If a negative PPD test has been documented within 3 months prior to screening, it does not need to be repeated; (10) Patients with a history of latent TB infection who have received an appropriate and documented course of therapy may be included if the screening examination and a chest X-ray performed within 3 months before screening reveals no evidence of current active infection. In that case, a TB screening test will not be required; (11) History of any opportunistic infections within 12 weeks prior to initiation of study treatment; (12) Demyelinating disease or history of PML; (13) Any current or recent (within 4 weeks prior to initiation of study treatment) signs or symptoms of infection; (14) Received oral antibiotics within 4 weeks prior to initiation of study treatment or IV antibiotics within 8 weeks prior to initiation of study treatment; (15) Received a live attenuated vaccine within 4 weeks prior to initiation of study treatment; (16) Neutropenia (absolute neutrophil count < 1500/µL) or Lymphopenia (absolute lymphocyte count < 500/µL).

The exclusion criteria related to concomitant medications for UC include: (1) Received any investigational treatment within 12 weeks prior to initiation of study treatment (or within 5 half lives of the investigational product, whichever is greater); (2) Previous exposure (within the previous 6 months) to rituximab; (3) Previous exposure to rhuMAb Beta7; (4) History of severe allergic or anaphylactic reactions to chimeric, human, or humanized antibodies or fusion proteins.

### Study Treatment

### Trial Drug and Placebo

RhuMAb Beta7 drug product and placebo are prepared by Genentech. They are clear to slightly opalescent, colorless to slightly yellow aqueous solutions. Both solutions are sterile and preservative-free liquid intended for IV and SC administration.

### Dosage and Administration

Patients will be randomly allocated (1:1:1) to receive either (1) 100 mg of rhuMAb Beta7 at Week 0 followed by placebo at Week 2 and 100 mg of rhuMAb Beta7 at Weeks 4 and 8; or (2) 420 mg of rhuMAb Beta7 at Week 0 followed by 300 mg at Weeks 2, 4, and 8; or (3) placebo at Weeks 0, 2, 4, and 8.

All doses will be delivered SC. Injections will be administered to the abdomen. If the abdomen is not available for injection, the patient may receive SC dose in the thigh. Each patient will receive one or more injections as needed for the particular dose to be administered.

### Assay Methods

### Anti-Therapeutic Antibody Assays

Serum samples will be obtained from all patients for assessment of ATAs. Serum ATA samples will be analyzed using a validated antibody-bridging electrochemiluminesence assay. PK samples may be used to assess ATA response if necessary. The assays will follow the overall guidance for ATA tests (Mire-Sluis et al. J Immunol Methods 289:1-16 (2004)). The assay method is described in detail above.

### Pharmacokinetic Assays

Serum samples will be obtained from all patients for determination and characterization of the PK of rhuMAb Beta7. Serum PK samples will be analyzed using a validated bridging ELISA, with a minimum quantifiable concentration of 12.5 ng/mL. The assay method is described in detail above.

### Pharmacodynamic Assays

Whole blood and/or serum samples will be analyzed using qualified methods for exploratory PD biomarker analysis, including but not limited to flow cytometry, qRT PCR, and/or ELISA. Additional details about the assays are described above. For the Phase II study, however, a different competing anti-beta7 antibody, FIB504, (which binds to the same epitope as rhuMAb Beta7) will be used instead of rhuMabBeta7.

### Efficacy Analyses

### Primary Efficacy Endpoint

The primary endpoint is maintenance of remission. Maintenance of remission refers to the proportion of patients in clinical remission at at a given time, e.g., at 6 months, 1, year, or 2 years. The difference between each rhuMAb Beta7 arm and placebo will be evaluated using the Mantel-Haenszel test statistic, stratified by concomitant treatment with corticosteroids, concomitant treatment with immunosuppressants, and previous anti-TNF exposure. Additionally, the difference between treatment groups will be evaluated by constructing 80% confidence intervals for the primary efficacy endpoint. Descriptive summary statistics will be provided for each treatment group.

Sustained remission can also be assessed. Sustained remission is the number of patients who once they have remission induced remain in remission over time. It is the individual paitent experience from the time remission is induced to the next flare. This means that the paitent does not require an increase in concomitant medication for UC to retain remission.

### Secondary Efficacy Endpoints

The secondary efficacy endpoints for this study are as follows: (1) The proportion of patients with clinical response at Week 6 and Week 10 where clinical response is defined by at least a 3-point decrease and 30% reduction from baseline in MCS and a ≥ 1-point decrease in rectal bleeding subscore or absolute rectal bleeding score of 0 or 1; (2) The proportion of patients in clinical remission (defined above) at Week 6; (3) The proportion of patients who achieve an endoscopic score and rectal bleeding score of 0.

The secondary efficacy endpoints will be analyzed in the same manner (described above) as the primary efficacy outcome measure.

### Safety Analysis

Safety will be assessed by recording adverse events, conducting clinical laboratory evaluations, and assessing immunogenicity by measuring ATAs. Patients will be analyzed according to actual treatment received.

Adverse events will be recorded from the time informed consent is given until a patient completes the study or discontinues prematurely. Separate summaries of adverse events will be provided for the screening period, the 10-week treatment period, and the 18-week follow-up period. Adverse events will be summarized by treatment group.

Clinical laboratory data (serum chemistry, hematology evaluations including complete blood count with differential and platelet counts, and urinalysis values) will be summarized by treatment group with use of descriptive statistics.

All patients will be included in the safety analysis.

### Pharmacokinetic and Pharmacodynamic Analyses

PK parameters derived from serum concentrations of rhuMAb Beta7, including Cₘₐₓ after the first and final dose, Tₘₐₓ after the first and final doses, AUC within the last dosing interval (AUC₅₇₋₈₅) and from Time 0 to time of last detectable observation (AUCₗₐₛₜ) or to infinity (AUC_{inf}), CL/F, V/F, and t_{1/2} will be estimated for all patients with use of either non-compartmental or population PK methods. Plots of individual and mean serum rhuMAb Beta7 concentration-time profiles will be prepared. Serum rhuMAb Beta7 concentrations and PK parameters will be listed by patient and dosing regimen and will be summarized by descriptive statistics.

PK data from this study may be combined with data from the Phase I study to perform a population PK analysis. Population typical value of PK parameters will be estimated for the entire study population, along with estimates of intra- and interpatient variance and an estimate of random error. Individual patient parameter estimates will be computed using the post hoc analysis procedure. A prospective analysis plan will be prepared, and the population PK analysis will be presented in a report separate from the Clinical Study Report. The population PK analysis may include an exploratory analysis to identify baseline covariates that affect the PK of rhuMAb Beta7 in this patient population. Baseline covariates to be examined will include demographics and other patient characteristics, such as disease severity and selected laboratory measures. Data will be summarized using appropriate descriptive statistics.

Assessment of exposure-clinical response relationship will be exploratory, and the correlations between rhuMAb Beta7 exposure (Cₘₐₓ, Cₘᵢₙ, or AUC) and clinical endpoints (such as MCS) may be evaluated.

All analyses related to PD biomarker objectives will be exploratory. Results will be summarized as absolute values and/or change from baseline. Additional PK and PD analysis will be conducted as appropriate.

### Preliminary Results of Open Label Extension Study

The first 16 patients in the open label extension study, all of whom had failed to respond to anti-TNF agents, were evaluated using a partial Mayo Clinic Score (pMCS). The pMCS incorporated three components of the full Mayo Clinic Score, which were: the number of stools the patient passed each day, the quantity of rectal bleeding experienced by the patient each day; and the Physician's global assessment of the patient's disease, which was based on the previous two components but also took into account any abdominal pain the patient suffered from and how well the patient was feeling in general in relation to their UC. The preliminary results for each of the 16 patients are shown in Figure 8.

As can be seen in Fig. 8, 13 out of the 16 patients demonstrated at least a one point improvement in the pMCS. Eleven of the 16 patients demonstrated a two point improvement in the pMCS. In addition, six of the patients were treated for 12-16 weeks and three-four of them demonstrated improvement over time. Thus, although preliminary, these data provide evidence of clinical activity of rhuMAb Beta7 in patients with moderate to severe UC refractory to anti-TNF therapy.

In summary, rhuMAb Beta7 binds α4β7 and αEβ7, receptors which regulate trafficking and retention of lymphocyte subsets, respectively, in the intestinal mucosa. Clinical studies have demonstrated the efficacy of an anti-α4 antibody (natalizumab) for the treatment of CD, and encouraging results have been observed for anti-α4β7 antibody (LDP02/MLN02/MLN0002/vedolizumab) in the treatment of UC. These findings validate α4β7 as a therapeutic target and support the hypothesis that the interaction between α4β7 and MAdCAM 1 contributes to the pathogenesis of IBD. The specificity of rhuMAb Beta7 for blocking of lymphocyte trafficking to the intestinal mucosa in addition to the potential additive therapeutic effects of targeting αEβ7 are compelling reasons to pursue clinical development of this antibody in IBD. The potential benefits of rhuMAb Beta7 may include a reduction in disease severity in patients with UC, as measured by the Mayo Clinical Score, and may also include induction of remission, also as measured by the Mayo Clinical Score.

## Claims

1. An integrin beta7 antagonist for use in the treatment of a gastrointestinal inflammatory disorder in a patient, the treatment comprising administering to the patient a therapeutically effective amount of an integrin beta7 antagonist, wherein the integrin beta7 antagonist is administered subcutaneously at a flat dose.

2. The integrin beta7 antagonist for use according to claim 1, wherein the integrin beta7 antagonist is a monoclonal anti-beta7 antibody.

3. The integrin beta7 antagonist for use according to claim 2, wherein the anti-beta7 antibody is administered at a flat dose of between about 50 mg and 450 mg.

4. The integrin beta7 antagonist for use according to claim 2, wherein the anti-beta7 antibody is administered at a flat dose between 50 mg and 450 mg.

5. The integrin beta7 antagonist for use according to claim 3 or 4, wherein the flat dose is 50 mg or about 50 mg.

6. The integrin beta7 antagonist for use according to claim 3 or 4, wherein the flat dose is 100 mg or about 100 mg.

7. The integrin beta7 antagonist for use according to claim 3 or 4, wherein the flat dose is 150 mg or about 150 mg.

8. The integrin beta7 antagonist for use according to claim 3 or 4, wherein the flat dose is 200 mg or about 200 mg.

9. The integrin beta7 antagonist for use according to claim 3 or 4, wherein the flat dose is 300 mg or about 300 mg.

10. The integrin beta7 antagonist for use according to claim 3 or 4, wherein the flat dose is 350 mg or about 350 mg.

11. The integrin beta7 antagonist for use according to claim 3 or 4, wherein the flat dose is 400 mg or about 400 mg.

12. The integrin beta7 antagonist for use according to claim 3 or 4, wherein the flat dose is 420 mg or about 420 mg.

13. The integrin beta7 antagonist for use according to claim 3 or 4, wherein the flat dose is 450 mg or about 450 mg.

14. The integrin beta7 antagonist for use according to claim 3 or 4, wherein the anti-beta7 antibody is administered once every week, or once or every two weeks, or once every four weeks, or once every six weeks, or once every eight weeks.

15. The integrin beta7 antagonist for use according to claim 14, wherein the anti-beta7 antibody is administered for a period of two months, or three months, or six months, or 12 months, or 18 months, or 24 months, or for the lifetime of the patient.

16. The integrin beta7 antagonist for use according to claim 1, wherein the patient is a human.

17. The integrin beta7 antagonist for use according to claim 1, wherein the gastrointestinal inflammatory disorder is an inflammatory bowel disease.

18. The integrin beta7 antagonist for use according to claim 17, wherein the inflammatory bowel disease is ulcerative colitis or Crohn's disease.

19. The integrin beta7 antagonist for use according to claim 2, wherein the anti-beta7 antibody is selected from a chimeric antibody, a human antibody, and a humanized antibody.

20. The integrin beta7 antagonist for use according to claim 19, wherein the anti-beta7 antibody is etrolizumab.

21. The integrin beta7 antagonist for use according to claim 19, wherein the anti-beta7 antibody is an antibody fragment.

22. The integrin beta7 antagonist for use according to claim 19, wherein the anti-beta7 antibody comprises six hypervariable regions (HVRs), wherein:
a. HVR-L1 comprises amino acid sequence A1-A11, wherein A1-A11 is RASESVDTYLH (SEQ ID NO:1); RASESVDSLLH (SEQ ID NO:7), RASESVDTLLH (SEQ ID NO:8), or RASESVDDLLH (SEQ ID NO:9) or a variant of SEQ ID NOs:1, 7, 8 or 9 (SEQ ID NO:26) wherein amino acid A2 is selected from the group consisting of A, G, S, T, and V and/or amino acid A3 is selected from the group consisting of S, G, I, K, N, P, Q, R, and T, and/or A4 is selected from the group consisting of E, V, Q, A, D, G, H, I, K, L, N, and R, and/or amino acid A5 is selected from the group consisting of S, Y, A, D, G, H, I, K, N, P, R, T, and V, and/or amino acid A6 is selected from the group consisting of V, R, I, A, G, K, L, M, and Q, and/or amino acid A7 is selected from the group consisting of D, V, S, A, E, G, H, I, K, L, N, P, S, and T, and/or amino acid A8 is selected from the group consisting of D, G, N, E, T, P and S, and/or amino acid A9 is selected from the group consisting of L, Y, I and M, and/or amino acid A10 is selected from the group consisting of L, A, I, M, and V and/or amino acid A11 is selected from the group consisting of H, Y, F, and S;
b. HVR-L2 comprises amino acid sequence B1-B8, wherein B1-B8 is KYASQSIS (SEQ ID NO:2), RYASQSIS (SEQ ID NO:20), or XaaYASQSIS (SEQ ID NO:21, where Xaa represents any amino acid) or a variant of SEQ ID NOs:2, 20 or 21 (SEQ ID NO:27) wherein amino acid B1 is selected from the group consisting of K, R, N, V, A, F, Q, H, P, I, L, Y and Xaa (where Xaa represents any amino acid), and/or amino acid B4 is selected from the group consisting of S and D, and/or amino acid B5 is selected from the group consisting of Q and S, and/or amino acid B6 is selected from the group consisting of S, D, L, and R, and/or amino acid B7 is selected from the group consisting of I, V, E, and K;
c. HVR-L3 comprises amino acid sequence C1-C9, wherein C1-C9 is QQGNSLPNT (SEQ ID NO:3) or a variant of SEQ ID NO:3 (SEQ ID NO:28) wherein amino acid C8 is selected from the group consisting of N, V, W, Y, R, S, T, A, F, H, I L, and M;
d. HVR-H1 comprises amino acid sequence D1-D10 wherein D1-D10 is GFFITNNYWG (SEQ ID NO:4);
e. HVR-H2 comprises amino acid sequence E1-E17 wherein E1-E17 is GYISYSGSTSYNPSLKS (SEQ ID NO:5), or a variant of SEQ ID NO:5 (SEQ ID NO:29)wherein amino acid E2 is selected from the group consisting of Y, F, V, and D, and/or amino acid E6 is selected from the group consisting of S and G, and/or amino acid E10 is selected from the group consisting of S and Y, and/or amino acid E12 is selected from the group consisting of N, T, A, and D, and/or amino acid 13 is selected from the group consisting of P, H, D, and A, and/or amino acid E15 is selected from the group consisting of L and V, and/or amino acid E17 is selected from the group consisting of S and G; and
f. HVR-H3 comprises amino acid sequence F2-F11 wherein F2 -F11 is MTGSSGYFDF (SEQ ID NO:6) or RTGSSGYFDF (SEQ ID NO:19); or comprises amino acid sequence F1-F11, wherein F1-F11 is AMTGSSGYFDF (SEQ ID NO:16), ARTGSSGYFDF (SEQ ID NO:17), or AQTGSSGYFDF (SEQ ID NO:18), or a variant of SEQ ID NOs:6, 16, 17, 18, or 19 (SEQ ID NO:30) wherein amino acid F2 is R, M, A, E, G, Q, S, and/or amino acid F11 is selected from the group consisting of F and Y.

23. The integrin beta7 antagonist for use according to claim 22, wherein the anti-beta7 antibody comprises three heavy chain hypervariable region (HVR-H1-H3) sequences and three light chain hypervariable region (HVR-L1-L3) sequences, wherein:
a. HVR-L1 comprises SEQ ID NO:7, SEQ ID NO:8 or SEQ ID NO:9;
b. HVR-L2 comprises SEQ ID NO:2;
c. HVR-L3 comprises SEQ ID NO:3;
d. HVR-H1 comprises SEQ ID NO:4;
e. HVR-H2 comprises SEQ ID NO:5; and
f. HVR-H3 comprises SEQ ID NO:6 or SEQ ID NO:16 or SEQ ID NO:17 or SEQ ID NO:19.

24. The integrin beta7 antagonist for use according to claim 23, wherein the anti-beta7 antibody comprises three heavy chain hypervariable region (HVR-H1-H3) sequences and three light chain hypervariable region (HVR-L1-L3) sequences, wherein:
a. HVR-L1 comprises SEQ ID NO:9;
b. HVR-L2 comprises SEQ ID NO:2;
c. HVR-L3 comprises SEQ ID NO:3;
d. HVR-H1 comprises SEQ ID NO:4;
e. HVR-H2 comprises SEQ ID NO:5; and
f. HVR-H3 comprises SEQ ID NO:19.

25. The integrin beta7 antagonist for use according to any one of claims 22 to 24, wherein the anti-beta7 antibody is etrolizumab.

26. The integrin beta7 antagonist for use according to claim 23, wherein the anti-beta7 antibody is administered at a flat dose of 100 mg every four weeks, wherein the patient suffers from ulcerative colitis, and wherein the patient experiences mucosal healing or a reduced rate of flare over time.

27. The integrin beta7 antagonist for use according to claim 23, wherein the anti-beta7 antibody is administered at a flat dose of 300 mg every four weeks, wherein the patient suffers from ulcerative colitis, and wherein the patient experiences mucosal healing or a reduced rate of flare over time.

28. The integrin beta7 antagonist for use according to any one of claims 1-27, wherein the integrin beta7 antagonist is administered with at least one additional compound selected from 5-aminosalicylic acid (5-ASA), azathioprine (AZA), 6-mercaptopurine (6-MP), and methotrexate.

29. The integrin beta7 antagonist for use according to any one of claims 1-27, wherein the patient previously failed at least one biological agent.

30. The integrin beta7 antagonist for use according to claim 29, wherein the biological agent is selected from adalimumab, etanercept, infliximab, golimumab, certolizumab pegol, natalizumab, and vedolizumab.

31. The integrin beta7 antagonist for use according to any one of claims 1-27, wherein the integrin beta7 antagonist is administered with a self-inject device.

32. The integrin beta7 antagonist for use according to claim 31, wherein the self-inject device is selected from a prefilled syringe, microneedle device, autoinjector device and needle-free injection device.

33. Etrolizumab for use in the induction of remission in a patient suffering from ulcerative colitis comprising administering to the patient a therapeutically effective amount of etrolizumab, wherein etrolizumab is administered subcutaneously at a flat dose of either 100mg or 300 mg every four weeks.

34. Etrolizumab for use in the induction of remission according to claim 33, further comprising determining a Mayo Clinic Score and Mayo Clinic subscores, wherein the patient is determined to have an absolute Mayo Clinic Score ≤ 2 and no individual subscore >1.

35. Etrolizumab for use in the induction of remission according to claim 34, wherein remission is induced at least by week 10 following the first administration of etrolizumab.

36. Etrolizumab for use in the induction of sustained remission in a patient suffering from ulcerative colitis, comprising administering to the patient a therapeutically effective amount of etrolizumab, wherein etrolizumab is administered subcutaneously at a flat dose of either 100mg or 300 mg every four weeks, wherein remission is retained for a period of 8 weeks following induction of remission, or a period of 30 weeks following induction of remission, or a period of 50 weeks following induction of remission, or a period of 54 weeks or more following induction of remission.

37. Etrolizumab for use in the induction of sustained remission according toclaim 36, wherein the remission is steroid-free remission.

38. Etrolizumab for use in the induction of sustained remission according toclaim 37, wherein the steroid-free remission is for 20 weeks following induction of remission, or for 24 weeks or longer following induction of remission.
